# EUROPEAN PATENT APPLICATION

(11) **EP 2 857 501 A1**
(43) Date of publication of application: **08.04.2015**
(21) Application number: 13004776.4
(22) Date of filing: 03.10.2013
(51) Int. Cl.: C12N 5/0735, C12N 5/076

(54) **Reprogramming of pluripotent stem cells for improved control of their differentiation pathways**

(71) Applicant: ETH Zurich, 8092 Zurich (CH)
(72) Inventor: Vogel, Viola, 5400 Baden (CH); Moshfegh, Cameron, 8050 Zürich (CH)

(57) **Abstract**

The present invention relates to a method to reprogramming pluripotent stem cells (PSCs) by epigenetic conditioning and metabolic reprogramming into pPSCs with highly controllable biological functions, the cells obtained by said method as well as methods ofusing said cells.

## Description

The present invention relates to a method to reprogram pluripotent stem cells (PSCs) by epigenetic conditioning and metabolic reprogramming into pPSCs with highly controllable biological functions, the cells obtained by said method as well as methods of using said cells.

### BACKGROUND

Pluripotent stem cells (PSCs) have the potential to differentiate into cell types of all three germ layers as well as into germ cells. Therefore there is much interest in using their differentiation potential for clinical applications such as stem cell therapies, tissue and organ regeneration, fertility restoration, cancer therapy and research. However, when implanted in *vivo* undifferentiated or partially differentiated PSCs or a subset thereof, form teratomas or other cancers. Tumorigenicity of PSCs is currently the largest challenge for their clinical application. The current approaches to address the problem of tumorigenicity of PSCs include the generation of partially differentiated PSCs called progenitor cells with subsequent sorting of cells, introduction of PSC-selective suicide genes, or non-genetic selective killing of PSCs Partially differentiated progenitor cells would be committed already to a lineage, but be non-tumorigenic and these cells would be sorted from the rest of the cell population. But since differentiation is a gradual process and not an on/off switch, the sorting may not be efficient enough to eliminate tumorigenicity. It remains therefore unknown how safe such sorting approaches would be. The introduction of suicide genes into PSCs to selectively kill remaining PSCs after the cells have been differentiated has been described. However genetic manipulation is undesired and may lead to instability of the genome and immunogenic reactions of the host. Non-genetic selective killing of PSCs has been implemented by using antibodies which target undifferentiated human ESCs. However, it is uncertain whether the compounds used for killing PSCs would omit partially differentiated cells which retain part of the PSC identity. Also an *in vivo* application of such an approach would create the risk that naturally occurring stem cells of the body would be killed as well, potentially weakening the patient. In one approach PSC-derived cardiomyocytes were enriched and PSCs selectively killed by increasing the lactate concentration in the cell culture medium and therefore metabolically selecting the survival of the cardiomyocytes. However, this approach would not be applicable to any differentiated cell type.

In patent US20120288936 A1, polypeptides in combination with Dnmt inhibitors or histone deacetylase inhibitors are used to reprogram somatic cells into PSCs or to transform PSCs into other stem cell types. In patent US20100159459 A1, treatments to induce a hypomethylated genome to create PSCs and restore differentiation potential have been described. However, both approaches were not able to solve the problem of tumorigenicity.

In patent WO2013059829 A1, an approach is described to create non-tumorigenic PSCs by treating human fibroblasts with the ECM (extracellular matrix) component fibromodulin which are characterized by expression of the core pluripotency factors nanog, oct4 and sox2 as well as the negative cell cycle regulators p15 and p21. However, the reported efficiency of reprogramming was around 0.03% which indicates that an already present subpopulation of non-tumorigenic PSCs was selected instead of the fibroblasts actually being re-programmed. Furthermore, the resulting cells do not exhibit a germ-cell like character. A reprogramming efficiency of 0.03% may create large costs to sort and/or expand the desired cell populations for further applications. It would thus, be desirable to obtain the cell population in a more efficient manner. The method of the present invention achieves a much higher efficiency, since all characterizations performed on the reprogrammed cells showed clear and strong trends without further cell sorting. Reprogramming efficiency by stimulation with fibromodulin may depend on the cell type that is stimulated, while in the present invention reprogramming is not based on stimulation with growth factors or other proteins, but is achieved chemically by creating an epigenetically blanked state in a PSC, irrespective of its origin, and subsequent metabolic reprogramming constraints, which creates a much more thorough and efficient reprogramming than the stimulation with proteins only. Furthermore, the generation of germ cells may represent another advantage of the present invention compared to reprogramming with fibromodulin which does not lead to the creation of germ cells, since fertility related therapies are of high social and economical interest.

Cancer is currently the second highest cause of death after cardiovascular disease in industrialized countries. Since many cellular mechanisms can lead to and promote cancer development, cancer is a very heterogeneous and complex disease. There is an ongoing search to find new genes whose aberrant activity is involved in cancer. Finding new genes involved in cancer could lead the way for the development of new cancer drugs. Therefore, methods that could identify new cancer-related genes would be of great value.

Since cancer cells originate from the body's own cells they often are difficult to selectively target without harming other important cells in the body such as stem cells. There is an ongoing search to find new therapies which can selectively target cancer cells without harming other cells in the body. In particular, most cancer drugs also harm stem cells and current approaches aim to guide the cytotoxic compounds specifically to the site of tumors to avoid exposure of other parts of the body with the drug. For this antibody-based approaches are widely used. However, the active cancer drug itself is in most cases toxic to stem cells as well. It would therefore be of interest to find drugs which are only toxic to cancer cells. Current approaches to identify genes involved in cancer include screening for genes that can cause transformation of noncancerous cells into cancerous cells, identification of genes associated with chromosomal abberations and identification of viral oncogenes, different viral and non-viral mutagenesis methods and RNA interference. However, since cancer cells and stem cells often share very similar cellular regulatory mechanisms it is very promising to study how stem cells avoid the development of cancer. PSCs are stem cells which are at the same time tumorigenic. Yet their tumorigenicity does not originate from genetic mutations or other damages, but is an inherent property of this cell type. Reprogramming PSCs into a non-tumorigenic state would therefore be of great interest allowing to identify and investigate into genes and mechanisms involved in cancer development.

Cancer Therapy in males often leads to infertility due to damage to the eggs or the structures which support ovulation in females as well as the spermatogonial stem cell (SSC) population or the structures which support spermatogenesis in male. The only option available currently to preserve fertility after cancer treatment in young males is the cryopreservation of semen. However this option is not ideal and it would be preferable if the natural fertility could be restored again after the cancer treatment. On the other hand the situation looks even worse for male childhood cancer patients, since no fertile semen is available yet. In these cases cryopreservation of SSCs seems to be the only option available. This is also prone to problems such as long-term stability of the frozen SSCs or contamination with cancerous cells before the therapy. Infertility or subfertility in the male general population may also involve a decrease in spermatogonial stem cell number or function in certain cases. Better therapeutic options for preservation of fertility would therefore be of great value, especially to the group of cancer survivors. Currently, it has been shown in experiments with rhesus monkeys that the transplantation of spermatogonial stem cells (SSC) can restore spermatogenesis. These results gave much hope that if there was a way to obtain SSCs easily they could be used for fertility restoration in human males. There is also big interest in creating SSCs from other cell types such as PSCs and current work has been successful in showing the induction of certain SSC markers in PSCs such as Zbtb16. There has also been considerable progress in defining culture conditions which allow long-term culture of SSCs. One of the main growth factors that mediates SSC growth and identity is glial cell-line derived neurotrophic factor (GDNF). However, GDNF also has tumorigenic effects. Just recently reports have been published showing that the most undifferentiated SSCs are negative for the GDNF receptor Gfra1. It has also been reported that the SSC populations expresses Nanog. However, other work aiming to differentiate PSCs into SSCs reported the absence of Nanog expression. All of this is further complicated by the fact that even though partial SSC characteristics can be obtained *in vitro* from PSCs, tumorigenicity remains a great problem. There is thus, still an urgent need in the art to develop pluripotent stem cells which are none tumorigenic. Furthermore, the development of pluripotent stem cells exhibiting germ cell character would be highly desirable.

The generation of SSCs would also be of great economical interest in the farm animal industry, particularly the beef cattle industry, since the current artificial insemination techniques as used in breeding practice are connected with large logistical challenges. Having a convenient way to produce SSCs could save capital and time in the breeding of desired animal characteristics. Such technology may also show useful in the conservation of endangered animal species, which could be of great economical interest, for example in zoos.

When developing drugs for cancer therapy or other diseases such as hepatitis C or degenerative diseases, side effects such as damage to the germ cells leading to subfertility or infertility are a latent risk. Screening methods to identify the effects of drugs on the germ lineage would be of great benefit to patients and therefore of significant social and economical interest. Currently there exist no good upscalable model systems for gonocytes, SSCs or similar adult germ cells that would allow large scale toxicity screening of drugs. Therefore, it would be of great interest to have an easily obtainable and upscalable cell model system to test the toxicity of drugs on germ lineages during drug development.

Furthermore, such a screening system could also be used to test the toxicity of environmental pollutants such as herbicides, insecticides, industrial chemicals or nanoparticles.

In most cultured PSCs pluripotency is linked to cell proliferation. When PSCs reduce proliferation rates or enter proliferation arrest, their pluripotency is lost and the cells differentiate or apoptose. However, in many experimental and clinical applications such as tissue engineering, proliferation of the undifferentiated PSCs is not desired, making cell targeting difficult as well as introducing heterogeneity into the cell population. Therefore it would be of interest to have methods that could arrest proliferation of PSCs without loosing their pluripotency and allow the cells to restart proliferation at a later time point when the cells are already differentiated. Ideally such an arrest of cell proliferation should be achievable over a period of one to several days which would allow to subject the cells to various stimuli over a biologically meaningful time period. Currently no methods exist to reversibly arrest cell proliferation in PSCs for periods longer than 12-24 hours without significant loss of pluripotency and cell death. One study showed that the length of the Gap 1 phase of the cell cycle in ESCs can be increased by inhibition of cyclin dependent kinases (CDKs) without losing pluripotency. However, such drugs are cytotoxic to PSCs for time periods longer than 12 hours and this study was more of academic interest than a practical use. Since pluripotent, but quiescent stem cells with low proliferation have been found in the adult body, it is of great interest to understand what mechanisms regulate proliferation of PSCs. Understanding and controlling the proliferation of PSCs could represent an important step to overcome the tumorigenicity of PSCs. It It could also give valuable insights into mechanisms that regulate cancer differentiation and malignancy, since cancer cells proliferate in a misregulated manner.

The transport of cells is often associated with considerable logistic difficulties. Usually cells are transported in a frozen state and need to be constantly cooled below a certain temperature to avoid deterioration of cellular properties such as viability. For certain applications freezing and thawing of cells may cause undesirable cell changes in the cells which might interfere with the application of these cells. This may include cells that need to be in contact with a substrate to be used in a clinical application and would needed to be recultured and/or expanded for a long time before they could be used if they were disconnected from their substrate. Certain slow growing cells may be transported without freezing, but PSCs so far cannot be transported over long distances without freezing the cells.

Currently different freezing media are available to freeze PSCs with minimal loss of cell viability, but so far there is no method to transport PSCs for longer times without freezing the cells. To identify such a transportation method would be highly desirable.

### SUMMARY OF THE INVENTION

The present invention relates in a first aspect to a method of reprogramming pluripotent stem cells (PSC) comprising the steps (a) conditioning PSCs epigenetically, and (b) metabolically reprogramming PSCs.

In a second aspect, the present invention relates to a differentiated cell reprogrammed by the method of the first aspect.

In a third aspect the present invention relates to a pluripotent stem cell reprogrammed by the method of the first aspect.

In a fourth aspect, the present invention relates to a reprogrammed pluripotent stem cell (pPSC) characterized in that the expression of germ cell markers, tumour suppressor markers, oxidative phosphorylation markers, pentose phosphate pathway markers, NADPH generating enzyme markers, glycolysis markers, glycogen metabolism markers, and/or pluripotency markers is altered.

In a fifth aspect the present invention relates to a reprogrammed pluripotent stem cell pPSC) according to the second, third or fourth aspect of the present invention for use in treating cancer.

In a sixth aspect the present invention relates to a reprogrammed pluripotent stem cell (pPSC) according to the third or fourth aspect of the present invention for use in treating infertility in male caused by anti-cancer therapy or other therapy which damages the SSC population within the testis, environmental factors, heat, cold, food, radiation, chemical toxicity, infection, inflammation, autoimmune disease, physical injury or genetics

In a seventh aspect the present invention relates to a reprogrammed pluripotent stem cell (pPSC) according to the second, third or fourth aspect of the present invention for use in cell or tissue therapy for tissue and organ regeneration of the heart, cardiovascular system, brain, neurological system, eye, ear, liver, kidney, pancreas, endocrine glands, lung, intestines, muscle, skin, hair, joints, bones, and/or teeth.

In an eighth aspect the present invention relates to a method of screening for a pharmaceutical, preferably screening for a pharmaceutical directed against cancer or a degenerative disease or screening for contraception drugs, comprising the use of a differentiated cell according to the second aspect of the present invention and/or a reprogrammed pluripotent stem cell (pPSC) according to the third or fourth aspect of the present invention.

In a ninth aspect the present invention relates to a method of testing the toxicity of a pharmaceutical comprising the use of a differentiated cell according to the second aspect of the present invention and/or a reprogrammed pluripotent stem cell (pPSC) according to the third or fourth aspect of the present invention.

In a tenth aspect the present invention relates to a method of testing the toxicity of a environmental substance, preferably a molecule or particle, comprising the use of a differentiated cell according to the second aspect of the present invention and/or a reprogrammed pluripotent stem cell (pPSC) according to the third or fourth aspect of the present invention. In preferred embodiments the substance may cause cancer.

In an eleventh aspect the present invention relates to a pharmaceutical comprising a differentiated cell according to the second aspect of the present invention and/or a reprogrammed pluripotent stem cell (pPSC) according to the third or fourth aspect of the present invention.

In a twelfth aspect the present invention relates to a method of identifying genes involved in disease, preferably involved in a disease selected from the group consisting of cancer, infertility, oligospermia, aspermia, hypospermia, azoospermia, teratospermia, asthenozoospermia, cardiovascular disease, atherosclerosis, hepatitis, fatty liver disease, cirrhosis, primary sclerosing cholangitis, hemochromatosis, chronic kidney disease, glomerulonephritis, polycystic kidney disease, alzheimer's disease, parkinson's disease, multiple sclerosis, amyotrophic lateral sclerosis, motor neuron diseases, lewy body disease, huntington's disease, spinocerebellar ataxia, friedreich's ataxia, spinal muscular atrophy, retinopathy, macular degeneration and diabetes comprising the use of a differentiated cell according to the second aspect of the present invention and/or a reprogrammed pluripotent stem cell (pPSC) according to the third or fourth aspect of the present invention.

In a thirdteen aspect the present invention relates to a method of producing germ cells comprising the use of a reprogrammed pluripotent stem cell (pPSC) according to the third or fourth aspect of the present invention.

In a fourteen aspect the present invention relates to a method of producing sperm cells or oocytes *in vitro* comprising the use of a reprogrammed pluripotent stem cell (pPSC) according to the third or fourth aspect of the present invention.

In a fifteens aspect the present invention relates to a method of treating a patient, preferably a patient suffering from cancer, comprising administering to a subject a differentiated cell according to the second aspect of the present invention or a reprogrammed pluripotent stem cell (pPSC) according to the third or fourth aspect of the present invention.

### LIST OF FIGURES

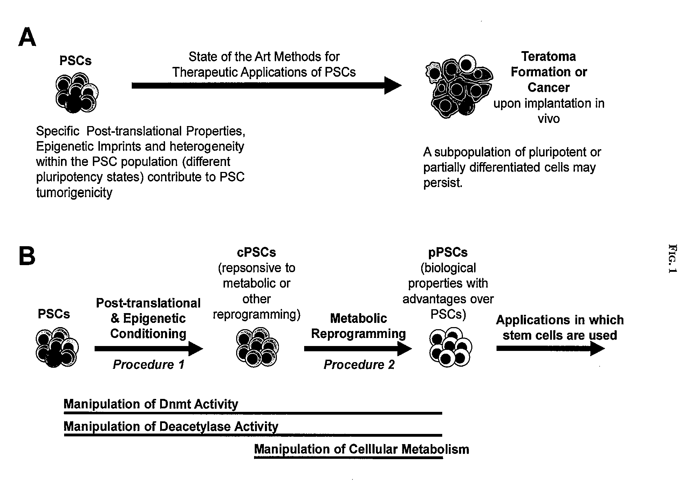

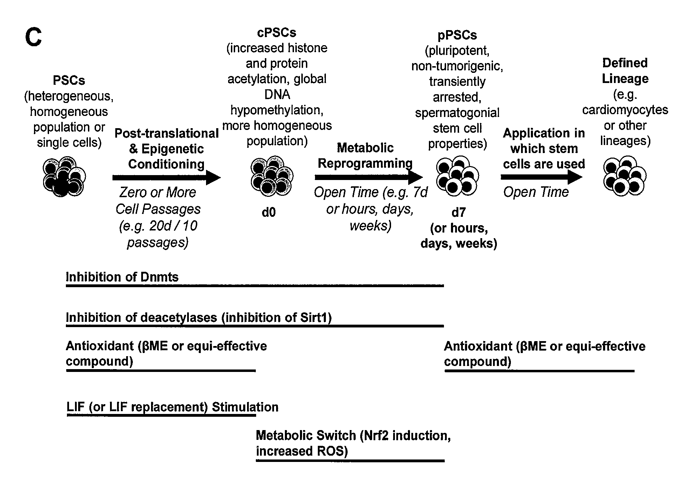

- **Fig. 1**: Schematic Illustration of (A) the state of the art, (B) the principle method of reprogramming PSCs according to the present invention; and (C) a specific example of the method of reprogramming PSCs according to the present invention
- **Fig. 2**: Cell Proliferation and Apoptosis During Metabolic Reprogramming
- **Fig. 3**: Cyclin Protein Expression During Metabolic Reprogramming
- **Fig. 4**: Core Pluripotency Marker Expression During Metabolic Reprogramming
- **Fig. 5**: Core Pluripotency Protein Expression and Stat3 Phosphorylation Status During Metabolic Reprogramming
- **Fig. 6**: Core Pluripotency mRNA Expression Profiles During and After Metabolic Repro gramming
- **Fig. 7**: Naive Pluripotency mRNA Expression Profiles During and After Metabolic Reprogramming
- **Fig. 8**: Primed Pluripotency mRNA Expression Profiles During and After Metabolic Reprogramming
- **Fig. 9**: Germ Cell mRNA Expression Profiles During and After Metabolic Reprogramming
- **Fig. 10**: CDK Inhibitor mRNA Expression Profiles During and After Metabolic Reprogramming
- **Fig. 11**: Antioxidant Enzyme mRNA Expression Profiles During and After Metabolic Reprogramming
- **Fig. 12**: Nrf2 Nuclear Localization During Metabolic Reprogramming
- **Fig. 13**: Metabolic Enzyme I mRNA Expression Profiles During and After Metabolic Reprogramming
- **Fig. 14**: Metabolic Enzyme II mRNA Expression Profiles During and After Metabolic Reprogramming
- **Fig. 15**: pPSCs Resume Proliferation After Removal of Metabolic Reprogramming Conditions
- **Fig. 16**: Cardiogenic mRNA Expression Profiles of pPSCs During Induction of Cardiogenic Differentiation
- **Fig. 17**: Cardiogenic Protein Expression Profiles of pPSCs During Induction of Cardiogenic Differentiation

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise; all technical and scientific terms used herein have the same meanings as commonly understood by one of ordinary skill in the art.

Several documents are cited throughout the text of this specification. Each of the documents cited herein (including all patents, patent applications, scientific publicatiorts, manufacture's specifications, instructions etc.), whether supra or infra, is hereby incorporated by reference in its entirety. Nothing herein is to be construed as an admission that the invention is not entitled to antedate such disclosure by virtue of prior invention. Some of the documents cited herein are characterized as being *"incorporated by reference* ". In the event of a conflict between the definitions or teachings of such incorporated references and definitions or teachings recited in the present specification, the text of the present specification takes precedence.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used in this specification and the appended claims, the singular forms "a", "an", and "the" include plural referents, unless the content clearly dictates otherwise.

The term "about" when used in connection with a numerical value is meant to encompass numerical values within a range having a lower limit that is 5% smaller than the indicated numerical value and having an upper limit that is 5% larger than the indicated numerical value.

### DEFINITIONS

The terms "polynucleotide" and "nucleic acid" are used interchangeably herein and are understood as a polymeric or oligomeric macromolecule made from nucleotide monomers. Nucleotide monomers are composed of a nucleobase, a five-carbon sugar (such as but not limited to ribose or 2'-deoxyribose), and one to three phosphate groups. Typically, a polynucleotide is formed through phosphodiester bonds between the individual nucleotide monomers. In the context of the present invention referred to nucleic acid molecules include but are not limited to ribonucleic acid (RNA), deoxyribonucleic acid (DNA), and mixtures thereof such as e.g. RNA-DNA hybrids. The nucleic acids, can e.g. be synthesized chemically, e.g. in accordance with the phosphotriester method (see, for example, Uhlmann, E. & Peyman, A. (1990) Chemical Reviews, 90, 543-584). Aptamers are nucleic acids which bind with high affinity to a polypeptide. Aptamers can be isolated by selection methods such as SELEmir146-a (see e.g. Jayasena (1999) Clin. Chem., 45, 1628-50; Klug and Famulok (1994) M. Mol. Biol. Rep., 20, 97-107; US 5,582,981) from a large pool of different single-stranded RNA molecules. Aptamers can also be synthesized and selected in their mirror-image form, for example as the L-ribonucleotide (Nolte et al. (1996) Nat. Biotechnol., 14, 1116-9; Klussmann et al. (1996) Nat. Biotechnol., 14, 1112-5). Forms which have been isolated in this way enjoy the advantage that they are not degraded by naturally occurring ribonucleases and, therefore, possess greater stability. Nucleic acids may be degraded by endonucleases or exonucleases, in particular by DNases and RNases which can be found in the cell It is, therefore, advantageous to modify the nucleic acids in order to stabilize them against degradation, thereby ensuring that a high concentration of the nucleic acid is maintained in the cell over a long period of time (Beigelman et al. (1995) Nucleic Acids Res. 23:3989-94; WO 95/11910; WO 98/37240; WO 97/29116). Typically, such a stabilization can be obtained by introducing one or more internucleotide phosphorus groups or by introducing one or more non-phosphorus internucleotides. Suitable modified internucleotides are compiled in Uhlmann and Peyman (1990), supra (see also Beigelman et al. (1995) Nucleic Acids Res. 23:3989-94; WO 95/11910; WO 98/37240; WO 97/29116). Modified internucleotide phosphate radicals and/or non-phosphorus bridges in a nucleic acid which can be employed in one of the uses according to the invention contain, for example, methyl phosphonate, phosphorothioate, phosphoramidate, phosphorodithioate and/or phosphate esters, whereas non-phosphorus internucleotide analogues contain, for example, siloxane bridges, carbonate bridges, carboxymethyl esters, acetamidate bridges and/or thioether bridges. It is also the intention that this modification should improve the durability of a pharmaceutical composition which can be employed in one of the uses according to the invention. Nucleic acids may be selected from the group consisting of a polynucleotide probe, a primer(s) (e.g. a primer pair), preferably a primcr(s) for polymerase chain reaction (PCR), reverse transcription (RT) reaction, or DNA sequencing, a peptide nucleic acid (PNA), a locked nucleic acid (LNA), a glycol nucleic acid (GNA), a threose nucleic acid (TNA), a microRNA (miRNA), and a small interfering RNA (siRNA).

The term "open reading frame" (ORF) refers to a sequence of nucleotides, that can be translated into amino acids. Typically, such an ORF contains a start codon, a subsequent region usually having a length which is a multiple of 3 nucleotides, but does not contain a stop codon (TAG, TAA, TGA, UAG, UAA, or UGA) in the given reading frame. Typically, ORFs occur naturally or are constructed artificially, i.e. by gene-technological means. An ORF codes for a protein where the amino acids into which it can be translated form a peptide-linked chain.

The term "expression level" refers to the amount of gene product present in the body or a sample at a certain point of time. The expression level can e.g. be measured/quantified/detected by means of the protein or mRNA expressed from the gene. The expression level can for example be quantified by normalizing the amount of gene product of interest present in a sample with the total amount of gene product of the same category (total protein or mRNA) in the same sample or a reference sample (e.g. a sample taken at the same time from the same individual or a part of identical size (weight, volume) of the same sample) or by identifying the amount of gene product of interest per defined sample size (weight, volume, etc.). The expression level can be measured or detected by means of any method as known in the art, e.g. methods for the direct detection and quantification of the gene product of interest (such as mass spectrometry) or methods for the indirect detection and measurement of the gene product of interest that usually work via binding of the gene product of interest with one or more different molecules or detection means (e.g. primer(s), probes, antibodies, protein scaffolds) specific for the gene product of interest. The determination of the level of gene copies comprising also the determination of the absence or presence of one or more fragments (e.g. via nucleic acid probes or primers, e.g. quantitative PCR, Multiplex ligation-dependent probe amplification (MLPA) PCR) is also within the knowledge of the skilled artisan.

In the context of the different aspects of present invention, the term "peptide" refers to a short polymer of amino acids linked by peptide bonds. It has the same chemical (peptide) bonds as proteins, but is commonly shorter in length. The shortest peptide is a dipeptide, consisting of two amino acids joined by a single peptide bond. There can also be a tripeptide, tetrapeptide, pentapeptide, etc. Preferably, the peptide has a length of up to 8, 10, 12, 15, 18 or 20 amino acids. A peptide has an amino end and a carboxyl end, unless it is a cyclic peptide.

In the context of the different aspects of present invention, the term "polypeptide" refers to a single linear chain of amino acids bonded together by peptide bonds and preferably comprises at least about 21 amino acids. A polypeptide can be one chain of a protein that is composed of more than one chain or it can be the protein itself if the protein is composed of one chain.

In the context of the different aspects of present invention, the term "protein" refers to a molecule comprising one or more polypeptides that resume a secondary and tertiary structure and additionally refers to a protein that is made up of several polypeptides, i.e. several subunits, forming quaternary structures. The protein has sometimes non-peptide groups attached, which can be called prosthetic groups or cofactors.

In the context of present invention, the primary structure of a protein is the sequence of amino acids in the polypeptide chain. The secondary structure in a protein is the general three-dimensional form of local segments of the protein. It does not, however, describe specific atomic positions in three-dimensional space, which are considered to be tertiary structure. In proteins, the secondary structure is defined by patterns of hydrogen bonds between backbone amide and carboxyl groups. The tertiary structure of a protein is the three-dimensional structure of the protein determined by the atomic coordinates. The quaternary structure is the arrangement of multiple folded or coiled protein or polypeptide molecules in a multi-subunit complex.

The individual parts of a protein may either be permanently or temporarily connected to each other. Parts of a protein that are permanently connected are translated from a single ORF and are not later separated co- or post-translationally. Parts of protein that are connected temporarily may also derive from a single ORF but are divided co-translationally due to separation during the translation process or post-translationally due to cleavage of the peptide chain, e.g. by an endopeptidase. Additionally or alternatively, parts of a protein may also be derived from two different ORF and are connected post-translationally, for instance through covalent bonds. Proteins (including protein derivatives, protein variants, protein fragments, protein segments, protein epitops and protein domains) can be further modified by chemical modification. This means such a chemically modified protein comprises other chemical groups than the 20 naturally occurring amino acids. Examples of such protein modifications include without limitation phosphorylation, glycosylation, acetylation, citrullination, ADP-ribosylation, ubiquitination, SUMOylation, and methylation.

Chemical modifications of a protein may provide altered properties as compared to the non-modified polypeptide, e.g. one or more of enhanced stability, increased biological half-life, increased water solubility, altered (e.g. increased or decreased) electrical charge, altered mechanical properties, altered substrate, ligand or interaction partner specificity, altered subcellular localization, altered transcriptional activity or altered cell signaling activity such as altered metabolic, epigenetic, apoptotic, mechanical, cell morphological or cell cycle signalling activity.

Such chemical modifications of a protein may occur co- or post-translationally.

The term "co-translational" used herein refers to events that occur during the translation process of a nucleotide triplet into an amino acid chain. Those events typically alter or modify the chemical or structural properties of the resultant amino acid chain. Examples of co-translational events include but are not limited to events that may stop the translation process entirely or interrupted the peptide bond formation resulting in two discreet translation products.

The term "post-translational" used herein refers to events that occur after the translation of a nucleotide triplet into an amino acid and the formation of a peptide bond to the proceeding amino acid in the sequence. Such post-translational events may occur after the entire peptide/polypeptide/protein was formed or already during the translation process on those parts of the peptide/polypeptide/protein that have already been translated. Post-translational events typically alter or modify the chemical or structural properties of the resultant peptide/polypeptide/protein.

Post-translational modifications include but are not limited to protein modifications such as methylation (mediated by methyltransferases and demethylases), acetylation (mediated by acetyltransferases and deacetylases), phosphorylation (mediated by kinases and phosphatases), citrullination (mediated by peptidylarginine deiminases), ADP-ribosylation (mediated by ADP-ribosyltransferases), ubiquitination (mediated by E1, E2, E3 enzyme ubiquitin cascades and isopeptidases), SUMOylation (mediated by E1, E2, E3 enzyme SUMO cascades and deSUMOylating enzymes) and glycosylation (mediated by various transferases).

For example, DNA methylation is mediated by DNA methyltransferases and takes typically place at CpG sites (cytosine-phosphate-guanine sites) where a methyl group is transferred to cytosine resulting in 5-methylcytosine. Protein methylation takes typically place at arginine and lysine residues and is mediated by peptidylarginine methyltransferases (PRMTs) and lysine methyltransferases. Arginine can be methylated once or twice, while lysine can be methylated once, twice or three times. Histones are methylated by transfer of a methyl group from S-adenosylinethionine by Histone methyltransferases.Protein acetylation typically takes place on lysine residues while acetyl-coenzyme A serves as an acetyl donor. Acetylation and deacetylation reactions are typically mediated by histone acetyltransferases and histone deacetylases, respectively.

Phosphorylation of proteins typically takes place on Serine, which is the most common target for phosphorylation, but can also take place on threonine, tyrosine, histidine and aspartate residues. Kinases thereby transfer phosphate groups to a protein, while phosphatases dephosphorylate proteins, removing phosphate groups.

Citrullination is the conversion of the amino acid arginine in a protein to citrulline as mediated by peptidylarginine deiminases which replaces an aldimine group by a ketone group.

ADP-ribosylation of proteins takes typically place on arginine, glutamic acid or aspartic acid residues where an ADP-ribose group from nicotinamide adenine dinucleotide is transferred to the protein by ADP-ribosyltransferase. The addition of multiple ADP-ribose units forming branched chains is called poly ADP-ribosylation and is mediated by poly ADP-ribose polymerases.

Ubiquitin is a small 8.5kDa protein where the last amino acid of ubiquitin, being a glycine, is attached to a lysine of a target protein mediated by ubiquitin-activating enzymes, ubiquitin-conjugating enzymes and ubiquitin ligases (El, E2 and E3 enzymes).

Small Ubiquitin-like Modifier (SUMO) are proteins that can be attached and detached to target proteins similar to ubiquitination. After a C-terminal peptide is cleaved of SUMO, the terminal glycine of SUMO is linked to a glycine of a target protein by E1, E2, E3 enzyme SUMO cascades, while deSUMOylating enzymes detach SUMO from target proteins.

Glycosylation is the attachment of a carbohydrate to a hydroxyl unit of proteins, lipids or other organic molecules mediated by various transferases. Often nucleotide sugars act as glycosyl donors in enzymatic glycosylation reactons.

The term "epigenetic" or "epigenetic modification" as used herein refers to functionally relevant modifications of the genome that do not involve a change in the nucleotide sequence. Examples of such modifications are DNA methylation and histone modification, both of which serve to regulate gene expression without altering the underlying DNA sequence. Currently known epigenetic modifications include but are not limited to RNA and DNA methylation as well as histone modifications such as the methylation, acetylation, methylation, phosphorylation, citrullination, ADP-ribosylation, ubiquitination, SUMOylation and/or glycosylation of histone.

The term "marker", "biomarker" or "indicator" are used interchangeably herein. In the context of present invention, a biomarker can be defined as a substance within a biological system that is used as an indicator of a biological state of said system. In the art, the term "biomarker" is sometimes also applied to refer to means for the detection of said endogenous substances (e.g. antibodies, nucleic acid probes etc, imaging systems). In the context of the present invention, however, the term "biomarker" shall be only applied for the substance, not for the detection means. Thus biomarkers can be any kind of molecule present in a living organism, such as a nucleic acid (DNA, mRNA, miRNA, rRNA etc.), a protein (cell surface receptor, cytosolic protein etc.), a metabolite or hormone (blood sugar, insulin, estrogen, etc.), a molecule characteristic of a certain modification of another molecule (e.g. sugar moieties or phosphoryl residues on proteins, methyl-residues on genomic DNA) or a substance that has been internalized by the organism or a metabolite of such a substance.

The term "means for detection" or "means for detecting" as used herein refers to any means suitable for specific detection of a protein or nucleic acid, especially in a sample, in isolated organic matter (e.g. isolated protein or nucleic acid), a tissue, an organ or an animal body. The detection is usually mediated by a specific binding to the target molecule, i.e. to a target protein or target nucleic acid.

The term "sample" or "sample of interest" are used interchangeably herein, referring to a part or piece of a tissue, organ or individual, typically being smaller than such tissue, organ or individual, intended to represent the whole of the tissue, organ or individual. Upon analysis a sample provides information about the tissue status or the health or diseased status of an organ or individual. Examples of samples include but are not limited to fluid samples such as blood, serum, plasma, synovial fluid, urine, saliva, and lymphatic fluid, or solid samples such as tissue extracts. Further examples of samples are cell cultures or tissue cultures such as but not limited to cultures of embryonic stcm cells (ESCs), induced pluripotent stem cells (iPSCs), primordial germ cells (PGCs), embryonic germ cells (EGCs), epiblast stem cells (EpiSCs), epiblast-like cells (EpiLC), spermatogonial stem cells (SSCs), very small embryonic-like cells (VSELs), haploid embryonic stem cells (hPSCs), and cells that express Nanog and Oct4.

The term "reference sample" as used herein, refers to a sample which is analysed in a substantially identical manner as the sample of interest and whose information is compared to that of the sample of interest. A reference sample thereby provides a standard allowing for the evaluation of the information obtained from the sample of interest. In the context of the present invention a reference sample may be a sample of the same cells taken at an earlier or later time point in case a period of time has lapsed between taking of the reference sample and taking of the sample of interest. Such period of time may represent years (e.g. 1, 2, 3, 4, 5, 10, 15, 20, 25, 30, 35, 40, 45, 50, 60, 70, 80, 90, 100 years), months (1, 2, 3, 4, 5, 6, 7, 8, 9, 10, 11, 12 months), weeks (e.g. 1, 2, 3, 4, 5, 6, 7, 8 weeks), days (e.g. 1, 2, 3, 4, 5, 10, 15, 20, 25, 30, 35, 40, 45, 50, 60, 70, 80, 90, 100, 200, 300, 400, 500 days), hours (1, 2, 3, 4, 5, 6, 7, 8, 9, 10, 11, 12 hours), minutes (e.g. 1, 2, 3, 4, 5, 10, 15, 20, 25, 30, 35, 40, 45, 50, 60 minutes), or seconds (e.g. 1, 2, 3, 4, 5, 10, 15, 20, 25, 30, 35, 40, 45, 50, 60 seconds). A reference sample may be a sample of cells exhibiting a different state of differentiation, i.e. a sample of cells which are less differentiated (i.e. exhibit a higher potency) or more differentiated (i.e. exhibit a lower potency) than the cells in the sample of interest. A reference sample may be a sample of cells exhibiting a different metabolic state, i.e. cells whose metabolism is unaltered in case the cells of the sample of interest are metabolically reprogrammed.

Epiblast stem cells (EpiSCs) can be used as a reference sample for cells in a primed pluripotent state which have a glycolytic metabolism Ground state embryonic stem cells and iPSC cells can be used as a reference sample for naive pluripotent stem cells. Spermatogonial Stem Cells (SSCs) can be used as a reference sample for spermatogenesis-competent male germ cells. Spermatocytes can be used as a reference sample for germ cells which mainly have an oxidative phosphorylative metabolism. Gonocytes can be used as a reference sample for germ cells with a glycolytic metabolism. Cardiomyocytes, hepatocytes, neurons and pancreatic beta cells can be used as reference samples for more differentiated cell types. Fibroblasts can be used as a reference sample for non-pluripotent cells. Male and female germ cells can be used as sex specific reference samples.

The terms "lowered" or "decreased" level of an indicator refer to the level of such indicator in the sample being reduced in comparison to the reference or reference sample. The terms "elevated" or "increased" level of an indicator refer to the level of such indicator in the sample being higher in comparison to the reference or reference sample.

Analysis of a sample and/or a reference sample may be accomplished on a visual or chemical basis. Visual analysis includes but is not limited to microscopic imaging or radiographic scanning of a cell, tissue, organ or individual allowing for morphological evaluation of a sample. Chemical analysis includes but is not limited to the detection of the presence or absence of specific indicators or alterations in their amount or level.

Protein detection methods include but are not limited to Western blotting and enzyme-linked immunosorbent assay (ELISA).

Western blotting allows the detection of specific proteins (native or denatured) from extracts made from cells or tissues, before or after any purification steps. Proteins are generally separated by size using gel electrophoresis before being transferred to a synthetic membrane (typically nitrocellulose or PVDF) via dry, semi-dry, or wet blotting methods. The membrane can then be probed using antibodies using methods similar to immunohistochemistry, but without a need for fixation. Detection is typically performed using peroxidase linked antibodies to catalyze a chemiluminescent reaction. Western blotting is a routine molecular biology method that can be used to semi quantitatively or quantitatively compare protein levels between extracts. The size separation prior to blotting allows the protein molecular weight to be gauged as compared with known molecular weight markers. Western blotting is an analytical technique used to detect specific proteins in a given sample of tissue homogenate or extract. It uses gel electrophoresis to separate proteins by the length of the polypeptide (denaturing conditions) or by the 3-D structure of the protein (native/ non-denaturing conditions).

The enzyme-linked immunosorbent assay or ELISA is a diagnostic method for quantitatively or semi-quantitatively determining protein concentrations from blood plasma, serum or cell/tissue extracts in a multi-well plate format (usually 96-wells per plate). Broadly, proteins in solution are adsorbed to ELISA plates. Antibodies specific for the protein of interest are used to probe the plate. Background is minimized by optimizing blocking and washing methods (as for IHC), and specificity is ensured via the presence of positive and negative controls. Detection methods are usually colorimetric or chemiluminescence based.

Suitable methods of detecting mRNA include but are not limited to Northern blot analysis, nuclease protection assays (NPA), in situ hybridization, and reverse transcription-polymerase chain reaction (RT-PCR).

For the Northern blotting procedure, RNA samples may be first separated by size via electrophoresis in an agarose gel under denaturing conditions. The RNA is then transferred to a membrane, crosslinked and hybridized with a labeled probe. Nonisotopic or high specific activity radio labeled probes can be used including random-primed, nick-translated, or PCR-generated DNA probes, in vitro transcribed RNA probes, and oligonucleotides. Additionally, sequences with only partial homology (e.g., cDNA from a different species or genomic DNA fragments that might contain an exon) may be used as probes.

The Nuclease Protection Assay (NPA) is an extremely sensitive method for the detection and quantitation of specific mRNAs. The basis of the NPA is solution hybridization of an antisense probe (radio labeled or non-isotopic) to an RNA sample. After hybridization, single-stranded, unhybridized probe and RNA are degraded by nucleases. The remaining protected fragments are separated e.g. on an acrylamide gel. Solution hybridization is typically more efficient than membrane-based hybridization, and it can accommodate up to 100 µg of sample RNA, compared with the 20-30 µg maximum of blot hybridizations. NPAs are also less sensitive to RNA sample degradation than Northern analysis since cleavage is only detected in the region of overlap with the probe (probes are usually about 100-400 bases in length).

In RT-PCR, an RNA template is copied into a complementary DNA (cDNA) using a retroviral reverse transcriptase. The cDNA is then amplified exponentially by PCR. Relative quantitative RT-PCR involves amplifying an internal control simultaneously with the gene of interest. The internal control is used to normalize the samples. Once normalized, direct comparisons of relative abundance of a specific mRNA can be made across the samples. Competitive RT-PCR is used for absolute quantitation. This technique involves designing, synthesizing, and accurately quantitating a competitor RNA that can be distinguished from the endogenous target by a small difference in size or sequence. Known amounts of the competitor RNA are added to experimental samples and RT-PCR is performed. Signals from the endogenous target are compared with signals from the competitor to determine the amount of target present in the sample.

The above methods may include nucleic acid labeling. A series of techniques are known to the skilled person allowing for labeling of DNA, RNA or oligonuleotides. These include for example Nick translational labeling, random primed DNA labeling, PCR labeling of DNA probes and oligonucleotide 3'/5' end labeling, transcriptional labeling of RNA probes, oligonucleotide 3'/5' end labeling and oligonucleotide tailing.

The nick translation method is based on the ability of DNase I to introduce randomly distributed nicks into DNA. DNA polymerase I synthesizes DNA complementary to the intact strand in a 5' → 3' direction using the 3'-OH termini of the nick as a primer. The 5' → 3' exonucleolytic activity of DNA Polymerase I simultaneously removes nucleotides in the direction of synthesis. The polymerase activity sequentially replaces the removed nuclcotidcs with isotope-labeled or hapten-labeled deoxyribonucleoside triphosphates. At low temperature (15°C), the unlabeled DNA in the reaction is thus replaced by newly synthesized labeled DNA. Common labels include digoxigenin-, biotin-, or fluorochromes such as fluorescein or tetramethylrhodamin.

The method of "random primed" DNA labeling is based on the hybridization of a mixture of all possible hexanucleotides to the DNA to be labeled. All sequence combinations are represented in the hexanucleotide primer mixture, which leads to binding of primer to the template DNA in a statistic manner. Thus an equal degree of labeling along the entire length of the template DNA is guaranteed. The complementary strand is synthesized from the 3' OH termini of the random hexanucleotide primer using Klenow enzyme, labeling grade. Modified deoxyribonucleoside triphosphates (e.g. [32P]-, [35S]-, [3H]-, [125I]-, digoxigenin- or biotin-labeled) present in the reaction are incorporated into the newly synthesized complementary DNA strand.

The polymerase chain reaction (PCR) allows the amplification of minute amounts of DNA. The only prerequisite is that some sequence information of the target sequence is known for synthesizing the appropriate primers. The combination of labeling with PCR is a powerful tool for the analysis of PCR products, and also for the preparation of labeled probes from small amounts of a respective target sequence. For example digoxigenin, a steroid hapten, may be used to label DNA, RNA, or oligonucleotides for hybridization, and subsequent color-or luminescent detection. The digoxigenin is usually coupled to dUTP via an alkali-labile ester bond. The labeled dUTP can be easily incorporated by enzymatic nucleic-acid synthesis using DNA polymerases.

Oligonucleotides may enzymatically be labeled at their 3'-end with terminal transferase either by incorporation of a label such as single digoxigenin-labeled dideoxyuridine-triphosphate (DIG-ddUTP) or by the addition of a longer nucleotide tail. Terminal Transferase catalyzes the template independent addition of deoxy- and dideoxynucleoside triphosphates to the 3'OH ends of double and single-stranded DNA fragments and oligonucleotides. Terminal transferase incorporates digoxigenin-, biotin-, and fluorochrome-labeled deoxy- and dideoxynucleotides as well as radioactive labeled deoxy-and dideoxynucleotides. Alternatively or additionally, oligonucleotides may be labeled at the 5'-terminus, e.g. by reacting with a phosphoramidite in a final step according to the classical solid phase phosphoramidite synthesis method. By this process a 5'-terminal amino function is created. Treatment with ammonia releases the oligonucleotide from the support and cleaves the protecting groups. In the subsequent step the digoxigenin moiety is introduced at the 5'-position.

Different labels are known which may be used in the above labeling methods. Some of them including their detection are exemplarily described in the following:

Biotin-labeled compounds can be detected for example by anti-biotin antibodies or by streptavidin conjugates. Anti-biotin antibodies (e.g. monoclonal anti-biotin antibody or Fab-fragment, conjugated with alkaline phosphatase (AP)) may be used in the detection of biotin-labeled nucleic acids by enzyme immunoassay with luminescence on nylon membranes. This method of detection may be employed for detection of biotin labeled nucleic acids on membranes (e.g. Southern blots, dot blots), in cells and tissues (e.g. in situ hybridization), immunoblotting, immunohistochemistry or ELISA. Streptavidin conjugates are used for the detection of biotin-labeled substances (e.g., biotinylated antibodies) which can be used for several immunological detection systems. For this, streptavidin e.g. from Strcptomyccs avidinii could be coupled to alkaline phosphatase or to ß-peroxidase. This method of detection may be employed with immunoblotting, immunohistochemistry or ELISA.

Probe-target hybrids may be detected with an enzyme-linked immunoassay. This immunochemical detection step is usually more sensitive than radioactive detection procedures. In this assay, the membrane may be blocked to prevent non-specific interaction of the antibody with the filter. Alkaline phosphatase-conjugated antibody, specific for digoxigenin, recognizes the digoxigenin molecule on the labeled hybrid. Addition of an alkaline phosphatase substrate allows the visualization of the hybrids.

For chemiluminescence detection, suitable substrates for alkaline phosphatase such as disodium 3-(4-methoxyspiro {1,2-dioxetane-3,2-(5-chloro)tricyclo [3.3.1.13,7]decan)-4-yl)phenyl phosphate or disodium 4-chloro-3-(methoxyspiro {1,2-dioxetane-3,2-(5-chloro)tricyclo [3.3.1.13,7]decan}-4-yl)phenyl phosphate belong to the group of the dioxetane phenyl phosphates. Upon dephosphorylation by alkaline phosphatase, an intermediate is formed whose decomposition results in light emission which can be recorded e.g. on X-ray film.

Colorimetric detection of DIG-labeled probes is usually performed with colorless substrates which form a redox system. Examples are like 5-bromo-4-chloro-3-indolyl-phosphate and 4-Nitro-blue-tetrazolium-chloride. 5-bromo-4-chloro-3-indolyl-phosphate is oxidized by the alkaline phosphatase to indigo by release of a phosphate group. In parallel, 4-Nitro-blue-tetrazolium-chloride is reduced to diformazan. The reaction products form a water insoluble dark blue to brownish precipitate, depending on the type of membrane.

Various reporter molecules can be coupled to detecting antibodies to visualize the specific probe-target hybridization including, but not limited to, enzyme-coupled antibodies, fluorochrome-labeled antibodies (detection by fluorescent microscope and specific filters which allow visualization of the wavelength emitted by the fluorescent dye) and antibodies coupled to colloidal gold (detection by electron microscope on cryostatic sections).

Multiple simultaneous hybridizations can be performed by using combinations of digoxigenin-, biotin- and fluorochrome-labeled probes to localize different chromosomal regions or different RNA sequences in one preparation. Such multiprobe experiments are made possible by the availability of different fluorescent dyes coupled to antibodies. These include fluorescein or FITC (fluorescein isothiocyanate; yellow), rhodamine or TRITC (tetramethylrhodamine isothiocyanate; red) and AMCA (amino-methylcoumarin acetic acid; blue).

The term "cell" as used herein refers to an autonomous self-replicating unit that may exist as functional independent unit of life (as in the case of unicellular organism), or as sub-unit in a multicellular organism (such as in plants and animals) that is specialized into carrying out particular functions towards the cause of the organism as a whole. Cells are membrane bound structure containing biomolecules including but not limited to nucleic acids, proteins, and polysaccharides. There are two distinct types of cells: prokaryotic cells (e.g. bacterial cells) and eukaryotic cells (e.g. fungi, plant or animal cell). As used herein, a cell or a stem cell is derived from any mammal, bird, reptile, amphibian or fish, i.e. preferably a cell or stem cell according to the present invention is a mammal cell, bird cell, reptile cell, amphibian cell or fish cell. Typically, a cell is a cell derived from an animal selected from the group consisting of laboratory animals (e.g. mouse, rat, hamster or frog), domestic animals (including e.g. guinea pig, rabbit, horse, donkey, cow, sheep, goat, pig, chicken, duck, camel, cat, dog, turtle, tortoise, snake, or lizard), or primates including chimpanzees, bonobos, gorillas and human beings. It is particularly preferred that the "subject" is a human being. Thus, typically a cell according to the present invention is selected from the group consisting of laboratory animal cells (e.g. mouse cells, rat cells, hamster cells or frog cells ), domestic animal cells (including e.g. guinea pig cells, rabbit cells, horse cells, donkey cells, cow cells, sheep cells, goat cells, pig cells, chicken cells, duck cells, camel cells, cat cells, dog cells, turtle cells, tortoise cells, snake cells, or lizard cells), or primate cells including chimpanzee cells, bonobo cells, gorilla cells and human cells.

Typically multicellular organisms contain cells specialised for different functions. Most distinct cell types arise from a single totipotent cell that differentiates into hundreds of different cell types during the course of development.

The term "stein cell" as used herein refers to an undifferentiated cell of a multicellular organism which has the potential to differentiate into cell types of all three germ layers as well as into germ cells. The term "stem cell" as used herein includes but is not limited to omnipotent stem cells, pluripotent stem cells, multipotent stem cells, oligopotent stem cells, and unipotent cells. The more cell types a stem cell can differentiate into, the greater its "potency".

Omnipotent (totipotent) cells are the zygote created by the fusion of an egg and a sperm cell, including the cells produced by the first few divisions of the zygote. Omnipotent cells can be isolated by dissociating blastomeres (cells created by cleavage of the zygote). Omnipotent stem cells can differentiate into embryonic and extraembryonic cell types and are thus able to construct a complete, viable organism.

Pluripotent stem cells are the descendants of omnipotent cells and can differentiate into all cells derived from any of the three germ layers, i.e. into the cells of the endoderm (interior stomach lining, gastrointestinal tract, the lungs), mesoderm (muscle, bone, blood, urogenital), and ectoderm (epidermal tissues and nervous system). Exemplified, pluripotent, embryonic stem cells originate from the inner mass cells within a blastocyst. The stem cells can become any tissue in the body, excluding a placenta. Pluripotent stem cells express the core pluripotency factors Nanog and Oct4, as well as typically Sox2, EsrrB, Nr5a2, Klf4 and Tbx3. Pluripotent stem cells in the naive state of pluripotency (corresponding to the inner cell mass of the blastocyst) express Dppa3, Dazl, Stra8, Piwil2, Prdm14, Rex1, Dax1, Fbxo15 and the cell surface marker SSEA1, while pluripotent stem cells in the primed state (corresponding to the implanting and post-implantation epiblast) show reduced expression of naive pluripotency markers and increased expression of Nodal, Eomes, Gata6, Foxa2, Cer1 and the later germ layer markers for mesoderm (T, brachyury), ectoderm (Fgf5) and endoderm (Sox17). Besides the inner cell mass of the blastocyst there is evidence that pluripotent stem cells could also be found in the adult body in the bone marrow in the form of very small embryonic like cells (VSELs) which have been reported to express the cell surface marker CD133 (prominin-1) by which they could be isolated. The existence of VSELs however is still under debate.

Multipotent stem cells are able to differentiate into a number of different cell types, however only into those cell types of a closely related family. Exemplified, hematopoietic cells are multipotent blood stem cell which are able to differentiate into several types of blood cell types (e.g. lymphocytes, monocytes, neutrophils) but are not able to differentiate into brain cells, bone cells or other non-blood cell types. Multipotent stem cells include among others hematopoietic stem cells and mesenchymal stem cells. Mesenchymal stem cells are mostly located in the bone marrow. Human mesenchymal stem cells and are positive for CD105, CD166, CD29, and CD44, while testing negative for CD14, CD34 and CD45. Hematopoietic stem cells are also located in the bone marrow and are positive for CD90 and CD133, while differences exist between species such as human and mouse. For example, mouse hematopoietic stem cells are low in expression of CD34, but high in expression of CD38, while human the opposite case is true for human hematopoietic stem cells.

Oligopotent stem cells are only able to differentiate into a limited number of cell types. Examples of oligopotent stem cells include but are not limited to lymphoid and myeloid stem cells. Oligopotent cells are distributed in various tissues and organs depending on the cell types in which they can differentiate.

Unipotent cells can produce only one cell type, their own, but have the property of self-renewal, which distinguishes them from non-stem cells. Unipotent cells include but are not limited to progenitor cells and muscle stem cells.

An induced pluripotent stem cells (iPSCs) is a pluripotent stem cell artificially derived from a non-pluripotent cell, typically an adult somatic cell, by inducing the expression of specific genes and transcription factors. Induced pluripotent stem cells can be divided typically into two classes of pluripotent stem cells, naive pluripotency or primed pluripotency with the expression of respective markers. While most mouse iPSCs are characterized as being in a naïve state of pluripotency, most human iPSCs are described in a primed state of pluripotency. It is assumed that this difference has epigenetic causes. However, human iPSCs can be brought into a naive state of pluripotency resembling mouse iPSCs, by changing culture conditions such as the depletion of Mbd3 which is a member of the Mbd3/NuRD (nucleosome remodelling and deacetylation) repressor complex. Mouse iPSCs can be brought into a primed state by stimulation of Activin/Nodal and FGF signaling.

The term "differentiated cell" as used herein refers to a cell which is derived from a stem cell and which turns driven by different environmental cues (such as e.g. cell-cell interaction, exposure to hormones or other signalling molecules) and intrinsic differences (such as e.g. those caused by the uneven distribution of molecules during division), into a specialized cell able to fulfil a specific function within the organism. For instance, the human body comprises about 300 different cell types. Cells of the human body derived from the endoderm include but are not limited to exocrine secretory epithelial cells (e.g. salivary gland, mammary gland, prostate gland, and sweat gland cells), and hormone secreting cells (e.g. gut and respiratory tract cells, thyroid gland cells). Cells of the human body derived from the ectoderm include but are not limited to cells of the integumentary system (e.g. epithelial cell such as keratinizing epithelial cells) and the nervous system (e.g. Sensory transducer cells, Autonomic neuron cells, Sense organ and peripheral neuron supporting cells, Central nervous system neurons and glial cells, and Lens cells). Cells of the human body derived from the mesoderm include but are not limited to metabolism and storage cells, barrier function cells (e.g. cells of the lung, gut, exocrine glands and urogenital tract), extracellular matrix cells (e.g. fibroblasts, condrocytes, osteoblasts, osteocytes), contractile cells (e.g. skeletal muscle cells, smooth muscle cells heart muscle cells, myoepithelial cells), blood and immune system cells (e.g. Erythrocyte, B cells, T cells, monocytes, neutrophil granulocyte, eosinophil granulocyte, basophil granulocyte, mast cells, dendritic cells), germ cells (e.g. oogonium/oocyte, spermatid, spermatocyte, spermatozoon), nurse cells (e.g. Ovarian follicle cell, Thymus epithelial cell), and interstitial cells.

Examples of differentiated human cell types include but are not limited to cardiomyocytes, cardiac pacemaker cells, skeletal muscle cells, smooth muscle cells, vascular smooth muscle cells, endothelial cells, kidney glomerulus parietal cells, kidney glomerulus podocytes, kidney proximal tubule brush border cells, loop of Henle thin segment cells, thick ascending limb cells, kidney distal tubule cells, kidney collecting duct cells, interstitial kidney cells, hepatocytes, sinusoidal hepatic endothelial cells, hepatic stellate cells, kupffer cells, neurons, pyramidal cells, basket cells, betz cells, medium spiny neurons, purkinje cells, renshaw cells, lugaro cells, unipolar brush cells, granule cells, anterior horn cells, spindle cells, schwann cells, satellite cells, olfactory ensheathing cells, retinal ganglion cells, retinal cone cells, retinal rod cells, langerhans cells, melanocytes, epithelial cells, fibroblasts, keratinocytes, merkel cells, hair follicle cells, osteoblasts, osteoclasts, macrophages, neutrophils, dendritic cells, eosinophils, mast cells, basophils, natural killer cells, lymphocytes, T cells, B cells, hematopoietic stem cells, mesenchymal stem cells, pancreatic α cells, pancreatic β cells, pancreatic gamma cells and pancreatic delta cells.

The terms "metabolism" or "cell metabolism" or "metabolic reaction" are used interchangeably herein referring to the set of life-sustaining chemical transformations within the cells of living organisms based on enzyme-catalyzed reactions. In the context of the present application the term cell metabolism also includes the antioxidant system and the redox system of the cell. Examples of metabolic reactions include but are not limited to reactive oxygen species (ROS) signalling, cellular glycolysis, oxidative phosphorylation, pentose phosphate pathway, glycogen metabolism and/or the antioxidant systems.

Reactive oxygen species (ROS) are chemically reactive molecules containing oxygen. The term "ROS signalling" refers to the process of ROS generated during aerobic metabolism typically by oxidative phosphorylation, which act as second messengers in cellular signalling. ROS are essential regulators of cellular metabolism and are generated in virtually all cells either by the mitochondrial electron transport chain or by NADPH oxidase. Oxidative phosphorylation is required for aerobic metabolism. During the process of oxidative phosphorylation the oxidoreduction energy generated over the mitochondrial electron transport chain is bound in a high energy phosphate group in the form of ATP. Cytochrome c oxidase is the final component in the electron transport chain and catalyzes the reduction of oxygen (O₂) to water (H₂O), where oxygen serves as the final electron acceptor. However incomplete reduction of oxygen does also occur and leads to the generation of highly reactive oxygen metabolites which include superoxide radicals (O₂⁻) and hydrogen peroxide (H₂O₂), while hydroxyl radicals (OH•) can form in the presence of transition metal ions. These partially reduced oxygen species are described as ROS. If unchecked by antioxidative enzyme systems of the cells ROS can have deleterious effects and lead to cellular damage, aging and cell death. However, ROS are also involved in non-deleterious cellular processes and serve an important regulatory role in the cell. For example oxidation of transcription factors by hydrogen peroxide can lead to a conformational change and direct activation of gene expression. The current paradox of ROS signalling is that too much ROS damage the cell by oxidation of vital cellular components, but a lack of or too little ROS impairs important physiological funtions and cellular signalling mechanisms. Therefore, ROS signalling is a highly regulated and balanced system in the cell.

The term "cellular glycolysis" refers to the metabolic pathway that converts glucose C₆H₁₂O₆, into pyruvate, CH₃COCOO- + H+. The free energy released in this process is used to form the high-energy compounds ATP (adenosine triphosphate) and NADH (reduced nicotinamide adenine dinucleotide). Enzymes involved in glycolysis include hexokinase, phosphofructokinase, pyruvate kinase, lactate dehydrogenase, pyruvate dehydrogenase kinase. In the first step of glycolysis, glucose is phosphorylated by hexokinase (HK) to form glucose 6-phosphate (G6P). G6P is then rearranged into fructose 6-phosphate (F6P) by glucose phosphate isomerase. Fructose can also enter the glycolytic pathway by phosphorylation at this point. Phosphofructokinase 1 (PFK1) catalyzes the phosphorylation of F6P to form fructose 1,6-bisphosphate (F1,6BP). This step is virtually irreversible and represents the rate limiting step in the glycolytic process. Aldolase splits the hexose ring of F1,6BP into two triose sugars, dihydroxyacetone phosphate (DHAP) and glyceraldehyde 3-phosphate (GADP). triosephosphate isomerase (TPI) interconverts DHAP with GADP, wich proceeds further in glycolysis. The triose sugars are dehydrogenated and inorganic phosphate is added to them, forming 1,3-bisphosphoglycerate catalysed by glyceraldehyde phosphate dehydrogenase (GAPDH). Phosphoglycerate kinase (PGK) transfers a phosphate form 1,3-bisphosphoglycerate to ADP forming ATP and 3-phosphoglycerate. phosphoglycerate mutase (PGM) catalyzes the formation of 2-phosphoglycerate. Then, enolase (ENO) catalyzes the formation of phosphoenolpyruvate from 2-phosphoglycerate. In a final step, pyruvate is formed from by dephosphorylation of phosphoenolpyruvate catalysed by pyruvate kinase (PK).Glycolysis does not only serve the production of ATP, but the intermediate molecules of glycolysis can serve as important metabolic intermediates for biosynthetic pathways such as lipid, nucleotide and amino acid biosynthesis. Therefore, for certain cellular functions a high glycolytic flux is a prerequisite. It is for example known that naïve pluripotent stem cells need to switch to a highly glycolytic metabolism in order to transition into the epiblast stage.

Lactate dehydrogenase (Ldha) catalyzes the conversion of pyruvate, the final product of glycolysis, to lactate. Pyruvate dehydrogenase kinase 1 (Pdkl) inactivates the enzyme pyruvate dehydrogenase by phosphorylating it. Pyruvate dehydrogenase complex converts pyruvate to acetyl-coA, which is then oxidized in the mitochondria to produce energy. By downregulating the activity of this complex, Pdk1 decreases the oxidation of pyruvate in mitochondria and increase the conversion of pyruvate to lactate. Both Ldha and Pdk1 are enzymes that act at the last part of glycolysis and lead to an increase in lactate production. They are good markers for the quantification of glycolysis since lactate serves as a way the cell can dispose the product of a glycolytic flux that is higher than the energy needs of the cell. Therefore by measuring enzymes involved in increased lactate production, the amount of cellular glycolysis can be estimated.

The term "oxidative phosphorylation" refers to the metabolic pathway in which the mitochondria in cells use their structure, enzymes, and energy released by the oxidation of nutrients to reform ATP. Oxidative phosphorylation harbors the electron transport chain consisting of complex I (NADH dehydrogenase), complex II (succinate dehydrogenase), complex III (cytochrome bc1 complex) and complex IV (cytochrome c oxidase). The enzyme complexes I to IV represent the electron transport chain found in the inner cell membrane of the mitochondrion. Enzymes in the electron transport chain use the energy from the oxidation of NADPH to pump protons across the inner membrane of the mitochondrion. The build-up of protons in the mitochondrial inter-membrane space causes an electrochemical gradient to build up across the mitochondrial membrane which is then used by ATP synthase to generate ATP. NADH-coenzyme Q oxidoreductase (complex I) catalyzes the two electron oxidation of NADH by ubiquinone (coenzyme Q10). As the electrons pass through this complex, protons are pumped from the matrix into the intermembrane space. Succinate-Q oxidoreductase (complex II) oxidizes succinate to fumarate and reduces ubiquinone which releases less energy than the oxidation of NADH. Complex II does not transfer protons across the membrane. Electron transfer flavoprotein-Q oxidoreductase is another entry point in the electron transport chain and accepts electrons from electron-transferring flavoprotein in the mitochondrial matrix to reduce ubiquinone. Q-cytochrome c oxidoreductase (complex III) catalyzes the oxidation of one molecule of ubiquinol and the reduction of two molecules of cytochrome c. A cytochrome is an electron-transferring protein which carries at least one heme group. Ubiquinol is formed on the inner side of the membrane and ubiquinone on the outer, causing a net transfer of protons across the membrane which contributed to the proton gradient. Cytochrome c oxidase (complex IV) mediates the final reaction in the electron transport chain and transfers electrons to oxygen, while pumping protons across the membrane. Oxygen as the final electron acceptor is reduced to water (H₂O). Incomplete reduction of oxygen generates highly reactive oxygen radicals which are described as reactive oxygen species (ROS).

The term "pentose phosphate pathway" refers to the metabolic process that generates NADPH and pentoses. In a first oxidative phase NADPH is generated, while in a second phase, the non-oxidative synthesis of pentoses takes place. glucose 6-phosphate dehydrogenase (G6pd), 6-phosphogluconolactonase (Pgls) and 6-phosphogluconate dehydrogenase (Pgd) catalyze the conversion of glucose-6-phosphate (G6P) into ribulose 5-phosphate (R5P) and utilizes the generated energy to reduce NADP+ to NADPH. R5P can be further transformed into metabolites of glycolysis or used for the biosynthesis of nucleotides (DNA and RNA) or used for the production of coenzymes (ATP, coenzyme A, NAD or FAD). NADPH serves as areducing agent and maintains a reducing environment withing the cellular cytoplasm. In the non-oxidative part of the pentose phosphate pathway C4, C5, C6 and C7 carbohydrates can be interconverted by the action of transketolase (Tkt) and transaldolase (Taldo). Transaldolase transfers a C3 unit, while transketolase transfers a C2 unit. Ribulose 5-Phosphate Isomerase transforms ribulose 5-phosphate into ribose 5-phosphate, while ribulose 5-Phosphate 3-Epimerase can transform ribulose 5-phosphate into xylulose 5-phosphate. Transketolase can then transfer one C2 units from xylulose 5-phosphate to ribose 5-phosphate creating glyceraldehyde 3-phosphate and sedoheptulose 7-phosphate. Transaldolase can transfer a C3 unit from sedoheptulose 7-phosphate to glyceraldehyde 3-phosphate creating erythrose 4-phosphate and fructose 6-phosphate. Finally, a transketolase can then transfer a C2 unit from xylulose 5-phosphate to erythrose 4-phosphate creating glyceraldehyde 3-phosphate and fructose 6-phosphate

The term "glycogen metabolism" refers to the metabolic pathways in which glycogen is synthesised (glycogenesis) or degraded (glycogenolysis). For instance the enzymes glycogen phosphorylase and glycogen synthase are involved in glycogen metabolism. Glycogen synthase (Gys1) catalyzes the synthesis of glycogen from monomers of UDP-glucose, progressively lengthening the glycogen chain with α1→4 bonded glucose. The glycogen-branching enzyme, amylo (α1-4) to (α1-6) transglycosylase, catalyzes the transfer of glucose residues into the interior of the glycogen chain. Glycogen phosphorylase (Pygl) catalyzes the cleavage of glycogen from the nonreducing ends of the chain to produce monomers of glucose-1-phosphate, which is then converted to glucose 6-phosphate (G6P) by phosphoglucomutase. A debranching enzyme removes the alpha(1-6) branches in branched glycogen to transform the chain into a linear polymer. The G6P monomers produced can enter the glycolysis pathway or the pentose phosphate pathway.

The term "antioxidant systems" refers to ... Cellular antioxidant systems include enzymes such as superoxide dismutases, glutathione peroxidases, glutathione reductases, catalases, NADPH dehydrogenase quinones, hemeoxygenases and master regulators of antioxidant responses such as Nrf2.

Superoxide dismutases (SODs) catalyse the breakdown of superoxide (O₂) into oxygen and hydrogen peroxide, while catalases or peroxyredoxins further reduce hydrogen peroxide (H₂O₂ to water (H₂O). Pcroxyrcdoxins also catalyse the reduction of organic hydroperoxides and peroxynitrite. Sulfiredoxin 1 (Srxn1) contributes to oxidative stress resistance by reducing cysteine-sulfinic acid formed under exposure to oxidants in peroxiredoxins.

Thioredoxin systems consist of thioredoxins and thioredoxin reductases, which can regenerate thioredoxins. In its active state, thioredoxin works as an efficient reducing agent and scavenges reactive oxygen species, which helps to maintain other proteins in their reduced state. After being oxidized, the active thioredoxin is regenerated by the action of thioredoxin reductase, using NADPH as an electron donor.

The glutathione system comprises glutathione and glutathione peroxidase. Glutathione is an important antioxidant, since it's thiol group allows it to act as reducing agent and can be reversibly oxidized and reduced. Glutathione peroxidase catalyzes the oxidation of glutathione which reduces radicals such as hydrogen peroxidation products. Glutamate-cysteine ligase (Gclc) is the first rate limiting enzyme of glutathione synthesis. Glutathione S-Transferase Mu 1 (Gstm1) catalyzes the conjugation of reduced glutathione with potentially toxic substances, and thereby helps in the detoxification process of cells.

NAD(P)H dehydrogenase [quinone] 1 (Nqo1) prevents the one electron reduction ofquinones that results in the production of radical species and therefore plays an importantn role in detoxification processes.

The Nrf2-Keap1 system represents a cellular antioxidant defense system as well as a sensor for cellular redox status. Normally Nrf2 is bound by Keap1 in the cytoplasm and remains inactive. However, when electrophilic compounds covalently modify Keap1, Nrf2 is released and is translocated into the nucleus where it binds to antioxidant response elements (AREs) in the promoter region of antioxidant and detoxifying enzymes such as Nqo1, Srxnl, Gclc, Gclm and Gstm1. Nrf2 also has been shown to activate transcription of enzymes of the pentose phosphate pathway including G6pd, Pgd, Taldo1 and Tkt as well as NADPH generating enzymes Me1 and Idh1.

The term "re-programming" refers to the process of altering an existing system, e.g. such as altering a specific system in the cell. Re-programming may occur naturally or may be induced artificially. For instance in the process of cell differentiation stem cells are re-programmed to turn into differentiated somatic cells. Said re-programming may occur naturally during the development of a multicellular organism. Artificially, typically via biotechnological means, somatic cells may be re-programmed to loose their differentiated state and to turn into multi-or even pluripotent cells (e.g. into induced pluripotent stem cells (iPSC)).

In the context of the present invention the term "conditioning" refers to the process of preparing a cell for a subsequent treatment. For instance, cells may be conditioned to make them responsive for subsequent re-programming to which they would not respond without being conditioned previously.

As used in the context of the present invention, the term "metabolic reprogramming" refers to processes wherein the metabolism of a cell is altered artificially, typically via biotechnological means. Typically, cells are metabolically reprogrammed by altering one or more of the metabolic reactions in the cell including but are not limited to reactive oxygen species (ROS) signalling, cellular glycolysis, oxidative phosphorylation, pentose phosphate pathway, glycogen metabolism and/or the antioxidant systems. Metabolic reprogramming may be achieved by influencing one or more of the molecules, i.e. proteins, nucleic acids or sugar molecules, involved in the respective metabolic reaction. Metabolic reprogramming may result in a increased or decreased presence or activity of molecules involved in the metabolic reaction. Metabolic reprogramming may be effected by siRNAs, shRNAs, agonistic or antagonistic peptides, genetic manipulation, epigenetic manipulation, and pharmacologic compounds targeting one or more molecules involved in the respective metabolic reaction.

For instance metabolic reprogramming of ROS signalling pathways may be affected by redox cycling agents which generate superoxide radicals such as quinones like hydroquinone, paraquat, menadione, ROS inducers like methyl 3-(4-nitrophenyl) propiolate or metal ions such as iron.

Metabolic reprogramming of the cellular glycolysis may occur by but is not limited to treatments with inhibitors of aerobic glycolysis. This includes hexokinase inhibitors like Bromopyruvic acid, 2-Deoxy-D-glucose or Lonidamine mitochondrial hexokinase inhibitor or inhibitors of lactate dehydrogenase (Ldha) like (±)-Gossypol or Sodium oxamate or inhibitors of pyruvate dehydrogenase kinase (PDK) like (+)-Dehydroabietylamine, Dichloroacetic acid, Potassium dichloroacetate or Sodium dichloroacetate. Inhibition of glycolysis may have anti-cancer effects, since most cancer cells are highly glycolytic and also rely heavily on glycolysis for survival.

Metabolic reprogramming of the oxidative phosphorylation may occur by but is not limited to treatments with inhibitors of oxidative phosphorylation like malonate, oxaloacetate, rotenone, cyanide, oligomycin or 2,4-Dinitrophenol. Oxidative phosphorylation enhancers include glucose deprivation, rapamycin, galactose or dexamethasone. Enhancers of oxidative phosphorylation may have anti-cancer effects, since most cancer cells are highly glycolytic and also rely heavily on glycolysis for survival.

Metabolic reprogramming of the pentose phosphate pathway may be affected by inhibitors of pentose phosphate pathway like 6-aminonicotinamide (6-AN), epiandrosterone (EPI), and dehydroepiandrosterone (DHEA) or inducers of pentose phosphate pathway including xenobiotics like trans-stilbene oxide or benzil.

Metabolic reprogramming of the glycogen metabolism may occur by but is not limited to treatments with inhibitors of glycogen synthase kinase 3 (GSK3) like SB-216763, CHIR 99021 or BIO which increase glycogen synthase activity, but may have more effects on the cell since not all their targets are known. GSK3 inhibitors arc used to maintain PSCs in the naïve state of pluripotency. Glycogen phosphorylase inhibitors such as FR258900 may be used to suppress glycogen breakdown which may limit availability of substrates for glycolysis and stimulate oxidative phosphorylation.

Metabolic reprogramming of antioxidant systems may occur by but is not limited to treatments with synthetic superoxide dismutase/catalase mimetics like EUK 134, or other direct antioxidants, like α-tocopherol, vitamin C, n-acetyl-cysteine, lipoic acid or stilbene glycosides may be used to change antioxidant status of cells.

The term "tissue" as used herein, refers to an ensemble of cells of the same origin which fulfil a specific function concertedly. Examples of a tissue include but are not limited to bone, cartilage, connective tissue, muscle tissue, nervous tissue, and epithelial tissue. Multiple tissues together form an "organ" to carry out a specific function. Examples of an organ include but are not limited to joint, skeleton, muscle, blood, brain, heart, liver, kidney, stomach, and skin.

As used herein, a "subject" means any mammal, bird, reptile, amphibian or fish that may benefit from the present invention. Preferably, an individual is selected from the group consisting of laboratory animals (e.g. mouse, rat, hamster or frog), domestic animals (including e.g. guinea pig, rabbit, horse, donkey, cow, sheep, goat, pig, chicken, duck, camel, cat, dog, turtle, tortoise, snake, or lizard), or primates including chimpanzees, bonobos, gorillas and human beings. It is particularly preferred that the "subject" is a human being.

The term "disease" and "disorder" are used interchangeably herein, referring to an abnormal condition, especially an abnormal medical condition such as an illness or injury, wherein a tissue, an organ or an individual is not able to efficiently fulfil its function anymore. Typically, but not necessarily, a disease is associated with specific symptoms or signs indicating the presence of such disease. The presence of such symptoms or signs may thus, be indicative for a tissue, an organ or an individual suffering from a disease. An alteration of these symptoms or signs may be indicative for the progression of such a disease. A progression of a disease is typically characterised by an increase or decrease of such symptoms or signs which may indicate a "worsening" or "bettering" of the disease. The "worsening" of a disease is characterised by a decreasing ability of a tissue, organ or organism to fulfil its function efficiently, whereas the "bettering" of a disease is typically characterised by an increase in the ability of a tissue, an organ or an individual to fulfil its function efficiently. A tissue, an organ or an individual being at "risk of developing" a disease is in a healthy state but shows potential of a disease emerging. Typically, the risk of developing a disease is associated with early or wcak signs or symptoms of such disease. In such case, the onset of the disease may still be prevented by treatment. Examples of a disease include but are not limited to traumatic diseases, inflammatory diseases, infectious diseases, cutaneous conditions, endocrine diseases, intestinal diseases, neurological disorders, joint diseases, genetic disorders, autoimmune diseases, and various types of cancer.

"Symptoms" of a disease are implication of the disease noticeable by the tissue, organ or organism having such disease and include but are not limited to pain, weakness, tenderness, strain, stiffness, and spasm of the tissue, an organ or an individual. "Signs" or "signals" of a disease include but are not limited to the change or alteration such as the presence, absence, increase or elevation, decrease or decline, of specific indicators such as biomarkers or molecular markers, or the development, presence, or worsening of symptoms.

The terms "pharmaceutical", "medicament" and "drug" are used interchangeably herein referring to a substance and/or a combination of substances being used for the identification, prevention or treatment of a tissue status or disease.

"Pharmaceutically acceptable" means approved by a regulatory agency of the Federal or a state government or listed in the U.S. Pharmacopeia or other generally recognized pharmacopeia for use in animals, and more particularly in humans.

The term "active ingredient" refers to the substance in a pharmaceutical composition or formulation that is biologically active, i.e. that provides pharmaceutical value. A pharmaceutical composition may comprise one or more active ingredients which may act in conjunction with or independently of each other. The active ingredient can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as but not limited to those derived from sodium, potassium, ammonium, calcium, ferric hydroxides, isopropylamine, triethylamine, 2-ethylamino ethanol, histidine, procaine, and the like.

The terms "preparation" and "composition" are intended to include the formulation of the active compound with encapsulating material as a carrier providing a capsule in which the active component with or without other carriers, is surrounded by a carrier, which is thus in association with it.

The term "carrier", as used herein, refers to a pharmacologically inactive substance such as but not limited to a diluent, excipient, or vehicle with which the therapeutically active ingredient is administered. Such pharmaceutical carriers can be liquid or solid. Liquid carrier include but are not limited to sterile liquids, such as saline solutions in water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions. A saline solution is a preferred carrier when the pharmaceutical composition is administered intravenously. Examples of suitable pharmaceutical carriers are described in "Remington's Pharmaceutical Sciences" by E. W. Martin.

Suitable pharmaceutical "excipients" include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

The term "adjuvant" refers to agents that augment, stimulate, activate, potentiate, or modulate the immune response to the active ingredient of the composition at either the cellular or humoral level, e.g. immunologic adjuvants stimulate the response of the immune system to the actual antigen, but have no immunological effect themselves. Examples ofsuch adjuvants include but are not limited to inorganic adjuvants (e.g. inorganic metal salts such as aluminium phosphate or aluminium hydroxide), organic adjuvants (e.g. saponins or squalene), oil-based adjuvants (e.g. Freund's complete adjuvant and Freund's incomplete adjuvant), cytokines (e.g. IL-1β, IL-2, IL-7, IL-12, IL-18, GM-CFS, and INF-γ) particulate adjuvants (e.g. immuno-stimulatory complexes (ISCOMS), liposomes, or biodegradable microspheres), virosomes, bacterial adjuvants (e.g. monophosphoryl lipid A, or muramyl peptides), synthetic adjuvants (e.g. non-ionic block copolymers, muramyl peptide analogues, or synthetic lipid A), or synthetic polynucleotides adjuvants (e.g polyarginine or polylysine).

As used herein, a "patient" or "subject" means any mammal, bird, reptile, amphibian or fish that may benefit from a treatment with a tumour vaccine described herein. Preferably, a "patient" is selected from the group consisting of laboratory animals (e.g. mouse, rat, hamster or frog), domestic animals (including e.g. guinca pig, rabbit, horse, donkey, cow, sheep, goat, pig, chicken, camel, cat, dog, turtle, tortoise, snake, or lizard), or primates including chimpanzees, bonobos, gorillas and human beings. It is particularly preferred that the "patient" is a human being.

As used herein, "treat", "treating" or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence ofthe disorder(s) in patients that have previously had the disorder(s); and (e) limiting or preventing recurrence of symptoms in patients that were previously symptomatic for the disorder(s).

As used herein, "prevent", "preventing", "prevention", or "prophylaxis" of a disease or disorder means preventing that such disease or disorder occurs in patient.

As used herein, "administering" includes in vivo administration, as well as administration directly to tissue ex vivo, such as vein grafts.

An "effective amount" or "therapeutically effective amount" is an amount of a therapeutic agent sufficient to achieve the intended purpose. The effective amount of a given therapeutic agent will vary with factors such as the nature of the agent, the route of administration, the size and species of the animal to receive the therapeutic agent, and the purpose of the administration. The effective amount in each individual case may be determined empirically by a skilled artisan according to established methods in the art.

### EMBODIMENTS

The present invention relates to the re-programming of pluripotent stem cells (PSCs) comprising at least two steps, the transformation of PSCs into epigenetically and optionally post-translationally conditioned PSCs (cPSCs) (step a) and the transformation of the conditioned cPSCs into metabolically reprogrammed PSCs (pPSCs) (step b).

Thus, in a first aspect the present invention relates to method of reprogramming pluripotent stem cells (PSC) comprising the steps
(a) conditioning PSCs epigenetically, and
(b) metabolically reprogramming PSCs.

Preferably, in step (a) the PSCs are conditioned into cPSCs (conditioned pluripotent stem cells), preferably to prepare the cell for a subsequent treatment, i.e. to make them responsive for the metabolic reprogramming of step (b). Preferably, in step (b) the cPSCs are metabolically reprogrammed into pPSCs (reprogrammed pluripotent stem cells).

The method of the first aspect of the present invention offers the technical solution to major clinical challenges. The obtained pPSCs are non-tumorigenic while still retaining their pluripotency representing a solution to the tumorigenicity problem of PSCs. This represents a major enabling technology for the clinical application ofPSCs in all fields ofmedicine.

Thus, in preferred embodiments of the first aspect of the present invention, the tumorigenicity of the PSCs is eliminated.

The method of the first aspect of the present invention further allows to reversibly arrest cell proliferation of PSCs in their pluripotent state over long time periods. This is a valuable tool for tissue engineering and stem cell research. Furthermore, the method of the first aspect of the present invention allow to transport the pPSCs for longer times such as days without having to freeze the cells.

Thus, in preferred embodiments of the first aspect of the present invention, the proliferation of the PSCs is arrested reversibly.

In further embodiments, step (a) of the method of the first aspect of the present invention, further comprises that the PSCs are conditioned post-translationally.

The metabollic reprogramming of step (b) may occur simultaneously or subsequentially to the conditioning of the PSCs in step (a). In preferred embodiments, the pluripotent cells are metabolically reprogrammed subsequent to being conditioned epigenetically and optionally being conditioned post-translationally.

In embodiments of the first aspect of the present invention, the pluripotent cells are conditioned epigenetically over one or more cell passages, preferably over 1 to 50 cell passages (i.e. over 1, 2, 3, 4, 5, 6, 7, 8, 9, 10, 11, 12, 13, 14, 15, 16, 17, 1.8, 19, 20, 21, 22, 23, 24, 25, 26, 27, 28, 29, 30, 31, 32, 33, 34, 35, 36, 37, 38, 39, 40, 41, 42, 43, 44, 45, 46, 47, 4-8, 49, or 50 cell passages).In preferred embodiments, the pluripotent cells are conditioned epigenetically over 1 to 30 cell passages, more preferably over 5 to 20 cell passages, most preferably over 10 cell passages.

In embodiments of the first aspect of the present invention, the pluripotent cells are conditioned epigenetically over one or more days, preferably over 1 to 50 days (i.e. over 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 days). In preferred embodiments, the pluripotent cells are conditioned epigenetically over 1 to 20 days, more preferably over 5 to 10 days, most preferably over 7 days.

In embodiments of the first aspect of the present invention, the PSCs are epigenetically conditioned by altering histone acetylation, and/or by altering genome methylation, more preferably.

In preferred embodiments histone acetylation and/or the genome methylation is increased or decreased. In particularly preferred embodiments genome methylation is altered by increasing or decreasing histone methylation and/or DNA methylation.

In further embodiments, the PSCs are epigenetically conditioned by decreasing histone deacetylase activity or by increasing histone acetyltransferase activity, and/or by inhibiting methyltransferase activity.

It is particularly preferred that the PSCs are epigenetically conditioned by inhibiting the activity of histone deacetylase class I, class IIA, class IIB, class III and/or class IV, preferably of class III, by inhibiting the activity of lysine- and/or arginine-specific histone methyltransferase (HMT) activity and/or by inhibiting the activity of DNA methyltransferase (Dnmt).

Preferably, the PSCs are epigenetically conditioned by inhibiting Sirtuin, preferably Sirtuin 1, deacetylase and/or inhibiting DNA methyltransferase (Dnmt) 1 and/or 3.

In preferred embodiments, Dnmts are inhibited by siRNAs, shRNAs, peptides, genetic manipulation and/or a pharmacologic compound, preferably selected from the group consisting of decitabine (2'-deoxy-5-azacytidine), 5-azadeoxycytidine, zebularine, (-)-epigallocatechin-3-gallate (EGCG), and RG108 (2-(1,3-dioxo-1,3-dihydro-2H-isoindol-2-yl)-3-(1H-indol-3-yl)propanoic acid). Preferably, all Dnmts are inhibited by RG108.

In preferred embodiments, Sirtuin is inhibited by siRNAs, shRNAs, peptides, genetic manipulation and/or a pharmacologic compound, preferably selected from the group consisting of Ex527 (6-chloro-2,3,4,9-tetrahydro-1H-carbazole-1-carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6. Preferably, Sirtuin 1 is inhibited by Ex527.

In embodiments of the first aspect the PSCs are further conditioned post-translationally. In preferred embodiments, the PSCs are conditioned post-translationally, by altering protein acetylation, phosphorylation, methylation, ribosylation, ubiquitination, SUMOylation, and/or glycosylation. Preferably, protein acetylation, phosphorylation, methylation, ribosylation, ubiquitination, SUMOylation, and/or glycosylation is increased or decreased.

In preferred embodiments, the PSCs are conditioned post-translationally by decreasing the activity of deacetylases targeting proteins involved in the cellular metabolism or the epigenetic status and/or by increasing the activity of acetyltransferases targeting proteins involved in the cellular metabolism or the epigenetic status.

It is preferred that the PSCs are post-translationally conditioned by increasing the acetylation of acetyltransferase target proteins selected from the group consisting of Nrf2; p53, p65, NFκB, E2F1, Foxo, PPARγ, PPARα, PGC-1α, Ku70, LXR, c-myc, HIF-2α, Hsf1, H3K9, H3K56, Rad51, Nbs1, Per2, Torc2, eNOS, Hes1, Hey2, and ß-Catenin.

Preferably, the PSCs are post-translationally conditioned by inhibiting deacetylase class I, class IIA, class IIB, class III and/or class IV, preferably of class III.

In particularly preferred embodiments, the PSCs are post-translationally conditioned by inhibiting Sirtuin, preferably Sirtuin 1, deacetylase. Preferably, Sirtuin is inhibited by siRNAs, shRNAs, peptides, genetic manipulation and/or pharmacologic compound, preferably selected from the group consisting of Ex527 (6-chloro-2,3,4,9-tetrahydro-1H-carbazole-1-carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6, most preferably by Ex527.

In further embodiments the PSCs are metabolically reprogrammed in step (b) of the method of the first aspect of the present invention by altering the metabolismn of the cells, preferably by altering the reactive oxygen species (ROS) signalling, cellular glycolysis, oxidative phosphorylation, pentose phosphate pathway, glycogen metabolism and/or the antioxidant systems.

In preferred embodiments, the PSCs are metabolically reprogrammed by increasing the ROS signalling, by inhibiting glycolysis, by increasing oxidative phosphorylation, by increasing the pentose phosphate pathway, by increasing glycogen synthase activity and/or by inducing antioxidant enzymes including but not limited to NADPH dehydrogenase quinone1 (Nqo1), Sulfiredoxin 1 (Srxn1), Glutamate-cysteine ligase catalytic subunit (Gclc), Glutamate-cysteine ligase modifier Subunit (Gclm), Glutathione S-transferase Mu 1 (Gstm1).

Preferably, the PSCs are metabolically reprogrammed by activation of regulators of antioxidant responses including but not limited to Nrf2, Hsp70 and prohibitin (PHB).

In particularly preferred embodiments, Nrf2 is activated by siRNAs, shRNAs, peptides, genetic manipulation and/or a pharmacologic compound, preferably selected from the group consisting of tert-butylhydroquinone (tBHQ), sulforaphane, protandim, hydroquinone, quercetin, iodoacetamide, cinnamaldehyde, 3,4-dichloroisocoumarin, spiperone, parthenolide, tosyl-L-phenyl-alanine chloromethyl ketone, Ethyl-4-chloro-1-methyl-2-oxo-1,2-dihydroquinoline-3-carboxylate, Bay 11-7085, SKF 83959 hydrobromide, (Z)-gugglesterone, 4-phenyl-3-furoxan-carbonitrile, 2-chloro-5-nitro-N-phenyl-benzamide (GW9662) and arecaidine propargyl ester hydrobromide. Preferably, Nrf2 is activated by tBHQ.

In further embodiments, the method of the first aspect of the present invention further comprises step
(c) sorting the PSCs, preferably via a cell surface marker selected from the group consisting of Thy1 (CD90), GFRa1, Csf1, Cxcr4, CD9, and CD24.

In preferred embodiments only reprogrammed pPSCs are selected by the sorting step (c).

In all embodiments of the first aspects of the present invention, the pluripotent cells are selected from the group consisting of embryonic stem cells (ESCs), induced pluripotent stem cells (iPSCs), primordial germ cells (PGCs), embryonic germ cells (EGCs), epiblast stem cells (EpiSCs), epiblast-like cells (EpiLC), spermatogonial stem cells (SSCs very small embryonic-like cells (VSELs), haploid embryonic stem cells (hPSCs), and cells that express Nanog and Oct4.

In all embodiments of the first aspects of the present invention, the pluripotent cells are derived from mammals, birds, reptiles, amphibians or fish. In preferred embodiments, the PSCs are derived from laboratory animals (e.g. mouse, rat, hamster or frog), domestic animals (including e.g. guinea pig, rabbit, horse, donkey, cow, sheep, goat, pig, chicken, camel, cat, dog, turtle, tortoise, snake, or lizard), or primates selected from the group consisting of chimpanzees, bonobos, gorillas and human beings. Thus, preferably the PSCs are cells selected from the group consisting of laboratory animal cells (e.g. mouse cells, rat cells, hamster cells or frog cells), domestic animal cells (including e.g. guinea pig cells, rabbit cells, horse cells, donkey cells, cow cells, sheep cells, goat cells, pig cells, chicken cells, duck cells, camel cells, cat cells, dog cells, turtle cells, tortoise cells, snake cells, or lizard cells), or primate cells including chimpanzee cells, bonobo cells, gorilla cells and human cells.

pPSCs reprogrammed by the method of the first aspect allow greatly improved control of their differentiation pathways as compared to PSCs. Accordingly, the method of the first aspect of the present invention may further comprise step
(d) differentiating the PSCs.

In preferred embodiments, the PSCs are differentiated into a cell derived from any of the three germ layers, i.e. into a cell that normally derives from the ectoderm, endoderm or mesoderm. Preferably, the PSCs are differentiated into a cell selected from the group consisting of exocrine secretory epithelial cells, hormone secreting cells, cells of the integumentary system, cells of the nervous system, metabolism and storage cells, barrier function cells, extracellular matrix cells, contractile cells, blood and immune system cells, germ cells, nurse cells, and interstitial cells.

In particularly preferred embodiments, the PSCs are differentiated into a cell selected from the group consisting of cardiomyocytes, cardiac pacemaker cells, skeletal muscle cells, smooth muscle cells, vascular smooth muscle cells, endothelial cells, kidney glomerulus parietal cells, kidney glomerulus podocytes, kidney proximal tubule brush border cells, loop of Henle thin segment cells, thick ascending limb cells, kidney distal tubule cells, kidney collecting duct cells, interstitial kidney cells, hepatocytes, sinusoidal hepatic endothelial cells, hepatic stellate cells, kupffer cells, neurons, pyramidal cells, basket cells, betz cells, medium spiny neurons, purkinje cells, renshaw cells, lugaro cells, unipolar brush cells, granule cells, anterior hom cells, spindle cells, schwann cells, satellite cells, olfactory ensheathing cells, retinal ganglion cells, retinal cone cells, retinal rod cells, langerhans cells, melanocytes, epithelial cells, fibroblasts, keratinocytes, merkel cells, hair follicle cells, osteoblasts, osteoclasts, macrophages, neutrophils, dendritic cells, eosinophils, mast cells, basophils, natural killer cells, lymphocytes, T cells, B cells, hematopoietic stem cells, mesenchymal stem cells, pancreatic α cells, pancreatic β cells, pancreatic gamma cells and pancreatic delta cells.

In preferred embodiments of the first aspect of the present invention, the PSCs are differentiated into cardimyocytes by Icariin, triiodothyronine or dorsomorphin homologue 1 (DMH1), into neurons by TCS2210, neurodazine, TWS119, retinoic acid or NGF (nerve growth factor), into hepatocytes by SJA710-6, FPH1 (2-(N-(5-chloro-2-methylphenyl)methylsulfonamido)-N-(2,6-difluorophenyl)acetamide) or HGF (hepatocyte growth factor), or into pancreatic beta cells by (-)-Indolactam V, IDE1, IDE2 or Stauprimide.

In a second aspect, the present invention relates to a differentiated cell reprogrammed by the method of the first aspect of the present invention. Thus, in embodiments ofthe second aspect of the present invention, the differentiated cell is reprogrammed by step
(a) conditioning PSCs epigenetically,
(b) metabolically reprogramming PSCs, and
(c) differentiating the PSCs.

Preferably, in step (a) the PSCs are conditioned into cPSCs (conditioned pluripotent stem cells), preferably to prepare the cell for a subsequent treatment, i.e. to make them responsive for the metabolic reprogramming of step (b). Preferably, in step (b) the cPSCs are metabolically reprogrammed into pPSCs (reprogrammed pluripotent stem cells).

In embodiments of the second aspect of the present invention, the tumorigenicity of the PSCs is eliminated.

In embodiments of the second aspect of the present invention, the proliferation of the PSCs is arrested reversibly.

In further embodiments, step (a) further comprises that the PSCs are conditioned post-translationally.

The metabolic reprogramming of step (b) may occur simultaneously or subsequentially to the conditioning of the PSCs in step (a). In preferred embodiments, the pluripotent cells are metabolically reprogrammed subsequent to being conditioned epigenetically and optionally being conditioned post-translationally.

In embodiments of the second aspect of the present invention, the pluripotent cells are conditioned epigenetically over one or more cell passages, preferably over 1 to 50 cell passages (i.e. over 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 cell passages).In preferred embodiments, the pluripotent cells are conditioned epigenetically over 1 to 30 cell passages, more preferably over 5 to 20 cell passages, most preferably over 10 cell passages.

In embodiments of the second aspect of the present invention, the pluripotent cells are conditioned epigenetically over one or more days, preferably over 1 to 50 days (i.e. over 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 days). In preferred embodiments, the pluripotent cells are conditioned epigenetically over 1 to 20 days, more preferably over 5 to 10 days, most preferably over 7 days.

In embodiments of the second aspect of the present invention, the PSCs are epigenetically conditioned by altering histone acetylation, and/or by altering genome methylation, more preferably.

In preferred embodiments histone acetylation and/or the genome methylation is increased or decreased. In particularly preferred embodiments genome methylation is altered by increasing or decreasing histone methylation and/or DNA methylation.

In further embodiments, the PSCs are epigenetically conditioned by decreasing histone deacetylase activity or by increasing histone acetyltransferase activity, and/or by inhibiting methyltransferase activity.

It is particularly preferred that the PSCs are epigenetically conditioned by inhibiting the activity of histone deacetylase class I, class IIA, class IIB, class III and/or class IV, preferably of class III, by inhibiting the activity of lysine- and/or arginine-specific histone methyltransferase (HMT) activity and/or by inhibiting the activity of DNA methyltransferase (Dnmt).

Preferably, the PSCs are epigenetically conditioned by inhibiting Sirtuin, preferably Sirtuin 1, deacetylase and/or inhibiting DNA methyltransferase (Dnmt) 1 and/or 3.

In preferred embodiments, Dnmts are inhibited by siRNAs, shRNAs, peptides, genetic manipulation and/or a pharmacologic compound, preferably selected from the group consisting of decitabine (2'-deoxy-5-azacytidine), 5-azadeoxycytidine, zebularine, (-)-epigallocatechin-3-gallate (EGCG), and RG108 (2-(1,3-dioxo-1,3-dihydro-2H-isoindol-2-yl)-3-(1H-indol-3-yl)propanoic acid). Preferably, all Dnmts are inhibited by RG108.

In preferred embodiments, Sirtuin is inhibited by siRNAs, shRNAs, peptides, genetic manipulation and/or a pharmacologic compound, preferably selected from the group consisting of Ex527 (6-chloro-2,3,4,9-tetrahydro-1H-carbazole-1-carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6. Preferably, Sirtuin 1 is inhibited by Ex527.

In embodiments of the second aspect the PSCs are further conditioned post-translationally. In preferred embodiments, the PSCs are conditioned post-translationally, by altering protein acetylation, phosphorylation, methylation, ribosylation, ubiquitination, SUMOylation, and/or glycosylation. Preferably, protein acetylation, phosphorylation, methylation, ribosylation, ubiquitination, SUMOylation, and/or glycosylation is increased or decreased.

In preferred embodiments, the PSCs are conditioned post-translationally by decreasing the activity of deacetylases targeting proteins involved in the cellular metabolism or the epigenetic status and/or by increasing the activity of acetyltransferases targeting proteins involved in the cellular metabolism or the epigenetic status.

It is preferred that the PSCs are post-translationally conditioned by increasing the acetylation of acetyltransferase target proteins selected from the group consisting of Nrf₂, p53, p65, NFκB, E2F1, Foxo, PPARγ, PPARα, PGC-1α, Ku70, LXR, c-myc, HIF-2α, Hsf1, H3K9, H3K56, Rad51, Nbs1, Per2, Torc2, eNOS, Hes1, Hey2, and ß-Catenin.

Preferably, the PSCs are post-translationally conditioned by inhibiting deacetylase class I, class IIA, class IIB, class III and/or class IV, preferably of class III.

In particularly preferred embodiments, the PSCs are post-translationally conditioned by inhibiting Sirtuin, preferably Sirtuin 1, deacetylase. Preferably, Sirtuin is inhibited by siRNAs, shRNAs, peptides, genetic manipulation and/or pharmacologic compound, preferably selected from the group consisting of Ex527 (6-chloro-2,3,4,9-tetrahydro-1H-carbazole-1-carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6, most preferably by Ex527.

In further embodiments the PSCs are metabolically reprogrammed in step (b) of the method of the first aspect of the present invention by altering the metabolism of the cells, preferably by altering the reactive oxygen species (ROS) signalling, cellular glycolysis, oxidative phosphorylation, pentose phosphate pathway, glycogen metabolism and/or the antioxidant systems.

In preferred embodiments, the PSCs are metabolically reprogrammed by increasing the ROS signalling, by inhibiting glycolysis, by increasing oxidative phosphorylation, by increasing the pentose phosphate pathway, by increasing glycogen synthase activity and/or by inducing antioxidant enzymes including but not limited to NADPH dehydrogenase quinone1 (Nqo1) Sulfiredoxin 1 (Srxn1), Glutamate-cysteine ligase catalytic subunit (Gclc), Glutamate-cysteine ligase modifier Subunit (Gclm), Glutathione S-transferase Mu 1 (Gstm1).

Preferably, the PSCs are metabolically reprogrammed by activation of regulators of antioxidant responses including but not limited to Nrf2, Hsp70 and prohibitin (PHB).

In particularly preferred embodiments, Nrf2 is activated by siRNAs, shRNAs, peptides, genetic manipulation and/or a pharmacologic compound, preferably selected from the group consisting of tert-butylhydroquinone (tBHQ), sulforaphane, protandim, hydroquinone, quercetin, iodoacetamide, cinnamaldehyde, 3,4-dichloroisocoumarin, spiperone, parthenolide, tosyl-L-phenyl-alanine chloromethyl ketone, Ethyl-4-chloro-1-methyl-2-oxo-1,2-dihydroquinoline-3-carboxylate, Bay 11-7085, SKF 83959 hydrobromide, (Z)-gugglesterone, 4-phenyl-3-furoxan-carbonitrile, 2-chloro-5-nitro-N-phenyl-benzamide (GW9662) and arecaidine propargyl ester hydrobromide. Preferably, Nrf2 is activated by tBHQ.

In further embodiments of the second aspect ofthe present invention the PSCs are sorted after the metabolic reprogramming of step (a) and before step (c) of differentiating the PSCs. In preferred embodiments the PSCs are sorted via one or more cell surface marker. Preferably, the cell surface marker is selected from the group consisting of Thyl (CD90), GFRa1, Csf1, Cxcr4, CD9, and CD24. In particularly preferred embodiments only reprogrammed pPSCs are selected by the sorting step.

In all embodiments of the second aspects of the present invention, the pluripotent cells are selected from the list consisting of embryonic stem cells (ESCs), induced pluripotent stem cells (iPSCs), primordial germ cells (PGCs), embryonic germ cells (EGCs), epiblast stem cells (EpiSCs), epiblast-like cells (EpiLC), spermatogonial stem cells (SSCs), very small embryonic-like cells (VSELs), and haploid embryonic stem cells (hPSCs), or cells that express Nanog and Oct4.

In all embodiments of the second aspects of the present invention, the pluripotent cells are derived from mammals, birds, reptiles, amphibians or fish. In preferred embodiments, the PSCs are derived from laboratory animals (e.g. mouse, rat, hamster or frog), domestic animals (including e.g. guinea pig, rabbit, horse, donkey, cow, sheep, goat, pig, chicken, camel, cat, dog, turtle, tortoise, snake, or lizard), or primates selected from the group consisting of chimpanzees, bonobos, gorillas and human beings. Thus, preferably the PSCs are cells selected from the group consisting of laboratory animal cells (e.g. mouse cells, rat cells, hamster cells or frog cells), domestic animal cells (including e.g. guinea pig cells, rabbit cells, horse cells, donkey cells, cow cells, sheep cells, goat cells, pig cells, chicken cells, duck cells, camel cells, cat cells, dog cells, turtle cells, tortoise cells, snake cells, or lizard cells), or primate cells including chimpanzee cells, bonobo cells, gorilla cells and human cells.

In preferred embodiments of the second aspect of the present invention, in step (c) the PSCs are differentiated into a cell derived from any of the three germ layers, i.e. into a cell that normally derives from the ectoderm, endoderm or mesoderm. Preferably, the PSCs are differentiated into a cell selected from the group consisting of exocrine secretory epithelial cells, hormone secreting cells, cells of the integumentary system, cells of the nervous system, metabolism and storage cells, barrier function cells, extracellular matrix cells, contractile cells, blood and immune system cells, germ cells, nurse cells, and interstitial cells.

In preferred embodiments of the second aspect of the present invention, in step (c) PSCs are differentiated into a cell selected from the group consisting of cardiomyocytes, cardiac pacemaker cells, skeletal muscle cells, smooth muscle cells, vascular smooth muscle cells, endothelial cells, kidney glomerulus parietal cells, kidney glomerulus podocytes, kidney proximal tubule brush border cells, loop of Henle thin segment cells, thick ascending limb cells, kidney distal tubule cells, kidney collecting duct cells, interstitial kidney cells, hepatocytes, sinusoidal hepatic endothelial cells, hepatic stellate cells, kupffer cells, neurons, pyramidal cells, basket cells, betz cells, medium spiny neurons, purkinje cells, renshaw cells, lugaro cells, unipolar brush cells, granule cells, anterior horn cells, spindle cells, schwann cells, satellite cells, olfactory ensheathing cells, retinal ganglion cells, retinal cone cells, retinal rod cells, langcrhans cells, melanocytes, epithelial cells, fibroblasts, keratinocytes, merkel cells, hair follicle cells, osteoblasts, osteoclasts, macrophages, neutrophils, dendritic cells, eosinophils, mast cells, basophils, natural killer cells, lymphocytes, T cells, B cells, hematopoietic stem cells, mesenchymal stem cells, pancreatic α cells, pancreatic β cells, pancreatic gamma cells and pancreatic delta cells.

In preferred embodiments of the second aspect of the present invention, the PSCs are differentiated into cardimyocytes by Icariin.

In a third aspect the present invention relates to pluripotent stem cells reprogrammed by the method of the first aspect of the present invention. Thus, in embodiments of the third aspect of the present invention, the pluripotent stem cells cell is reprogrammed by step
(a) conditioning PSCs epigenetically, and
(b) metabolically reprogramming PSCs.

Preferably, in step (a) the PSCs are conditioned into cPSCs (conditioned pluripotent stem cells), preferably to prepare the cell for a subsequent treatment, i.e. to make them responsive for the metabolic reprogramming of step (b). Preferably, in step (b) the cPSCs are metabolically reprogrammed into pPSCs (reprogrammed pluripotent stem cells).

In embodiments of the third aspect of the present invention, the tumorigenicity of the PSCs is eliminated.

In embodiments of the third aspect of the present invention, the proliferation of the PSCs is arrested reversibly.

In further embodiments, step (a) of the method of the third aspect of the present invention, further comprises that the PSCs are conditioned post-translationally.

The metabollic reprogramming of step (b) may occur simultaneously or subsequentially to the conditioning of the PSCs in step (a). In preferred embodiments, the pluripotent cells are metabolically reprogrammed subsequent to being conditioned epigenetically and optionally being conditioned post-translationally.

In embodiments of the third aspect of the present invention, the pluripotent cells are conditioned epigenetically over one or more cell passages, preferably over 1 to 50 cell passages (i.e. over 1, 2, 3, 4, 5, 6, 7, 8, 9, 10, 11, 12, 13, 14, 15, 16, 17, 18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 cell passages).In preferred embodiments, the pluripotent cells are conditioned epigenetically over 1 to 30 cell passages, more preferably over 5 to 20 cell passages, most preferably over 10 cell passages.

In embodiments of the third aspect of the present invention, the pluripotent cells are conditioned epigenetically over one or more days, preferably over 1 to 50 days (i.c. over 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 days). In preferred embodiments, the pluripotent cells are conditioned epigenetically over 1 to 20 days, more preferably over 5 to 10 days, most preferably over 7 days.

In embodiments of the third aspect of the present invention, the PSCs are epigenetically conditioned by altering histone acetylation, and/or by altering genome methylation, more preferably.

In preferred embodiments histone acetylation and/or the genome methylation is increased or decreased. In particularly preferred embodiments genome methylation is altered by increasing or decreasing histone methylation and/or DNA methylation.

In further embodiments, the PSCs are epigenetically conditioned by decreasing histone deacetylase activity or by increasing histone acetyltransferase activity, and/or by inhibiting methyltransferase activity.

It is particularly preferred that the PSCs are epigenetically conditioned by inhibiting the activity ofhistone deacetylase class I, class IIA, class IIB, class III and/or class IV, preferably of class III., by inhibiting the activity of lysine- and/or arginine-specific histone methyltransferase (HMT) activity and/or by inhibiting the activity of DNA methyltransferase (Dnmt).

Preferably, the PSCs are epigenetically conditioned by inhibiting Sirtuin, preferably Sirtuin 1, deacetylase and/or inhibiting DNA methyltransferase (Dnmt) 1 and/or 3.

In preferred embodiments, Dnmts are inhibited by siRNAs, shRNAs, peptides, genetic manipulation and/or a pharmacologic compound, preferably selected from the group consisting of decitabine (2'-deoxy-5-azacytidine), 5-azadeoxycytidine, zebularine, (-)-epigallocatechin-3-gallate (EGCG), and RG108 (2-(1,3-dioxo-1,3-dihydro-2H-isoindol-2-yl)-3-(1H-indol-3-yl)propanoic acid). Preferably, all Dnmts are inhibited by RG108.

In preferred embodiments, Sirtuin is inhibited by siRNAs, shRNAs, peptides, genetic manipulation and/or a pharmacologic compound, preferably selected from the group consisting of ExS27 (6-chloro-2,3,4,9-tetrahydro-1H-carbazole-1-carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6. Preferably, Sirtuin 1 is inhibited by Ex527.

In embodiments of the third aspect the PSCs are further conditioned post-translationally. In preferred embodiments, the PSCs are conditioned post-translationally, by altering protein acetylation, phosphorylation, methylation, ribosylation, ubiquitination, SUMOylation, and/or glycosylation. Preferably, protein acetylation, phosphorylation, methylation, ribosylation, ubiquitination, SUMOylation, and/or glycosylation is increased or decreased.

In preferred embodiments, the PSCs are conditioned post-translationally by decreasing the activity of deacetylases targeting proteins involved in the cellular metabolism or the epigenetic status and/or by increasing the activity of acetyltransferases targeting proteins involved in the cellular metabolism or the epigenetic status.

It is preferred that the PSCs are post-translationally conditioned by increasing the acetylation of acetyltransferase target proteins selected from the group consisting of Nrf2, p53, p65, NFκB, E2F1, Foxo, PPARγ, PPARα, PGC-1α, Ku70, LXR, c-myc, HIF-2α, Hsf1, H3K9, H3K56, Rad51, Nbs1, Per2, Torc2, eNOS, Hes1, Hey2, and ß-Catenin.

Preferably, the PSCs are post-translationally conditioned by inhibiting deacetylase class I, class IIA, class IIB, class III and/or class IV, preferably of class III.

In particularly preferred embodiments, the PSCs are post-translationally conditioned by inhibiting Sirtuin, preferably Sirtuin 1, deacetylase. Preferably, Sirtuin is inhibited by siRNAs, shRNAs, peptides, genetic manipulation and/or pharmacologic compound, preferably selected from the group consisting of Ex527 (6-chloro-2,3,4,9-tetrahydro-1H-carbazole- I -carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6, most preferably by Ex527.

In further embodiments the PSCs are metabolically reprogrammed in step (b) by altering the metabolismn of the cells, preferably by altering the reactive oxygen species (ROS) signalling, cellular glycolysis, oxidative phosphorylation, pentose phosphate pathway, glycogen metabolism and/or the antioxidant systems.

In preferred embodiments, the PSCs are metabolically reprogrammed by increasing the ROS signalling, by inhibiting glycolysis, by increasing oxidative phosphorylation, by increasing the pentose phosphate pathway, by increasing glycogen synthase activity and/or by inducing antioxidant enzymes including but not limited to NADPH dehydrogenase quinone1 (Nqo1), Sulfiredoxin 1 (Srxn1), Glutamate-cysteine ligase catalytic subunit (Gclc), Glutamate-cysteine ligase modifier Subunit (Gclm), Glutathione S-transferase Mu 1 (Gstm1).

Preferably, the PSCs are metabolically reprogrammed by activation of regulators of antioxidant responses including but not limited to Nrf2, Hsp70 and prohibitin (PHB).

In particularly preferred embodiments, Nrf2 is activated by siRNAs, shRNAs, peptides, genetic manipulation and/or a pharmacologic compound, preferably selected from the group consisting of tert-butylhydroquinone (tBHQ), sulforaphane, protandim, hydroquinone, quercetin, iodoacetamide, cinnamaldehyde, 3,4-dichloroisocoumarin, spiperone, parthenolide, tosyl-L-phenyl-alanine chloromethyl ketone, Ethyl-4-chloro-1-methyl-2-oxo-1,2-dihydroquinoline-3-carboxylate, Bay 11-7085, SKF 83959 hydrobromide, (Z)-gugglesterone, 4-phenyl-3-furoxan-carbonitrile, 2-chloro-5-nitro-N-phenyl-benzamide (GW9662) and arecaidine propargyl ester hydrobromide. Preferably, Nrf2 is activated by tBHQ.

In further embodiments, the PSCs are sorted after the metabolic reprogramming of step (b). In preferred embodiments the PSCs are sorted via one or more cell surface marker. Preferably, the cell surface marker is selected from the group consisting of Thy1 (CD90), GFRa1, Csf1, Cxcr4, CD9, and CD24. In particularly preferred embodiments only reprogrammed pPSCs are selected by the sorting step.

In all embodiments of the third aspects of the present invention, the pluripotent cells are selected from the list consisting of embryonic stem cells (ESCs), induced pluripotent stem cells (iPSCs), primordial germ cells (PGCs), embryonic germ cells (EGCs), epiblast stem cells (EpiSCs), epiblast-like cells (EpiLC), spermatogonial stem cells (SSCs), very small embryonic-like cells (VSELs), and haploid embryonic stem cells (hPSCs), or cells that express Nanog and Oct4.

In all embodiments of the third aspects of the present invention, the pluripotent cells are derived from mammals, birds, reptiles, amphibians or fish. In preferred embodiments, the PSCs are derived from laboratory animals (e.g. mouse, rat, hamster or frog), domestic animals (including e.g. guinea pig, rabbit, horse, donkey, cow, sheep, goat, pig, chicken, camel, cat, dog, turtle, tortoise, snake, or lizard), or primates selected from the group consisting of chimpanzees, bonobos, gorillas and human beings. Thus, preferably the PSCs are cells selected from the group consisting of laboratory animal cells (e.g. mouse cells, rat cells, hamster cells or frog cells), domestic animal cells (including e.g. guinea pig cells, rabbit cells, horse cells, donkey cells, cow cells, sheep cells, goat cells, pig cells, chicken cells, duck cells, camel cells, cat cells, dog cells, turtle cells, tortoise cells, snake cells, or lizard cells), or primate cells including chimpanzee cells, bonobo cells, gorilla cells and human cells.

In a fourth aspect, the present invention relates to a reprogrammed pluripotent stem cell (pPSC) characterized in that the expression level of one or more of the the germ cell markers is altered, preferably increased. In preferred embodiments, the germ cell markers are selected from the group consisting of Nanos2, Tdrd1, Ddx4, Zbtb16, Plk1s1, Cxcr4, Dazl, Stra8, and Piwil2. Preferably, the expression level of Nanos2 is increased. Preferably, the germ cell markers are increased in comparison to the expression level in embryonic stem cells.

In embodiments of the fourth aspect of the present invention, the pluripotent cells are characterized in that the expression levels of one or more of the tumour suppressors is altered, preferably increased. The alteration of the tumour suppressor markers may occur in conjunction with or independent of the alteration of the germ cell markers. Preferably, the expression level of the CDK inhibitors is increased. In preferred embodiments, the CDK inhibitors are selected from the group consisting ofpl5, p16, p21, p27 and p57. Preferably, the expression level of the tumour suppressors is increased in comparison to the expression level in embryonic stem cells.

In embodiments of the fourth aspect of the present invention, the pluripotent cells are characterized in that the expression levels of one or more of the cell metabolism markers are altered, preferably increased or decreased. Alteration of the cell metabolism markers may occur in conjunction with or independent of each other, and in conjunction with or independent of the alteration ofthe germ cell markers or the tumour inhibitor markers.

In embodiments of the fourth aspect of the present invention, the pluripotent cells are characterized in that the expression levels of one or more of the oxidative phosphorylation markers are increased. In preferred embodiment, the oxidative phosphorylation markers are selected from the group consisting of Cox7a1, Cox6b2, Cox8c and Sco2. Preferably, the expression level of the oxidative phosphorylation markers is increased in comparison to the expression level in embryonic stem cells.

In embodiments of the fourth aspect of the present invention, the pluripotent cells are characterized in that the expression levels of one or more of the pentose phosphate pathway markers are altered. In preferred embodiments, the pentose phosphate pathway markers are selected from the group consisting of G6pd, Pgd, Taldo1 and Tkt. In preferred embodiments, the expression level of the pentose phosphate pathway markers G6pd, Pgd, and/or Taldo 1 is increased, preferably increased in comparison to the expression level in embryonic stem cells. In further preferred embodiments, the expression level of the pentose phosphate pathway marker Tkt is decreased, preferably decreased in comparison to the expression in embryonic stem cells.

In embodiments of the fourth aspect of the present invention, the pluripotent cells are characterized in that the expression levels of one or more of the NADPH generating enzyme markers is increased. In preferred embodiments, the NADPH generating enzyme markers arc selected from the group consisting of Mel and Idhl. Preferably, the expression level of NADPH generating enzyme markers is increased in comparison to the expression level in embryonic stem cells.

In embodiments of the fourth aspect of the present invention, the pluripotent cells are characterized in that the expression levels of one or more of the glycolysis markers are decreased. In preferred embodiments the glycolysis markers are selected from the list consisting of Ldha and Pdk1. Preferably, the expression level of glycolysis markers is decreased in comparison to the expression level in embryonic stem cells.

In embodiments of the fourth aspect of the present invention, the pluripotent cells are characterized in that the expression levels of one or more of the glycogen metabolism markers is increased. In preferred embodiments, the glycogen metabolism markers are selected from the group consisting of Pygl and Gys1. Preferably, the expression level of the glycogen metabolism markers is increased in comparison to the expression level in embryonic stem cells.

In embodiments of the fourth aspect of the present invention, the pluripotent cells are characterized by the expression levels of one or more of the pluripotency markers. Alteration of the expression level of the pluripotency markers may occur in conjunction with or independent of each other, and in conjunction with or independent of the alteration of the germ cell markers, the tumour inhibitor markers and/or the cell metabolism markers.

In embodiments of the fourth aspect of the present invention, the pluripotent cells are characterized in that the expression levels of one or more of the core pluripotency markers remains unaltered. Preferably, the preferably core pluripotency markers are selected from the group consisting of Nanog, Oct4, Sox2, Esrrb, Nr5a2, Klf4 and Tbx3. In preferred embodiments, the core pluripotency markers remain unaltered in comparison to the expression in embryonic stem cells.

In embodiments of the fourth aspect of the present invention, the pluripotent cells are characterized in that the expression levels of one or more of the primed pluripotency markers are increased. In preferred embodiment, the primed pluripotency markers are selected from the group consisting of Nodal, Eomes, Gata6, Foxa2, Cer1, Sox17, T and Fgf5. In preferred embodiments, the primed pluripotency markers are increased in comparison to the expression in embryonic stem cells.

In embodiments of the fourth aspect of the present invention, the pluripotent cells are characterized in that the expression levels of one or more of the cell surface markers is increased. In preferred embodiment, the cell surface markers are selected from the group consisting of Thy1 (CD90), GFRa1, Csf1, Cxcr4, CD9, and CD24. In preferred embodiments, the cell surface markers are increased in comparison to the expression in embryonic stem cells.

In all embodiments of the fourth aspects of the present invention, the pluripotent cells are selected from the list consisting of embryonic stem cells (ESCs), induced pluripotent stem cells (iPSCs), primordial germ cells (PGCs), embryonic germ cells (EGCs), epiblast stem cells (EpiSCs), epiblast-like cells (EpiLC), spermatogonial stem cells (SSCs), very small embryonic-like cells (VSELs), and haploid embryonic stem cells (hPSCs), or cells that express Nanog and Oct4.

In all embodiments of the fourth aspects of the present invention, the pluripotent cells are derived from mammals, birds, reptiles, amphibians or fish. In preferred embodiments, the PSCs are derived from laboratory animals (e.g. mouse, rat, hamster or frog), domestic animals (including e.g. guinea pig, rabbit, horse, donkey, cow, sheep, goat, pig, chicken, camel, cat, dog, turtle, tortoise, snake, or lizard), or primates selected from the group consisting of chimpanzees, bonobos, gorillas and human beings. Thus, preferably the PSCs are cells selected from the group consisting of laboratory animal cells (e.g. mouse cells, rat cells, hamster cells or frog cells), domestic animal cells (including e.g. guinea pig cells, rabbit cells, horse cells, donkey cells, cow cells, sheep cells, goat cells, pig cells, chicken cells, duck cells, camel cells, cat cells, dog cells, turtle cells, tortoise cells, snake cells, or lizard cells), or primate cells including chimpanzee cells, bonobo cells, gorilla cells and human cells.

In a fifth aspect the present invention relates to a reprogrammed pluripotent stem cell (pPSC) according to the second, third or fourth aspect of the present invention for use in treating cancer.

pPSCs may be used to restore fertility of cancer patients after cancer therapy, in particular of male cancer patients where the spermatogonial stem cell population has been damaged. pPSCs may also be used to test the toxicity of cancer drugs on stem cells, in particular on germ cells. pPSCs may also be used to generate other types of stem cells which may be depleted by cancer therapy, such as mesenchymal stem cells to be transplanted into the body of cancer patients after cancer therapy.

The reprogrammed pPSCs of the second, third or fourth aspect of the present invention represent non-tumorigenic pluripotent cells most similar to spermatogonial stem cells in the body and may thus be used in treating infertility in male.

Accordingly, in a sixth aspect the present invention relates to a reprogrammed pluripotent stem cell (pPSC) according to the third or fourth aspect of the present invention for use in treating infertility in male. In preferred embodiments, the infertility is caused by anti-cancer therapy or by other therapy which damages the SSC population within the testis, environmental factors, heat, cold, food, radiation, chemical toxicity, infection, inflammation, autoimmune disease, physical injury or genetics.

The cell proliferation of the pPSCs can be arrested for up to 7 days or even longer while retaining pluripotency. This proliferation arrest is reversible and pPSCs can be induced to differentiate into defined lineages. This uncouples cell proliferation from pluripotency and therefore represents a very valuable tool for tissue engineering and stem cell science.

Thus, in a seventh aspect the present invention relates to a reprogrammed pluripotent stem cell (pPSC) according to the second, third or fourth aspect of the present invention for use in cell or tissue therapy for tissue and organ regeneration of the heart, cardiovascular system, brain, neurological system, eye, ear, liver, kidney, pancreas, endocrine glands, lung, intestines, muscle, skin, hair, joints, bones, and/or teeth.

Since the reprogrammed pPSCs of the second, third or fourth aspect of the present invention represent non-tumorigenic and pluripotent cells most similar to spermatogonial stem cells in the body, these cells can serve as a tool to test whether cancer drug have potentially detrimental effects on stem cells of the body.

Accordingly, in an eighth aspect the present invention relates to a method of testing the toxicity of a pharmaceutical, preferably a pharmaceutical directed against cancer or contraception drugs, comprising the use of a differentiated cell according to the second aspect of the present invention and/or a reprogrammed pluripotent stem cell (pPSC) according to the third or fourth aspect of the present invention.

Moreover, since pPSCs of the second, third or fourth aspect of the present invention can be generated patient-specific, they have the same genetic background as the patient from which they were produced and could be used to screen cancer drugs for individualized cancer therapy.

Thus, in a ninth aspect the present invention relates to a method of screening for a pharmaceutical, preferably a pharmaceutical directed against cancer or contraception drugs, comprising the use of a differentiated cell according to the second aspect of the present invention and/or a reprogrammed pluripotent stem cell (pPSC) according to the third or fourth aspect of the present invention.

In a tenth aspect the present invention relates to a method of testing the toxicity of an environmental substance, preferably a molecule or particle, comprising the use of a differentiated cell according to the second aspect of the present invention and/or a reprogrammed pluripotent stem cell (pPSC) according to the third or fourth aspect of the present invention. In preferred embodiments the substance may cause cancer.

In a eleventh aspect the present invention relates to a pharmaceutical comprising a differentiated cell according to the second aspect of the present invention and/or a reprogrammed pluripotent stem cell (pPSC) according to the third or fourth aspect of the present invention.

In preferred embodiments, the pharmaceutical further comprises a pharmaceutically acceptable carrier and/or excipient and optionally one or more additional active substances.

The transformation of PSCs into non-tumorigenic pPSCs according to the first aspect of the present invention also offers a new approach to discover genes which are critically involved in controlling cancer. Since the re-programmed pPSCs of the third or fourth aspect of the present invention are pluripotent and non-tumorigenic they are an ideal tool to identify new genes involved in cancer, preferably tumour suppressing genes. Furthermore, the reprogrammed pPSC of the second, third or fourth aspect of the present invention provide an excellent model system to identify genes involved in the development of other diseases.

Thus, in an twelfth aspect the present invention relates to a method of identifying genes involved in disease comprising the use of a differentiated cell according to the second aspect of the present invention and/or a reprogrammed pluripotent stem cell (pPSC) according to the third or fourth aspect of the present invention.

In preferred embodiments of the eleventh aspect of the present invention, the disease is selected from the group consisting of cancer, infertility, oligospermia, aspermia, hypospermia, azoospermia, teratospermia, asthenozoospermia, cardiovascular disease, atherosclerosis, hepatitis, fatty liver disease, cirrhosis, primary sclerosing cholangitis, hemochromatosis, chronic kidney disease, glomerulonephritis, polycystic kidney disease, alzheimer's disease, parkinson's disease, multiple sclerosis, amyotrophic lateral sclerosis, motor neuron diseases, lewy body disease, huntington's disease, spinocerebellar ataxia, friedreich's ataxia, spinal muscular atrophy, retinopathy, macular degeneration and diabetes.

The reprogrammed pPSCs of the third or fourth aspect ofthe present invention represent non-tumorigenic SSCs which are positive for all SSC markers tested including Nanos2 and Zbtb16 as well as Nanog. Of special interest is that the expression of Nanos2 could be achieved without the use of GDNF which is a potentially tumorigenic growth factor. These SSCs can directly implanted into the testis to restore fertility. The here created SSCs can also be used to create other germ cell lineages such as in vitro sperm for fertility restoration or fertility research. This solution is of particular interest to cancer survivors who do not have their SSCs frozen. Autologous SSCs can be created for these patients using the method of the first aspect of the present invention. The reprogrammed pPSCs represent non-tumorigenic spermatogonial stem cells (SSCs) which can be used for fertility restoration, *in vitro* germ cell differentiation, and (fertility) research.

Accordingly, in a twelfth aspect the present invention relates to a method of producing germ cells comprising the use of a reprogrammed pluripotent stem cell (pPSC) according to the third or fourth aspect of the present invention.

In a fourteenth aspect the present invention relates to a method of producing sperm cells or oocytes *in vitro* comprising the use of a reprogrammed pluripotent stem cell (pPSC) according to the third or fourth aspect of the present invention.

In a fifteenth aspect the present invention relates to a method of treating a patient, preferably a patient suffering from cancer, comprising administering to a subject a differentiated cell according to the second aspect of the present invention or a reprogrammed pluripotent stem cell (pPSC) according to the third or fourth aspect of the present invention.

In particular the present invention relates to the following aspects:
1. A method of reprogramming pluripotent stem cells (PSC) comprising the steps
   (a) conditioning PSCs epigenetically, and
   (b) metabolically reprogramming PSCs.
2. The method of aspect 1, wherein tumorigenicity of the PSCs is eliminated.
3. The method of aspect 1 or 2, wherein the proliferation of the PSCs is arrested reversibly.
4. The method of any of aspects 1 to 3, wherein in step (a) the PSCs are further conditioned post-translationally.
5. The method of any of aspects 1 to 4, wherein the pluripotent cells are metabolically reprogrammed subsequent to being conditioned epigenetically and optionally conditioned post-translationally.
6. The method of any of aspects 1 to 5, wherein the pluripotent cells are conditioned epigenetically over one or more cell passages, preferably over 1 to 50 cell passages, more preferably over 5 to 25 cell passages, most preferably over 10 cell passages.
7. The method of any of aspects 1 to 6, wherein the pluripotent cells are metabolically reprogrammed over one or more days, preferably over 1 to 20 days, more preferably over 5 to 10 days, most preferably over 7 days.
8. The method of any of aspects 1 to 7, wherein the PSCs are epigenetically conditioned by altering, preferably increasing or decreasing, histone acetylation, and/or by altering, preferably increasing or decreasing, genome methylation, more preferably by increasing or decreasing histone methylation and/or DNA methylation.
9. The method of any of aspects 1 to 8, wherein the PSCs are epigenetically conditioned by decreasing histone deacetylase activity or increasing histone acetyltransferase activity, and/or by inhibiting methyltransferase activity.
10. The method of any of aspects 1 to 9, wherein the PSCs are epigenetically conditioned by inhibiting the activity of histone deacetylase class I, class IIA, class IIB, class III. and/or class IV, by inhibiting the activity of lysine- and/or arginine-specific histone methyltransferase (HMT) activity and/or by inhibiting the activity of DNA methyltransferase (Dnmt).
11. The method of any of aspects 1 to 10, wherein the PSCs are epigenetically conditioned by inhibiting Sirtuin, preferably Sirtuin 1, deacetylase and/or inhibiting DNA methyltransferase (Dnmt) 1 and/or 3.
12. The method of aspect 11, wherein the Dnmt is inhibited by siRNAs, shRNAs, peptides, genetic manipulation and/or a pharmacologic compound, preferably selected from the group consisting of decitabine (2'-deoxy-5-azacytidine), 5-azadeoxycytidine, zebularine, (-)-epigallocatechin-3-gallate (EGCG), and RG108 (2-(1,3-dioxo-1,3-dihydro-2H-isoindol-2-yl)-3-(1H-indol-3-yl)propanoic acid).
13. The method of aspect 11, wherein Sirtuin is inhibited by siRNAs, shRNAs, peptides, genetic manipulation and/or a pharmacologic compound, preferably selected from the group consisting of Ex527 (6-chloro-2,3,4,9-tetrahydro-1H-carbazole-1-carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6.
14. The method of any of aspects 1 to 13, wherein the PSCs are conditioned post-translationally, preferably by altering, preferably increasing or decreasing, protein acetylation, phosphorylation, methylation, ribosylation, ubiquitination, SUMOylation, and/or glycosylation.
15. The method of any of aspects 1 to 14, wherein the PSCs are conditioned post-translationally by decreasing the activity of deacetylases targeting proteins involved in cellular metabolism, antioxidant, redox or epigenetic status and/or by increasing the activity of acetyltransferases targeting proteins involved in cellular metabolism, antioxidant, redox or epigenetic status.
16. The method of any of aspects 1 to 15, wherein the PSCs are post-translationally conditioned by increasing the acetylation of acetyltransferase target proteins selected from the group consisting of Nrf2, p53, p65, NFκB, E2F1, Foxo, PPARγ, PPARα, PGC-1α, Ku70, LXR, c-myc, HIF-2α, Hsf1, H3K9, H3K56, Rad51 Nbs1, Per2, Torc2, eNOS, Hes1, Hey2, and ß-Catenin.
17. The method of any of aspects 1 to 16, wherein the PSCs are post-translationally conditioned by inhibiting deacetylase class I, class IIA, class IIB, class III and/or class IV
18. The method of any of aspects 1 to 17, wherein the PSCs are post-translationally conditioned by inhibiting Sirtuin, preferably Sirtuin 1, deacetylase.
19. The method of aspect 18, wherein Sirtuin is inhibited by siRNAs, shRNAs, peptides, genetic manipulation and/or pharmacologic compound, preferably selected from the group consisting of Ex527 (6-chloro-2,3,4,9-tetrahydro-1H-carbazole-1-carboxamide), Cambinol (5-(2-Hydroxynaphthalen-1-ylmethyl)-6-phenyl-2-thioxo-2,3-dihydro-1H-pyrimidin-4-one), Salermide, Tenovin-1 and Tenovin-6.
20. The method of any of aspects 1 to 19, wherein the PSCs are metabolically reprogrammed by altering the reactive oxygen species (ROS) signalling, cellular glycolysis, oxidative phosphorylation, pentose phosphate pathway, glycogen metabolism and/or the antioxidant systems.
21. The method of aspect 20, wherein the PSCs are metabolically reprogrammed by increasing the ROS signalling, by inhibiting glycolysis, by increasing oxidative phosphorylation, by increasing the pentose phosphate pathway, by increasing glycogen synthase activity and/or by inducing antioxidant enzymes including but not limited to NADPH dehydrogenase quinone1 (Nqo1), Sulfiredoxin 1 (Srxn1), Glutamate-cysteine ligase catalytic subunit (Gclc), Glutamate-cysteine ligase modifier Subunit (Gclm), Glutathione S-transferase Mu 1 (Gstm1).
22. The method of aspect 21, wherein the PSCs are metabolically reprogrammed by activation of regulators of antioxidant responses including but not limited to Nrf2, Hsp70 and prohibitin (PHB).
23. The method of aspect 22, wherein Nrf2 is activated by siRNAs, shRNAs, peptides, genetic manipulation and/or a pharmacologic compound, preferably selected from the group consisting of tert-butylhydroquinone (tBHQ), sulforaphane, protandim, hydroquinone, quercetin, iodoacetamide, cinnamaldehyde, 3,4-dichloroisocoumarin, spiperone, parthenolide, tosyl-L-phenyl-alanine chloromethyl ketone, Ethyl-4-chloro-1-methyl-2-oxo-1,2-dihydroquinoline-3-carboxylate, Bay 11-7085, SKF 83959 hydrobromide, (Z)-gugglesterone, 4-phenyl-3-furoxan-carbonitrile, 2-chloro-5-nitro-N-phenyl-benzamide (GW9662) and arecaidine propargyl ester hydrobromide.
24. The method of any of aspects 1 to 23 further comprising the step (c) sorting the PSCs, preferably via a cell surface marker selected from the group consisting of Thy1 (CD90), GFRa1, Csf1, Cxcr4, CD9, and CD24.
25. The method of any of aspects 1 to 24, wherein the pluripotent cells are selected from the list consisting of embryonic stem cells (ESCs), induced pluripotent stem cells (iPSCs), primordial germ cells (PGCs), embryonic germ cells (EGCs), epiblast stem cells (EpiSCs), epiblast-like cells (EpiLC), spermatogonial stem cells (SSCs), very small embryonic-like cells (VSELs), and haploid embryonic stem cells (hPSCs).
26. The method of any of aspects 1 to 25, wherein the pluripotent cells are derived from laboratory animals (e.g. mouse, rat, hamster or frog), domestic animals (including e.g. guinea pig, rabbit, horse, donkey, cow, sheep, goat, pig, chicken, camel, cat, dog, turtle, tortoise, snake, or lizard), or primates selected from the group consisting of chimpanzees, bonobos, gorillas and human beings.
27. The method of any of aspects 1 to 26 further comprising the step (d) differentiating the PSCs, preferably differentiating the PSCs into cardiomyocytes, cardiac pacemaker cells, skeletal muscle cells, smooth muscle cells, vascular smooth muscle cells, endothelial cells, kidney glomerulus parietal cells, kidney glomerulus podocytes, kidney proximal tubule brush border cells, loop of Henle thin segment cells, thick ascending limb cells, kidney distal tubule cells, kidney collecting duct cells, interstitial kidney cells, hepatocytes, sinusoidal hepatic endothelial cells, hepatic stellate cells, kupffer cells, neurons, pyramidal cells, basket cells, betz cells, medium spiny neurons, purkinje cells, renshaw cells, lugaro cells, unipolar brush cells, granule cells, anterior horn cells, spindle cells, schwann cells, satellite cells, olfactory ensheathing cells, retinal ganglion cells, retinal cone cells, retinal rod cells, langerhans cells, melanocytes, epithelial cells, fibroblasts, keratinocytes, merkel cells, hair follicle cells, osteoblasts, osteoclasts, macrophages, neutrophils, dendritic cells, eosinophils, mast cells, basophils, natural killer cells, lymphocytes, T cells, B cells, hematopoietic stem cells, mesenchymal stem cells, pancreatic α cells, pancreatic cells, pancreatic gamma cells and pancreatic delta cells.
28. The method of aspect 24, wherein the PSCs are differentiated into cardimyocytes by Icariin.
29. A differentiated cell reprogrammed by the method of aspect 27 or 28.
30. A pluripotent stem cell reprogrammed by the method of any of aspects 1 to 26.
31. A reprogrammed pluripotent stem cell (pPSC) characterized in that the expression of the germ cell markers, preferably selected from the group consisting of Nanos2, Tdrd1, Ddx4, Zbtb16, Plk1s1, Cxcr4, Dazl, Stra8, and Piwil2, is increased, preferably increased in comparison to the expression in embryonic stem cells.
32. The pluripotent cell of aspect 31 further characterized in that the expression levels of tumour suppressor, preferably of CDK inhibitors, preferably selected from the group consisting of p15, p16, p21, p27 and p57, is increased, preferably increased in comparison to the expression in embryonic stem cells.
33. The pluripotent cell of aspects 31 or 32 further characterized in that the expression levels of the oxidative phosphorylation marker, preferably selected from the list consisting of Cox7a1, Cox6b2, Cox8c and Sco2 is increased, preferably increased in comparison to the expression in embryonic stem cells.
34. The pluripotent cell of any of aspects 31 to 33 further characterized in that the expression levels of the pentose phosphate pathway marker, preferably selected from the list consisting of G6pd, Pgd and Taldol is increased, preferably increased in comparison to the expression in embryonic stem cells.
35. The pluripotent cell of any of aspects 31 to 34 further characterized in that the expression levels of the pentose phosphate pathway marker, preferably selected from the list consisting of Tkt is decreased, preferably decreased in comparison to the expression in embryonic stem cells.
36. The pluripotent cell of any of aspects 31 to 35 further characterized in that the expression levels of the NADPH generating enzyme marker, preferably selected from the list consisting of Me1 and Idh1 is increased, preferably increased in comparison to the expression in embryonic stem cells.
37. The pluripotent cell of any of aspects 31 to 36, further characterized in that the expression levels of glycolysis marker, preferably selected from the list consisting of Ldha and Pdk1, is decreased, preferably decreased in comparison to the expression in embryonic stem cells.
38. The pluripotent cell of any of aspects 31 to 37, further characterized in that the expression levels of glycogen metabolism marker, preferably selected from the list consisting of Pyg1 and Gys1, is increased, preferably increased in comparison to the expression in embryonic stem cells.
39. The pluripotent cell of any of aspects 31 to 38 further characterized in that the expression of core pluripotency markers, preferably selected from the group consisting of Nanog, Oct4, Sox2, Esrrb, Nr5a2, Klf4 and Tbx3, remain unaltered in comparison to the expression in embryonic stem cells.
40. The pluripotent cell of any of aspects 31 to 39 further characterized in that the expression of the primed pluripotency markers selected from the group consisting of Nodal, Eomes, Gata6, Foxa2, Cer1, Sox17, T and Fgf5, is increased, preferably increased in comparison to the expression in embryonic stem cells.
41. The pluripotent cell of any of aspects 31 to 40 further characterized in that the cells are selected from the list consisting of embryonic stem cells (ESCs), induced pluripotent stem cells (iPSCs), primordial germ cells (PGCs), embryonic germ cells (EGCs), epiblast stem cells (EpiSCs), epiblast-like cells (EpiLC), spermatogonial stem cells (SSCs), very small embryonic-like cells (VSELs), and haploid embryonic stem cells (hPSCs).
42. The pluripotent cell of any of aspects 31 to 41 further characterized in that the cells are derived from laboratory animals (e.g. mouse, rat, hamster or frog), domestic animals (including e.g. guinea pig, rabbit, horse, donkey, cow, sheep, goat, pig, chicken, camel, cat, dog, turtle, tortoise, snake, or lizard), or primates selected from the group consisting of chimpanzees, bonobos, gorillas and human beings.
43. The pluripotent cell of any of aspects 31 to 42 further characterized in that the expression levels of the cell surface marker, preferably selected from the list consisting of Thy1 (CD90), GFRa1, Csf1, Cxcr4, CD9, CD24 is increased, preferably increased in comparison to the expression in embryonic stem cells.
44. A reprogrammed pluripotent stem cell (pPSC) according to any of aspects 30 to 43 for use in treating cancer.
45. A reprogrammed pluripotent stem cell (pPSC) according to any of aspects 30 to 43 for use in treating infertility in male caused by anti-cancer therapy or other therapy which damages the SSC population within the testis, environmental factors, heat, cold, food, radiation, chemical toxicity, infection, inflammation, autoimmune disease, physical injury or genetics.
46. A reprogrammed pluripotent stem cell (pPSC) according to any of aspects 30 to 43 for use in cell or tissue therapy for tissue and organ regeneration of the heart, cardiovascular system, brain, neurological system, eye, ear, liver, kidney, pancreas, endocrine glands, lung, intestines, muscle, skin, hair, joints, bones, and/or teeth.
47. A method of screening for a pharmaceutical, preferably a pharmaceutical directed against cancer or contraception drugs, comprising the use of a differentiated cell according to aspect 29 and/or a reprogrammed pluripotent stem cell (pPSC) according to any of aspects 30 to 43.
48. A method of testing the toxicity of a pharmaceutical, preferably a pharmaceutical directed against cancer or contraception drugs, comprising the use of a differentiated cell according to aspect 29 and/or a reprogrammed pluripotent stem cell (pPSC) according to any of aspects 30 to 43.
49. A method of testing the toxicity of an environmental substance (molecule or particle) preferably a substance that might cause cancer, comprising the use of a differentiated cell according to claim 29 and/or a reprogrammed pluripotent stem cell (pPSC) according to any of claims 30 to 43.
50. A method of identifying genes involved in disease, preferably involved in cancer, infertility, oligospermia, aspermia, hypospermia, azoospermia, teratospermia, asthenozoospermia, cardiovascular disease, atherosclerosis, hepatitis, fatty liver disease, cirrhosis, primary sclerosing cholangitis, hemochromatosis, chronic kidney disease, glomerulonephritis, polycystic kidney disease, alzheimer's disease, parkinson's disease, multiple sclerosis, amyotrophic lateral sclerosis, motor neuron diseases, lewy body disease, huntington's disease, spinocerebellar ataxia, friedreich's ataxia, spinal muscular atrophy, retinopathy, macular degeneration and diabetes comprising the use of a differentiated cell according to aspect 29 and/or a reprogrammed pluripotent stem cell (pPSC) according to any of aspects 30 to 43.
51. A method of producing germ cells comprising the use of a reprogrammed pluripotent stem cell (pPSC) according to any of aspects 30 to 43.
52. A method of producing sperm cells or oocytes *in vitro* comprising the use of a reprogrammed pluripotent stem cell (pPSC) according to any of aspects 30 to 43.
53. A method of treating a patient, preferably a patient suffering from cancer, comprising administering to a subject a differentiated cell according to aspect 29 or a reprogrammed pluripotent stem cell (pPSC) according to any of aspects 30 to 43.

### EXAMPLES

### Example 1

Mouse ESCs (mESCs) were conditioned, transforming them into cPSCs (cESCs) and subsequently reprogrammed into pPSCs for 7 days (d0 to d7) using the methods described in the invention.

### General Cell Culture

Serving as a model for PSCs, mESCs were purchased from Millipore (Millipore, Zug, Switzerland, MilliTrace™ Nanog GFP Reporter cell line, SCR089). These mESCs harboured a puromycin resistance gene and a Nanog GFP reporter (GFP coding sequence fused to the Nanog promoter) which can be used as an internal control for pluripotency. All cells were cultured in a CO₂ incubator at 37°C with a humified atmosphere containing 5% CO₂ in T25, T75 tissue culture flasks (TPP, Trasadingen, Switzerland) or 8 well µ-Slides (ibidi, Munich, Germany, µ-Slides 8 well ibiTreat, tissue culture treated, 80826), which were coated with EmbryoMax Gelatin Solution (0.1 %) (Millipore, ES-006-B) for 15 min at room temperature before cells were seeded. mESCs were passaged by volume 1:20 every 2 days (passaging at around 60%-80% confluency) using SteinPro^{®} Accutase^{®} (Life Technologies, Carlsbad, USA) to dissociate cells.

Basic medium (BM) consisted of Embryomax DMEM (Millipore, SLM-220-B), supplemented with 15% (v/v) Embryomax FBS (Millipore, ES-009-C), 1 mM sodium pyruvate (Millipore, TMS-005-C), 0.1 mM non-essential amino acids (Invitrogen, 11140), and 2 mM Glutamax-I Supplement (Invitrogen, 35050).

mESCs were cultured in basic growth medium (BGM), consisting of BM supplemented with 0.1 mM β-mercaptoethanol (βME) (Invitrogen, 31350-010), 10 ng/ml leukemia inhibitory factor (LIF) (Invitrogen, PMC4054) and 0.5 µg/ml puromycin (Invitrogen, A11138-03).

### Epigenetic Conditioning

mESCs were conditioned to transform them into cPSCs (cESCs) by culturing the mESCs in conditioning medium (CM) consisting of BGM supplemented with 5 µM Ex527 (Cayman chemical, Ann Arbor, MI, USA, 10009798) and 100 µM RG108 (Cayman chemical, 13302) for 10 cell passages, while passaging the cells by volume 1:20 every 2 days (passaging at around 60%-80% confluency). Ex527 and RG108 were used from stock solutions in dimethyl sulfoxide (DMSO). The total DMSO concentration in the culture medium during the conditioning was 0.03% (v/v). mESCs cultured in BGM supplemented with 0.03% (v/v) DMSO and passaged in the same manner in parallel to the cells being conditioned, served as a control (WT ESCs).

### Metabolic Reprogramming

cPSCs (cESCs) were reprogrammed to transform them into pPSCs. For this cESCs were seeded at a cell density of 10'000 cells/cm² and cultured in CM for 2 days. No further passages were performed at this point. The culture medium was then switched to reprogramming medium (RM) consisting of BM supplemented with 5 µM Ex527, 100 µM RG108 and 10 µM tBHQ (Sigma-Aldrich, St. Louis, MO, USA, tert-Butylhydroquinone, 112941) and cultured for a period of up to 7 days with medium replacement every 2 days. The total DMSO concentration in the culture medium during the reprogramming was 0.04% (v/v). βME, LIF and puromycin were not present in RM. As a negative control, PSCs (WT ESCs) which were previously passaged in BGM supplemented with 0.03% (v/v) DMSO, were only subjected to removal of βME, removal of LIF and removal of puromycin (not subjected to metabolic reprogramming), but otherwise treated in the same way as cESCs. The medium of WT ESCs at this point consisted of BM supplemented with 0.04% (v/v) DMSO.

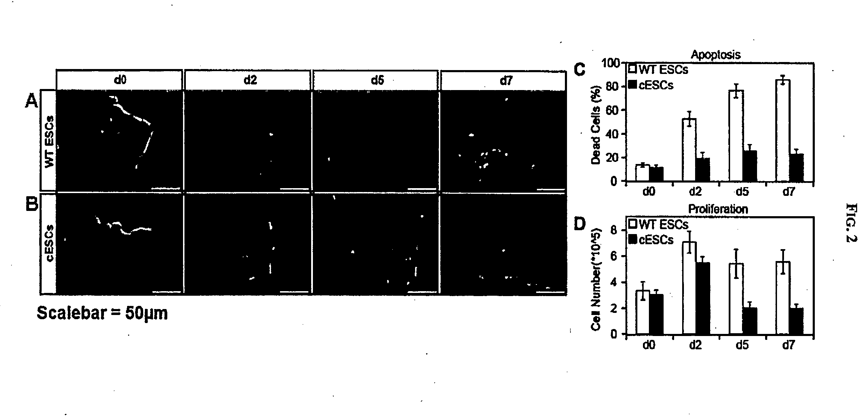

WT ESCs showed much higher rates of apoptosis and proliferation than cESCs during metabolic reprogramming until at d7 WT ESCs were mostly apoptotic and only very flat or elongated cells remained (Fig. 2 A). These cells were probably differentiated. The cESCs undergoing metabolic reprogramming (pPSCs) reduced proliferation and showed low apoptosis and from d2 onward no increase in the size of cell colonies was observed anymore (Fig. 2 B).

### Light Microscopy

Cells were fixed with formaldehyde (2.5% w/v) in phosphate-buffered saline (PBS) for 20 min and photographed using an Axiovert 200M inverted microscope (Carl Zeiss, Jena, Germany).

### Apoptosis and Proliferation Analysis

Cell apoptosis and proliferation were measured by trypan-blue staining (Sigma-Aldrich) and cell counting respectively. Briefly, 0.4% trypan blue solution (Sigma-Aldrich) was added to the culture medium of cells at a dilution of 1:25 and incubated at room temperature for 5 min. The supernatant ofthe cell cultures was collected and the remaining adherent cells dissociated using StemPro^{®} Accutase^{®} (Life Technologies). The dissociated cells were combined with the previously collected supernatant. Cells were then centrifuged and resuspended in phosphate-buffered saline (PBS), followed by counting of live and dead cells in triplicates using a cell counting chamber (Neubauer improved, 0.100mm depth, 1µl in mm², 0.0025 mm² area of smallest square). Total cell numbers were evaluated by adding the numbers of live and dead cells.

The results shown in Fig. 2 C and D indicate that the pPSCs (metabolically reprogrammed cESCs) were arrested in proliferation. To confirm these results, Western Blot analysis of the protein expression levels of the proliferation markers (Cyclin D1 and Cyclin B1) were performed.

### Western Blot Analysis

The medium of the cells was removed and the cells were washed 3x with PBS. The whole cell lysate extracts were collected by scraping cells on ice using 250 ul of RIPA buffer (Sigma-Aldrich) combined with both protease and phosphatsase inhibitors (Sigma-Aldrich). After lysate collection, the whole cell lysate samples were incubated on an end-over-end shaker for 10 min at 4°C. Aliquots (20 µl) were collected for protein quantification using Pierce BCA protein assay (Thermo Scientific, MA, USA). The remaining sample volume was further incubated with 5x Laemmli Sample Buffer (5% β-mercaptoethanol, reducing agent) for 5 min at 95°C on a bench Thermomixer (Eppendorf, Vaudaux, Switzerland). After the whole cell lysate preparation, samples were stored at -20°C. The western blot samples were loaded (10 mg/well), fractionated by SDS-PAGE (10% or 7% polyacrylamide) and transferred to a polyvinylidene difluoride (PDVF) membrane, according to the manufacturer's protocols (Bio-Rad, Hercules, CA, USA). The membrane incubations were performed on 10x Roti-Block (Carl Roth, Karlsruhe, Germany) TBS-T buffer, overnight at 4°C for primary antibodies and 1 h at room temperature for secondary antibodies. Primary antibodies and their dilutions were 1:1000 for Cyclin B1 (rabbit, Cell Signaling Technology, Danvers, MA, USA, 4138), Cyclin D1 (mouse, Cell Signaling Technology, 2926) and alpha-tubulin (mouse, Abcam, Cambridge, Massachusetts, USA, ab7750) antibodies. The used secondary antibodies were horseradish peroxidase (HRP, Jackson ImmunoResearch Laboratories, West Grove, PA, USA) alternating with alkaline phosphatase (AP, Promega, Madison, WI, USA) both at 1:3000 dilution, blots were further developed following manufactor's protocol using ECL plus substrate (GE Healthcare, Piscataway, NJ, USA) and CDPstar substrate (Sigma-Aldrich), respectively.

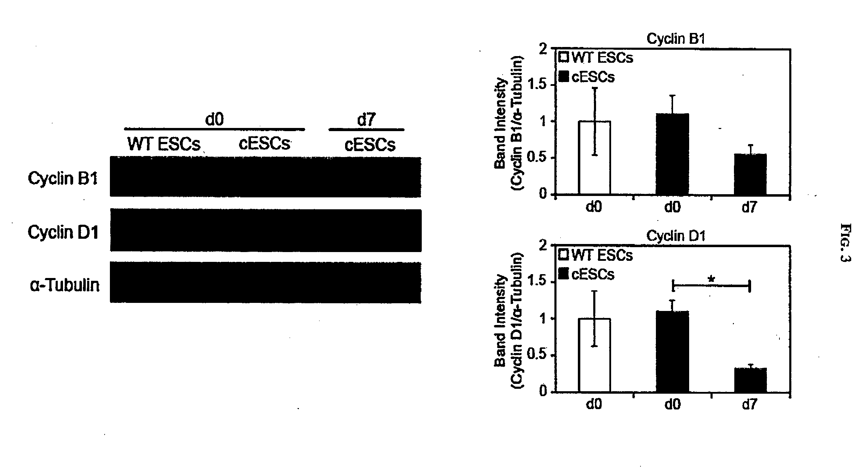

WT ESCs served as negative control for the starting point. Cyclin D1 was strongly downregulated while Cyclin B1 was slightly downregulated which confirms a reduced cell proliferation (see Fig. 3).

### Example 2 - Protein level of Pluripotency Markers during and after Metabolic Reprogramming

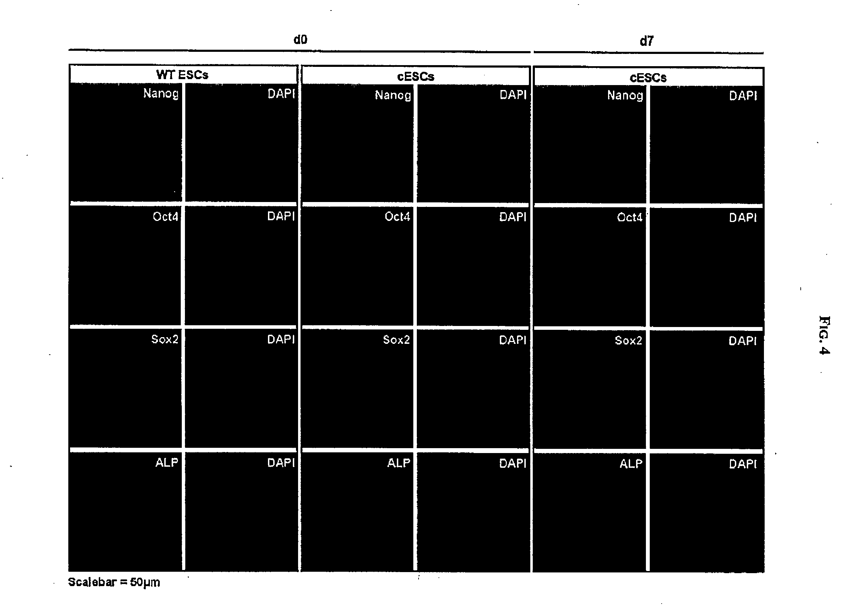

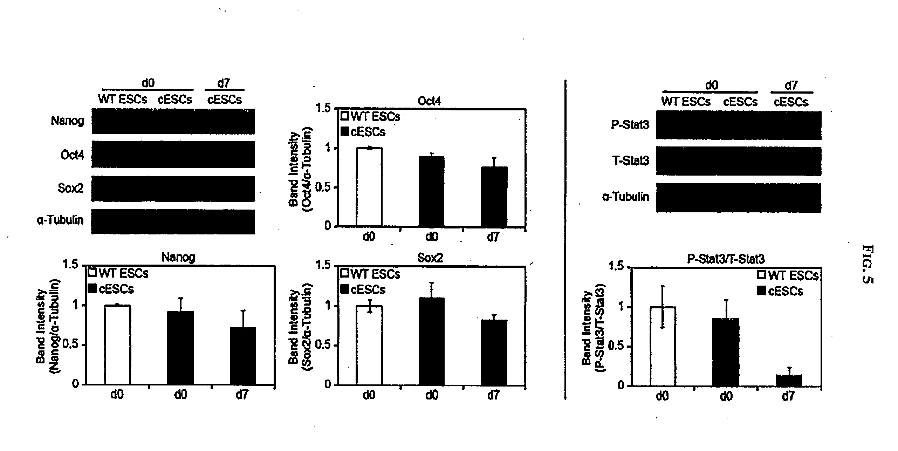

To analyse the pluripotency ofthe cESCs during the metabolic reprogramming, protein levels of pluripotency markers were analysed by immunofluorescence (Fig. 4) and Western Blot analysis (Fig. 5).

Western Blot Analysis was performed as described in Example 1. Primary antibodies and their dilutions were 1:1000 for Nanog (rabbit, Millipore, ab9220), Sox2 (mouse, Millipore, mab4343), Phospho Stat3 (rabbit, Cell Signaling Technology, 9131), total Stat3 (mouse, Cell Signaling Technology, 9139) and 1:500 for Oct4 (rabbit, Millipore, ab3209).

### Immunofluorescence

Cells were fixed with formaldehyde (2.5% w/v) in PBS for 20 min, permeabilized with PBS containing 0.3% Triton-X 100 for 10 min and blocked at room temperature for 1 h in PBS containing 2% albumin fraction V (Applichem, Darmstadt, Germany). Cells were then incubated with either anti-Nanog (rabbit, Millipore, ab9220), anti-Oct4 (rabbit, Abcam, ab19857), anti-Sox2 (mouse, Millipore, mab4343) or anti-ALP (mouse, Developmental Hybridoma Bank, B4-78) at a dilution of 1:500, 1:200, 1:200 and 1:100 respectively, in PBS containing 2% albumin fraction V (applichem) for 1 h at room temperature. Cells were then incubated with anti-rabbit IgG conjugated to Alexa Fluor 546 (donkey, Life Technologies, A10040) or anti-mouse IgG conjugated to Alexa Fluor 555 (goat, Life Technologies, A21424) at a dilution of 1:200 in PBS containing 2% albumin fraction V (Applichem). Cell nuclei were stained with DAPI (Life Technologies) at 3 µg/ml for 10 min at room temperature. Cells were mounted using ProLong^{®} Gold Antifade Reagent (Life Technologies) at room temperature over night. Images were acquired with a Leica TCS SP5 confocal microscope (Leica Microsystems, Mannheim, Germany) with a 63x PL APO CS 1.4 oil objective. Alexa Fluor 546 and Alexa Fluor 546 were excited with a 561 nm laser and DAPI was excited with a 405 nm laser. Images were acquired at a resolution of 512x512 pixels for a 245x245 µm field of view with a pinhole diameter of 95.5 µm. Images were asquired as z-stacks with a step size of 0.15 µm and processed as maximum z-projections.

Protein expression of core pluripotency factors Nanog, Oct4 and Sox2 as well as the pluripotency marker ALP (alkaline phosphatase) were analyzed by immunofluorescence in pPSCs (cESCs metabolically reprogrammed for 7 days; reprogramming as described in Example 1). WT ESCs served as negative control for the starting point. All core pluripotency markers and ALP were detected in pPSCs (cESCs on d7 of reprogramming) (see Fig. 4).

The protein expression levels of core pluripotency factors Nanog, Oct4 and Sox2 as well as of the LIF signaling marker phosphorylated Stat3 (P-Stat3) and total- (T-Stat3) were analysed by Western Blot in pPSCs metabolically reprogrammed for 7 days (reprogramming as described in Example 1). WT ESCs served as negative control for the starting point. While core pluripotency factors only sighlty decreased and mostly remained elevated, the ratio of P-Stat3 over T-Stat3 decreased strongly (see Fig. 5). This reduction is expected since cells are not stimulated by LIF anymore during metabolic reprogramming. LIF causes Stat3 phophorylation and activation and is required for the maintenance ofpluripotency in mESCs.

The expression of Oct4, Nanog and Sox2 in pPSCs suggest that the pPSCs are pluripotent. Since the ratio of P-Stat3 over T-Stat3 decreased significantly compared to WT ESCs, it can be concluded that pluripotency in pPSCs is maintained by a different mechanism than in WT ESCs.

### Example 3 - mRNA level of Pluripotency Markers during and after Metabolic Reprogramming

The **mRNA** expression profiles of pluripotency marker were analysed during and after metabolic reprogramming via real-time PCR. cPSCs (cESCs) were metabolically reprogrammed to pPSCs for 7days (d0 to d7) after which metabolic reprogramming conditions were removed and pPSCs were allowed to grow in base medium in the presence of βME only (d7 to d7+12). RNA was isolated for Real-Time PCR Analysis. PSCs (WT ESCs) served as control for the starting point. All mRNA expression levels were normalized to Gapdh mRNA expression levels.

### Real-Time PCR Analysis

Total RNA was isolated from ∼1×10⁶ cells per sample using the RNeasy Plus Mini Kit (Qiagen, Hombrechtikon, Switzerland) with RNase-free DNase treatment (Qiagen) following the manufacturer's protocol. For reverse transcription (RT) 2 µg total RNA was reverse transcribed into cDNA in a reaction volume of 20 µl using the iScript™ Advanced cDNA Synthesis Kit (Bio-Rad). Each RT reaction mix contained 4 µl 5x iScript advanced reaction mix and 1 µl iScript advanced reverse transcriptase. The 20 µl reactions were incubated at 42°C for 30 min, at 85°C for 5 min, and then held at 4°C. Real-Time PCR reactions were performed on the resulting cDNA using the CFX Connect™ Real-Time PCR Detection System (Bio-Rad), SsoAdvanced™ SYBR^{®} Green Supermix (Bio-Rad) and Hard-Shell^{®} Low-Profile Thin-Wall 96-Well Skirted PCR Plates (Bio-Rad). Briefly, following the RT step, 5 µl of the 100fold-diluted RT reaction was combined with 15 µl mastermix (2x SsoAdvanced™ SYBR^{®} Green Supermix, forward primer, reverse primer and nuclease-free water) in a 20 µl final volume. PCR reactions were incubated at 95°C for 3 min, followed by 39 cycles of 95°C for 10 s and 62°C for 30 s; and were run as duplicates of two independent experiments. PCR Primers were designed using PrimerSelect from the Lasergene software suite (DNASTAR, Madison, WI). All primers were synthesized at Microsynth (Microsynth, Balgach, Switzerland). Results were analyzed using CFX Manager™ software (Bio-Rad) and mRNA expression levels relative to Gapdh were calculated based on the ΔΔCt method. Gene abbreviations, NCBI reference sequences for mRNA (used as template for primer design), amplicon size and primer sequences are indicated in Table 1.

**Table 1**

| **Gene** | **mRNA ID (NCBI Reference Sequence)** | **Amplicon Size (bp)** | **Forward Primer Sequence (5'→3')** | **Reverse Primar Sequence (5'→3')** |
|---|---|---|---|---|
| Gapdh | NM_008084 | 90 | ACCCCCAATGTGTCCGTCGTG | AGATGCCTGCTTCACCACCTTCTTG |
| Nanog | AB093574 | 101 | ACCTCAGCCTCCAGCAGATGCAA | CCGCTTGCACTTCACCCTTGG |
| Oct4 | NM_013633 | 135 | TGCTGAAGCAGAAGAGGATCACCTTG | TGTTCTTAAGGCTGAGCTGCAAGGC |
| Sox2 | NM_011443.3 | 139 | AGGGTTCTTGCTGGGTTTTGATTCTG | AACGGTCTTGCCAGTACTTGCTCTCAT |
| Esrrb | NM 011934 | 262 | TGCCCGGGACCCAAGAGACATA | AGTGAGTTCCGGCTGGCTGAGGT |
| Nr5a2 | NM_030616 | 152 | CGCATGGGAAGGAAGGGACAAT | CCCCTGATCGAACTGAAGGGAAC |
| Klf4 | NM_010637 | 111 | GCCATTATTGTGTCGGAGGAAGAGGA | GCTCCCCCGTTTGGTACCTTTAGAAC |
| Tbx3 | NM_011535 | 127 | CGCCCTGTCCCTTTCAGTTTTGTC | AGTCCCGCGTTTCAAAGCAACAG |
| Dppa3 | NM_139218 | 181 | TGCAGCCGTACCTGTGGAGAACA | GTCCCGTTCAAACTCATTTCCTTCG |
| Dazl | NM_010021 | 262 | ACCTCCGGCTTATACAACTGTTAACTACCA | AAGCACTGCCCGACTTCTTCTGAA |
| Stra8 | NM_009292 | 130 | CAGCGCTATGTTTGCCACCTGC | TGGGGGCTCTGGTTCCTGGTT |
| Piwi12 | NM_021308 | 152 | TCCGCAAGGACAGAGAAGAACCC | TGCTCGTCCCAGTGGTAACAGAGAG |
| Prdm14 | NM_001081209 | 113 | CGCCACCACCGAGGAGGAGT | CCGGGTTCAACAAGGGAGCAGT |
| Rex1 | NM_009556 | 144 | TGGCTGCGAGAAGAGCTTTATTCAGTC | CGTGTATCCCCAGTGCCTCTGTCAT |
| Dax1 | MM_M7430 | 115 | GCGAGTGGTGGCAGCTGTCCTAC | GGCTGGTCTTCACCGCACACATAG |
| Fbxo15 | NM_015798 | 125 | GTGGGGCTGTGGCAGGAGAATG | GTAGTGTCGGGAGGCAATGTATAGGGAA |
| Nodal | NM_013611 | 194 | GAGGGCCCACTCACCATTGACATT | TCCAGTGCCCTGGGGTCCTTTAG |
| Eoraes | NM_010136 | 204 | GGCGCATGTTTCCTTTCTTGAGC | AGTGGGAGCCAGTGTTAGGAGATTCTG |
| Gata6 | NM_010258 | 161 | TGGCGTAGAAATGCTGAGGGTGAG | GTGTTACCGGAGCAAGCTTTTGACTTATT |
| Foxa2 | NM_010446 | 164 | CGCTCGGGACCCCAAGACATAC | TCTGCCGGTAGAAAGGGAAGAGGTC |
| Cerl | NM_009887 | 143 | CTGCCCATCAAAAGCCACGAAGTA | CCGGGAAAACGAATGGAAGTGC |
| Sox17 | NM_011441 | 60 | CGGCCGGCACCTACACTTACG | CGGCCGGCTCTACGGACACT |
| T | NM_009309 | 151 | TGCTTTCCCGAGACCCAGTTCATAG | AGTCCCCCCGTTCCTCCATTACAT |
| Fgf5 | NM_010203 | 94 | GGACAGAGGCCACCGCACACTAA | AATGAGGGCAGGGGGCAGATAAAA |
| Nanos2 | NM_194064 | 76 | ACCGGCCACCAGGCTCATACAC | GCCCACTGCGTCGGTAGAGAGACT |
| Tdrd1 | NM_001002238 | 244 | GTGGCCTGGCAGAAAACCTCACTT | CTGGCGTTTGCTGTCTCTTTCTTCC |
| Ddx4 | NM_010029 | 100 | AACGCCAAACCTTTATTCAGTGCTAC | TGCCCAACAGCGACAAACAAGTAACT |
| Zbtb16 | NM_001033324 | 86 | CCGCCCATTTTTACCCCCTACAA | ACCCCAGCCCATATCCTCTCAACA |
| Plk1s1 | NM_001033298 | 132 | AGATTCGCCTTCACGGAATATGTTTTACA | CATTGCCCTCCTGTCTTCAATAAGTGAC |
| Cxcr4 | NM_009911 | 157 | GACCGCCTTTACCCCGATAGCC | TGAGGGCCTTGCGCTTCTGG |
| Tex101 | NM_019981 | 225 | CTAATCGCCTCACGTTGGACTCTGG | CACCGCCTCTCCTCCTTGAGAAAC |
| p15 | NM_007670 | 133 | CCGCCTGCCGGTAGACTTGG | CTAGATGGGGCTGGGGAGAAAGAAG |
| p16 | NM_001040654 | 73 | CAAGAGCGGGGACATCAAGACATC | ACCACCCAGCGGAACACAAAGAG |
| p21 | NM_007669 | 65 | AGATCCACAGCGATATCCAGACATTCA | TCGGACATCACCAGGATTGGACAT |
| p27 | NM_009875 | 141 | AAGCACTGCCCGCATATGGAAGAA | GCGCGGGGGCCTGTAGTAGAA |
| p57 | NM_009876 | 119 | CTGAACGCCGAGGACCAGAACC | CGGTAGAAGGCGGGCACAGACT |
| Nqo1 | NM_008706 | 79 | CATCCTGCGTTTCTGTGGCTTCC | GCGGGCATCTGGTGGAGTGTG |
| Srxn1 | NM_029688 | 113 | TTGGCTGCATTGGCTTGGTTACTCT | CGCTCCAGGCCCTCTCACTACTACTC |
| Gclc | NM_010295 | 142 | TGCGGAGGCATCAAAGGGTTCT | AGTGGCCAGGTGATCATAAAGGTATCTTG |
| Gclm | NM_008129 | 200 | ATGGCTTCGCCTCCGATTGAA | CAGGAGGCCAGGTTAACTTGGTTACTATT |
| Gstin1 | NM_010358 | 144 | ACGTCCGCGGACTGACACACC | AGTCCAGGCCCAGCTTGAACTTCTC |
| Cox7a1 | NM_009944 | 93 | GAAAGGCGGGGGAATGGACAAC | GCCCAGCCCAAGCAGTATAAGCAGTA |
| Cox6b2 | NM_183405 | 95 | CCTTTGATCCGCGCTTCCCTAAC | GCGGCGATTCATGGTCTTCACAC |
| cox8c | NM_001039049 | 103 | GCCGCCTCAGCCACTCAGAAAG | GCAGCTGGGATGTAAAAGGTCGTAAAAA |
| Sco2 | NM_001111288 | 186 | CCGCGAGGGCTGAGAAGGAAC | CGGGGCAAATATCAGGGCAGTG |
| Ldha | NM_010699 | 127 | AGCGCGGTTCCGTTACCTGATG | GACGCCGGCAACATTCACACC |
| Pdkt | NM_172665 | 255 | GCTAGGCGGCTTTGTGATTTGTATTATGTTA | TCCCCGGTCACTCATCTTCACAGTC |
| Pygl | NM_133198 | 252 | CAGACGGGGACAAAGTGGGTCG | CAGCCGCAACTCCTTCCCTTCA |
| Gys1 | NM_030678 | 241 | AAGCCGTGCGCAAACAAGTATGG | ATTCGGCGGATGGTGGTCAAGAT |
| G6pd | NM_008062 | 111 | CTATGCCCGCTCACGACTCACAGT | GAATTACGGGCAAAGAACTCCTCCAG |
| Pgd | NM_001081274 | 146 | GAACCGGAGAACCCTGCTGTGACT | ATGCCGCATGCCCAGAACATC |
| Taldo1 | NM_011528 | 191 | GGTGGGCCTCAAGAGGAGCAGAT | TTGCCGACCCCAGCTTCTTTGTA |
| Tkt | NM_009388 | 210 | GCCACGCCAGTGACCGTATCATT | GGTCGAAGGCCCGTGTGAAGAA |
| Mel | NM_008615 | 294 | TGCCTTGGGGATTGCTCACTTG | ATGATGGGCCGCTCGTTGAAG |
| Idh1 | NM_00111320 | 180 | CCTGGGCCTGGAAAAGTAGAGATAACC | AGGCCAGCCCTTGGACAGAGC |
| Gata4 | NM_008092 | 215 | TGCCGAGGGTGAGCCTGTATGTAAT | TGCTGCTAGTGGCATTGCTGGAGT |
| Nkx2.5 | NM_008700 | 156 | GACCCTCGGGCGGATAAAAAAGAG | GCGCCGCTCCAGCrCGTAGA |
| Mef2c | NM_001170537 | 102 | ATCTCCGCGTTCTTATCCCACCTG | CCACCGGGGTAGCCAATGACTG |
| M1c2v | NM_010861 | 186 | AGGGCTCAGTCCTTCTCTTCTCCGT | TCCCGAGGGCAAAGGGTCACT |
| aMhc | NM_010856 | 252 | CGCATCAAGGAGCTCACCTACCAGA | GGCACCAATGTCCCGGCTCTT |

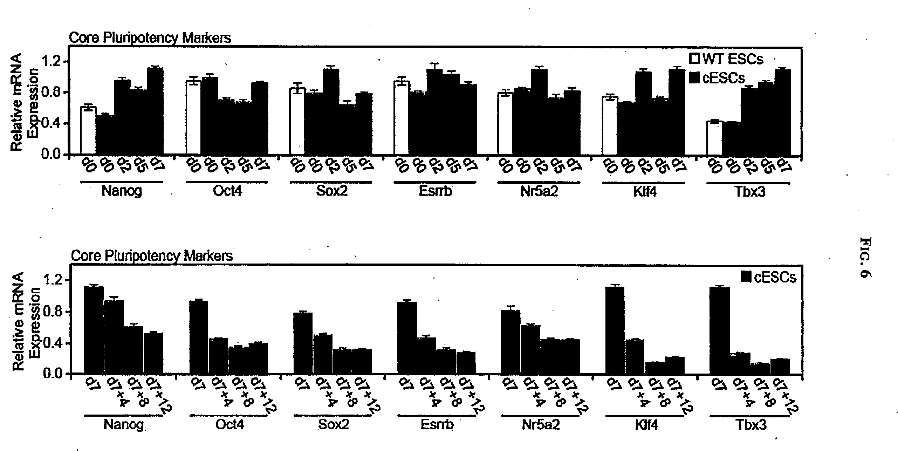

In Fig. 6 to 8 the upper panel shows mRNA expression levels from d0 to d7 during the metabolic reprogramming process while the lower panel shows mRNA expression levels from d7 to d7+12 when the metabolic reprogramming conditions were removed. During the removal ofreprogramming conditions pPSCs were cultured in BM (described in Example 1) supplemented with 0.1 mM βME and medium replaced every day.

The mRNA expression levels of core pluripotency markers Nanog, Oct4, Sox2, Esrrb, Nr5a2, Klf4 and Tbx3 are shown in Fig. 6. All core pluripotency marker expression levels remain unchanged except Tbx3 which shows a gradual upregulation from d0 to d7 during metabolic reprogramming. After metabolic reprogramming is stopped, all core plutipotency markers are downregulated.

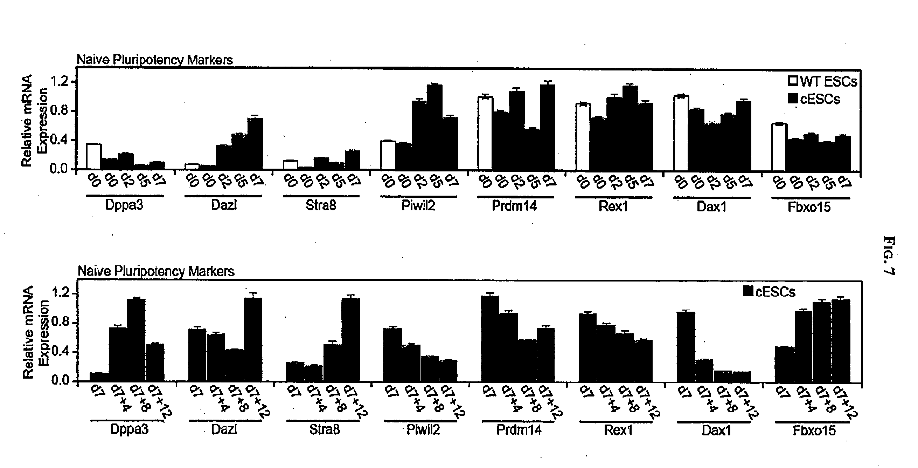

The mRNA expression levels of naïve pluripotency markers (all genes shown) and germ cell markers (Dazl, Piwil2 and Stra8) are shown in Fig. 7. During metabolic reprogramming Dazl and Piwil2 are upregulated while Stra8 is only slightly upregulated, Dppa3 is slighty downregulated and the other markers remain unchanged. After metabolic reprogramming is stopped Dppa3 and Stra8 are upregulated while all other markers except Fbxo15 are downregulated. Fbxo15 shows a gradual upregulation from d7 until d7+12. Dazl is also upregulated again on d7+12.

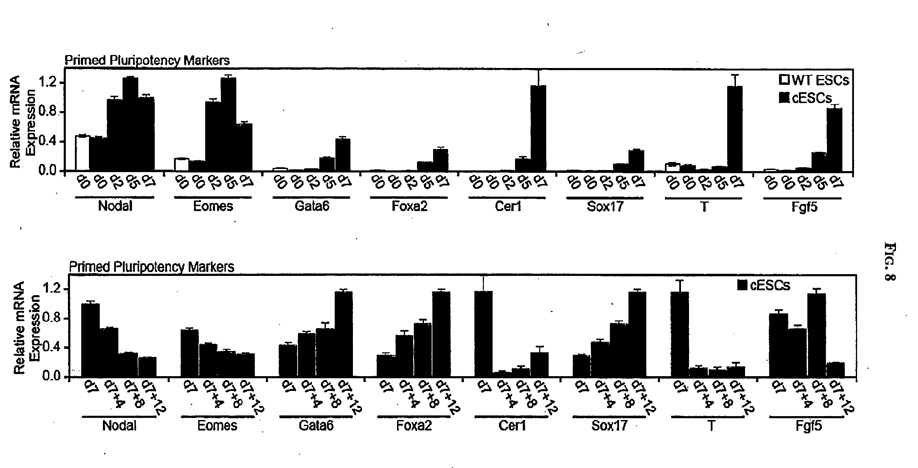

The mRNA expression levels of primed pluripotency marker (all genes shown) and late epiblast/early germ-layer markers (Sox17 for endoderm, T for mesoderm, Fgf5 for ectoderm) are shown in Fig. 8. All primed pluripotency markers are upregulated during metabolic reprogramming while the late-stage markers Sox17, T and Fgf5 are upregulated only from d5 onwards. After metabolic reprogramming is stopped Nodal, Eomes, Cer1, T and Fgf5 levels fal1, while Gata6 Foxa2 and Sox17 show increased expression. At d7+12 Cer1 is also upregulated again.

The mRNA expression of the core pluripotency factors Nanog, Oct4, Sox2, Esrrb, Nr5a2, Klf4 and Tbx3 further imply a pluripotent nature of pPSCs. The upregulation of Tbx3 during metabolic reprogramming is a first hint towards a germ cell characteristic of pPSCs, since it is known that Tbx3 improves the germ line competency of ESCs. The downregulation of the core pluripotency factors after withdrawal of reprogramming conditions confirms that pluripotency was maintained by the reprogramming conditions. The upregulation of Dazl and Piwil2 during metabolic reprogramming strongly imply that pPSCs assume a germ cell character. Renewed upregulation of Dazl after metabolic reprogramming implies that pPSCs give can give rise to other germ cell lincages. Downrcgulation of Dppa3 and only weak upregulation of Stra8 during reprogramming, but strong upregulation after release from reprogramming imply that the germ cell state induced by reprogramming conditions is very specific. The upregulation of primed pluripotency markers during reprogramming implies that pluripotency is maintained by very different mechanisms in pPSCs as compared to WT ESCs. Moreover the expression of T has been shown to be necessary for the maintenance ofSSCs. Further upregulation of Gata6, Foxa2 and Sox17 after metabolic reprogramming implies that these genes may be involved in the maintenance of germ cells that spontaneously emerge from pPSCs after reprogramming. Taken together these data imply that pPSCs are pluripotent, while pluripotency is not linked to a naïve state, but to a germ cell-like state.

### Example 4 - mRNA level of germ cell markers during and after metabolic reprogramming

cPSCs (cESCs) were metabolically reprogrammed to pPSCs for 7days (d0 to d7) after which metabolic reprogramming conditions were removed and pPSCs were allowed to grow in base medium in the presence of βME only (d7 to d7+12). RNA was isolated for Real-Time PCR Analysis. PSCs (WT ESCs) served as control for the starting point. All mRNA expression levels were normalized to Gapdh mRNA expression levels.

RT-PCR Analysis was performed as described in Example 3.

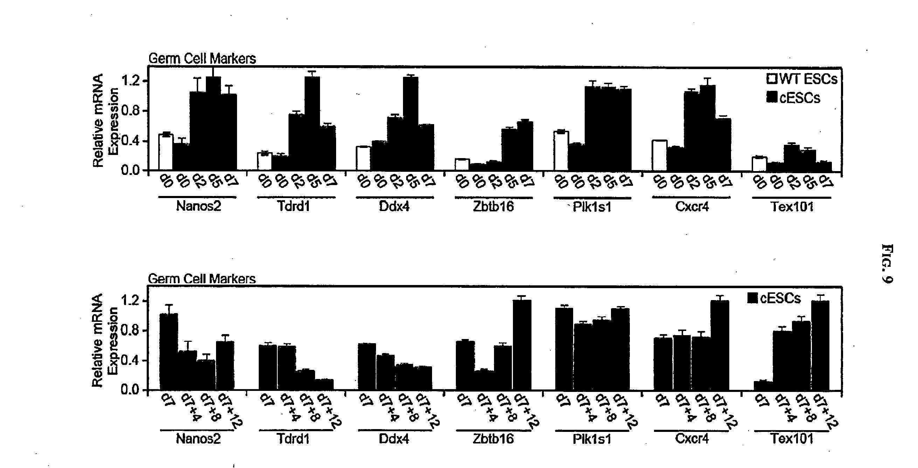

In Fig. 9 the mRNA expression levels of the germ cell markers Nanos2, Tdrd1, Ddx4, Zbtb16, Plk1s1, Cxcr4 and Tex101 are shown, of which Nanos2, Tdrd1, Ddx4, Zbtb16 and Cxcr4 are specific for spermatogonial stem cells (SSCs), while Plk1s1 is speficic for spermatocytes and spermatids and Tcx101 is specific for gonocytes, spermatocytes and spermatids. The upper panel shows mRNA expression levels from d0 to d7 during the metabolic reprogramming process while the lower panel shows mRNA expression levels d7 to d7+12 when the metabolic reprogramming conditions were removed. All germ cell markers are strongly upregulated during metabolic reprogramming, except for Tex101 which does not show increased expression during reprogramming.

Nanos2 is a master regulator of SSC identity and self-renewal. So far Nanos2 was known to only be activated by the presence of glial cell-line derived neurotrophic factor (GDNF). Here, Nanos2 expression was induced without addition of GDNF. This is important, since GDNF is known to exhibit tumorigenic effects.

After metabolic reprogramming is stopped germ cell markers fall except for Plk1s1 which remains at the same elevated expression levels, while Zbtb16 shows a gradual increase again until d7+12 and Tex101 shows a significant gradual upregulation until d7+12.

These results evidence that the reprogrammed pPSCs are non-tumorigenic and exhibit germ cell characteristics. During reprogramming pPSCs upregulate mRNA expression of key SSC specific genes, which strongly implies that pPSCs may be useful for restoration of fertility, especially when transplanted into the testis, since it has been shown that SSCs when transplanted into sterilized testis are able to resume spermatogenesis and restore fertility. The upregulation of Tex101 after reprogramming strongly implies that gonocytes or Further differentiated germ cell lineages such as spermatocytes spontaneously emerge from pPSCs which further evidences the germ cell potential of pPSCs.

### Example 5 - mRNA level of CDK inhibitors during and after metabolic Reprogramming

Conditioned cPSCs (cESCs) were metabolically reprogrammed to pPSCs for 7 days (d0 to d7) after which metabolic reprogramming conditions were removed and pPSCs were allowed to grow in base medium in the presence of βME only (d7 to d7+12). RNA was isolated for Real-Time PCR Analysis. PSCs (WT ESCs) served as control for the starting point. All mRNA expression levels were normalized to Gapdh mRNA expression levels.

RT-PCR Analysis was performed as described in Example 3.

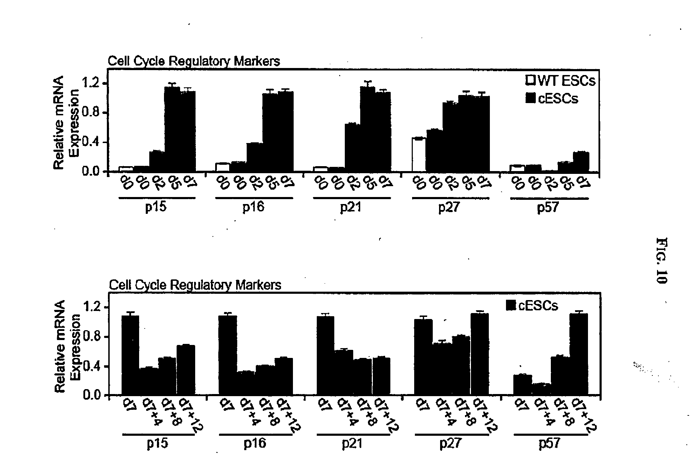

Fig. 10 shows the mRNA expression levels of CDK inhibitors (tumor suppressors) p15, p16, p21, p27 and p57. The upper panel shows mRNA expression levels from d0 to d7 during the metabolic reprogramming process while the lower panel shows mRNA expression levels d7 to d7+12 when the metabolic reprogramming conditions were removed. All CDK inhibitors except p57 are upregulated during metabolic reprogramming. After metabolic reprogramming is stopped CDK inhibitors levels decrease but do not fall back to their original expression levels, while p57 and p27 show a gradual increase until d7+12.

These result evidence the non-tumorigenic nature of the reprogrammed pPSCs.

### Example 6 - Metabolic markers during and after metabolic Reprogramming

cPSCs (cESCs) were metabolically reprogrammed to pPSCs for 7 days (d0 to d7) after which metabolic reprogramming conditions were removed and pPSCs were allowed to grow in base medium in the presence of βME only (d7 to d7+12). RNA was isolated for Real-Time PCR Analysis. PSCs (WT ESCs) served as control for the starting point. All mRNA expression levels were normalized to Gapdh mRNA expression levels.

RT-PCR Analysis was performed as described in Example 3.

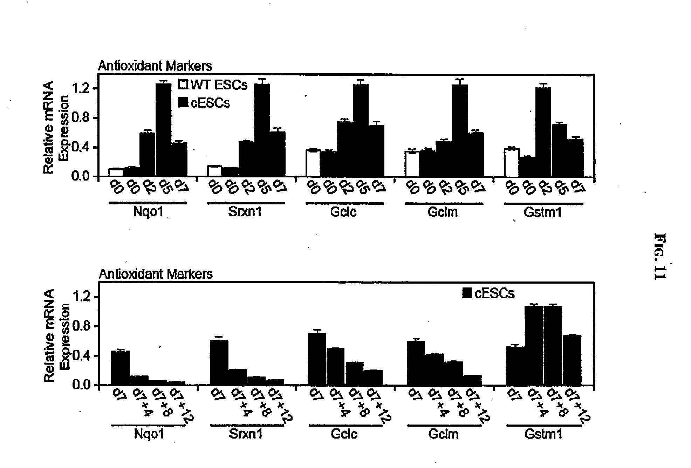

Fig. 11 shows the mRNA expression levels of Nrf2-target antioxidant enzymes Nqo1, Srxnl, Gclc, Gclm and Gstm1. The upper panel shows mRNA expression levels from d0 to d7 during the metabolic reprogramming process while the lower panel shows mRNA expression levels d7 to d7+12 when the metabolic reprogramming conditions were removed. All antioxidant markers are upregulated during metabolic reprogramming and fall back to their original levels after metabolic reprogramming is stopped.

The subcellular localization of the antioxidation regulator Nrf2 was immunofluorescently detected in cPSCs (cESCs) metabolically reprogrammed for 2 days (reprogramming as described in Example 1). As a negative control, PSCs (WT ESCs) which were previously passaged in BGM supplemented with 0.03% (v/v) DMSO, were only subjected to removal of βME, removal of LIF and removal of puromycin (not subjected to metabolic reprogramming), but otherwise treated in the same way as cESCs.

Immunofluorescent staining was performed as described in Example 2. Primary antibody was anti-Nrf2 (rabbit, Abcam, ab31163) used at 1:100 dilution.

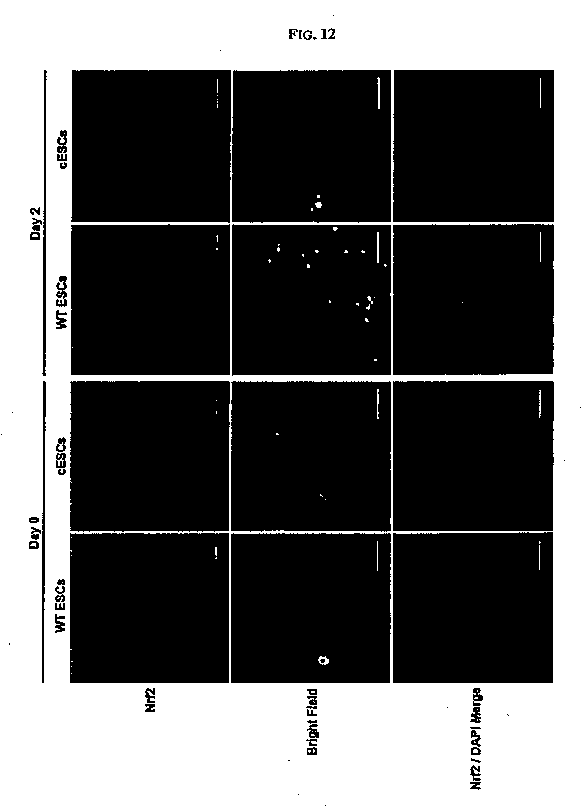

Nrf2 Protein is shown in red and DAPI staining is shown in blue. On day 2 Nrf2 protein is depleted from the cytoplasm of cPSCs that were subjected to metabolic reprogramming, but not in PSCs (see Fig. 12).

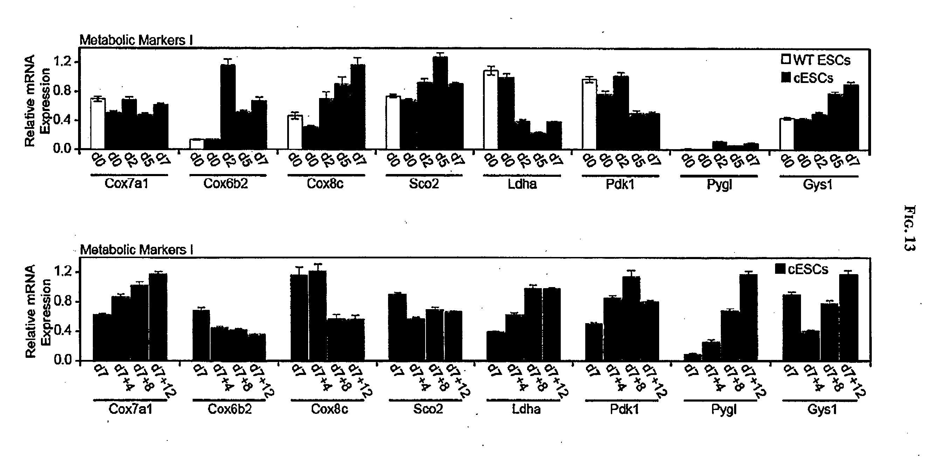

Fig. 13 shows the mRNA expression levels of oxidative phosphorylation (Cox7a1, Cox6b2, Cox8c and Sco2), glycolysis (Ldha, Pdk1) and glycogen metabolism (Pygl, Gys1). Oxidative Phosphorylative markers, except Cox7a1, are upregulated, glycolysis markers are downregulated and Gys1 is upregulated during metabolic reprogramming. After metabolic reprogramming is stopped, Cox7a1 is upregulated, the other oxidative phosphorylative markers are downregulated, glycolysis markers are upregulated and Gys1 and Pygl are upregulated.

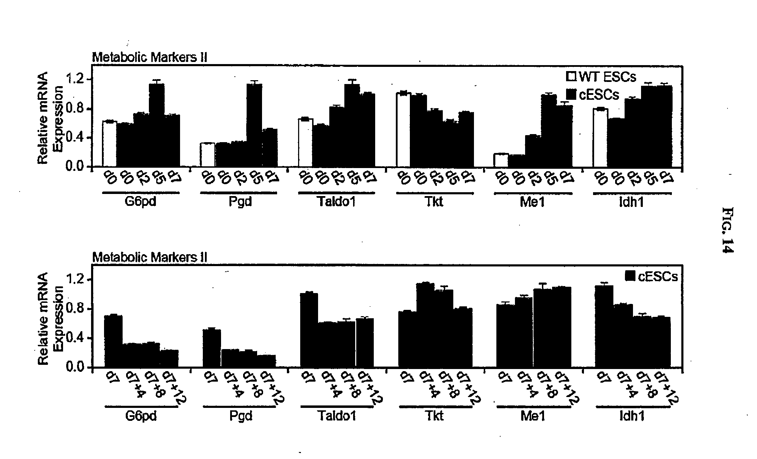

Fig. 14 shows the mRNA expression levels of pentose phosphate pathway (G6pd, Pgd, Taldo1 and Tkt) and NADPH generating enzymes (Me1 and Idh1). Pentose phosphate pathway markers, except Tkt, are upregulated and NADPH generating enzyme markers are upregulated during metabolic reprogramming. After metabolic reprogramming is stopped Tkt is slightly upregulated, but decreasing again on d7+12, while the other pentose phosphate pathway markers are downregulated, Me1 remains at an upregulated expression level and Idh1 is downregulated.

Nrf2 was depleted selectively in the cytoplasm of cPSCs undergoing reprogramming, but was still located in the nucleus. The oxidative phosphorylative and non-glycolytic metabolic switch also including increased glycogen synthase activity (Gys1), but reduced glycogen phosphorylase activity (Pygl), increased pentose phosphate pathway activity was confirmed. Furthermore an increased mRNA expression of NADPH generating enzymes Me1 and Idh1 was observed during metabolic reprogramming. The presence of tBHQ increases ROS which takes a central role in ROS signaling during metabolic reprogramming of cPSCs into pPSCs. Since PSCs normally cannot survive without exogenous antioxidants due to increased ROS and oxidative stress, the fact that βME was removed during metabolic reprogramming is a mechanism by which non-reprogrammed PSCs are eliminated by increasing ROS. pPSCs however are stabilized in their properties by the increase in ROS. Hence, PSCs were transformed into non-tumorigenic PSCs, specifically non-tumorigenic transiently cell cycle-arrested SSC-like cells by the method of creating pPSCs described in the invention.

### Example 8 - Proliferation After Removal of Metabolic Reprogramming Conditions

After the 7 days of metabolic reprogramming, all reprogramming and conditioning factors were removed and βME was added back to the culture medium to analyse whether pPSCs would resume proliferation.

During the removal of reprogramming conditions pPSCs were cultured in BM (described in Example 1) supplemented with 0.1 mM βME and medium replaced every day. pPSCs were fixed and analysed by light microscopy on d7+4 (4 days after removing reprogramming conditions) as described in example 1.

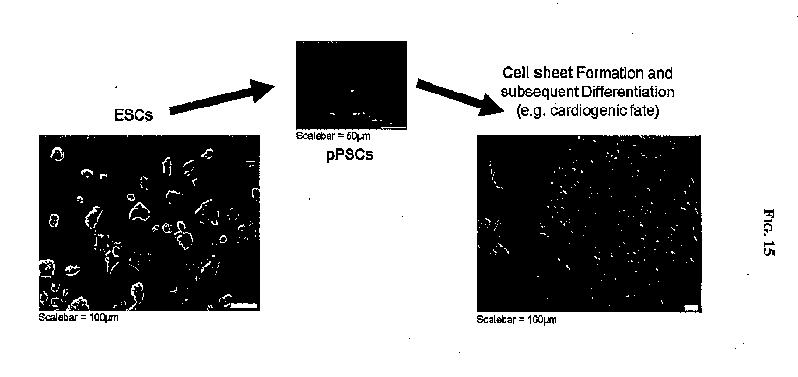

pPSCs did indeed restart proliferation, but did not show the original morphology of mouse ESCs. Instead released pPSCs were forming flat cell sheets growing radially from the original pPSC colonies (see Fig. 15). It can be seen on the light microscopy images that from the original cell colonies of pPSCs flat cell sheets radially grew out. This proves that pPSCs are able to resume proliferation, but do not fall back to the phenotype of their original PSC identity. Thus, reprogrammed pPSCs can be arrested in proliferation for up to 7 days and can resume proliferation subsequentially.

### Example 9 - Cardiogenic Differentiation

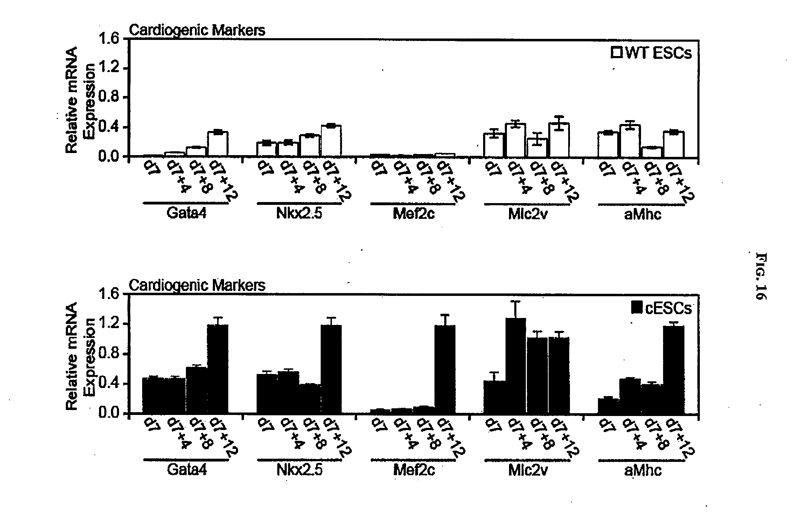

pPSCs (cESCs d7, Fig. 16, lower panel) and PSCs (WT ESCs, Fig. 16, upper panel) were subjected to 12 days stimulation with µM icariin to induce cardiogenic differentiation (d7 to d7+12). RNA was isolated every 4 days. Real-Time PCR Analysis showed that the mRNA expression of all tested cardiogenic markers were induced higher in pPSCs than in PSCs. All mRNA expression levels were normalized to Gapdh mRNA expression levels.

The culture medium during cardiogenic differentiation of pPSCs consisted of BM (described in Example 1) supplemented with 0.1 mM βME and 0.1 µM icariin (Sigma-Aldrich, I1286) while medium was replaced every day.

RT-PCR Analysis was performed as described in Example 3.

Mef2c, Mlc2v and aMhc mRNA expression did not show an increase in PSCs, but was strongly induced in pPSCs. It was also observed that in pPSCs spontaneously beating cells appeared after around d7+7. These results show that pPSCs are better suited for directed differentiation compared to PSCs.

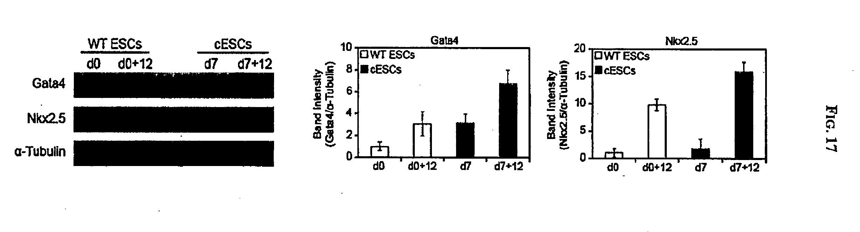

Western Blot Analysis (see Fig. 17) showed that the cardiogenic markers Gata4 and Nkx2.5 were more induced in pPSCs than in PSCs.

Western Blot Analysis was performed as described in Example 1. Primary antibodies and their dilutions were 1:1000 for Nkx2.5 (rabbit, Thermo Scientific, PA5-21686) and GATA4 (rabbit, Abcam, ab84593).

Our data therefore show that we have solved the problem of PSC tumorigenicity and have at the same time solved the problem of generating non-tumorigenic SSCs from PSCs. These pPSCs can be directly implanted into testis for fertility restoration without tumor formation. Our pPSCs are also a good source for *in vitro* differentiation of germ cells such as *in vitro* generation of mature spermatozoa and oocytes as well as a useful tool for (fertility) research. We exploited post-translational, epigenetic and metabolic cellular mechanisms to achieve this goal.

### SEQUENCE LISTING- FREE TEXT INFORMATION

SEQ ID NO: 1 ACCCCCAATGTGTCCGTCGTG
SEQ ID NO:2 AGATGCCTGCTTCACCACCTTCTTG
SEQ ID NO: 3 ACCTCAGCCTCCAGCAGATGCAA
SEQ ID NO: 4 CCGCTTGCACTTCACCCTTTGG
SEQ ID NO: 5 TGCTGAAGCAGAAGAGGATCACCTTG
SEQ ID NO: 6 TGTTCTTAAGGCTGAGCTGCAAGGC
SEQ ID NO: 7 AGGGTTCTTGCTGGGTTTTGATTCTG
SEQ ID NO: 8 AACGGTCTTGCCAGTACTTGCTCTCAT
SEQ ID NO: 9 TGCCCGGGACCCAAGAGACATA
SEQ ID NO: 10 AGTGAGTTCCGGCTGGCTGAGGT
SEQ ID NO: 11 CGCATGGGAAGGAAGGGACAAT
SEQ ID NO: 12 CCGCTGATCGAACTGAAGGGAAC
SEQ ID NO: 13 GCCATTATTGTGTCGGAGGAAGAGGA
SEQ ID NO: 14 GCTCCCCCGTTTGGTACCTTTAGAAC
SEQ ID NO: 15 CGCCCTGTCCCTTTCAGTTTTGTC
SEQ ID NO: 16 AGTCCCGCGTTTCAAAGCAACAG
SEQ ID NO: 17 TGCAGCCGTACCTGTGGAGAACA
SEQ ID NO: 18 GTCCCGTTCAAACTCATTTCCTTCG
SEQ ID NO: 19 ACCTCCGGCTTATACAACTGTTAACTACCA
SEQ ID NO: 20 AAGCACTGCCCGACTTCTTCTGAA
SEQ ID NO: 21 CAGCGCTATGTTTGCCACCTGC
SEQ ID NO: 22 TGGGGGCTCTGGTTCCTGGTT
SEQ ID NO: 23 TCCGCAAGGACAGAGAAGAACCC
SEQ ID NO: 24 TGCTCGTCCCAGTGGTAACAGAGAG
SEQ ID NO: 25 CGCCACCACCGAGGAGGAGT
SEQ ID NO: 26 CCGGGTTCAACAAGGGAGCAGT
SEQ ID NO: 27 TGGCTGCGAGAAGAGCTTTATTCAGTC
SEQ ID NO: 28 CGTGTATCCCCAGTGCCTCTGTCAT
SEQ ID NO: 29 GCGAGTGGTGGCAGCTGTCCTAC
SEQ ID NO: 30 GGCTGCTCTTCACCGCACACATAG
SEQ ID NO: 31 GTGGGGCTGTGGCAGGAGAATG
SEQ ID NO: 32 GTAGTGTCGGGAGGCAATGTATAGGGAA
SEQ ID NO: 33 GAGGGCCCACTCACCATTGACATT
SEQ ID NO: 34 TCCAGTGCCCTGGGGTCCTTTAG
SEQ ID NO: 35 GGCGCATGTTTCCTTTCTTGAGC
SEQ ID NO: 36 AGTGGGAGCCAGTGTTAGGAGATTCTG
SEQ ID NO: 37 TGGCGTAGAAATGCTGAGGGTGAG
SEQ ID NO: 38 CTGTTACCGGAGCAAGCTTTTGACTTATT
SEQ ID NO: 39 CGCTCGGGACCCCAAGACATAC
SEQ ID NO: 40 TCTGCCGGTAGAAAGGGAAGAGGTC
SEQ IDNO: 41 CTGCCCATCAAAAGCCACGAAGTA
SEQ ID NO: 42 CCGGGAAAACGAATGGAACTGC
SEQ ID NO: 43 CGGCCGGCACCTACACTTACG
SEQ ID NO: 44 CGGGCGGCTCTACGGACACT
SEQ ID NO: 45 TGCTTTCCCGAGACCCAGTTCATAG
SEQ ID NO: 46 AGTCCCCCGGTTCCTCCATTACAT
SEQ ID NO: 47 GGACAGAGGCCACCGCACACTAA
SEQ ID NO: 48 AATGAGGGCAGGGGGCAGATAAAA
SEQ ID NO: 49 ACCGGCGACCAGGCTCATACAC
SEQ ID NO: 50 GCCCACTGCGTCGGTAGAGAGACT
SEQ ID NO: 51 GTGGCCTGGCAGAAAACCTCACTT
SEQ TD NO: 52 CTGGCGTTTGCTGTCTCTTTCTTCC
SEQ ID NO: 53 AACGCCAAACCCTTTTATTCAGTGCTAC
SEQ ID NO: 54 TGCCCAACAGCGACAAACAAGTAACT
SEQ ID NO: 55 CCGCCCATTTTTACCCCCTACAA
SEQ ID NO: 56 ACCCCAGCCCATATCCTCTCAACA
SEQ ID NO: 57 AGATTCGCTCTTCACGGAATATGTTTTACA
SEQ ID NO: 58 CATTGCCCTCGTGTCTTCAATAAGTGAC
SEQ ID NO: 59 GACCGCCTTTACCCCGATAGCC
SEQ ID NO: 60 TGAGGGCCTTGCGCTTCTGG
SEQ ID NO: 61 CTAATCGCCTCACGTTGGACTCTGG
SEQ ID NO: 62 CACCGCCTCTCCTCCTTGAGAAAC
SEQ ID NO: 63 CCGCCTGCCGGTAGACTTGG
SEQ ID NO: 64 CTAGATGGGGCTGGGGAGAAAGAAG
SEQ ID NO: 65 CAAGAGCGGGGACATCAAGACATC
SEQ ID NO: 66 ACCACCCAGCGGAACACAAAGAG
SEQ ID NO: 67 AGATCCACAGCGATATCCAGACATTCA
SEQ ID NO: 68 TCGGACATCACCAGGATTGGACAT
SEQ ID NO: 69 AAGCACTGCCGGGATATGGAAGAA
SEQ ID NO: 70 GCGCGGGGGCCTGTAGTAGAA
SEQ ID NO: 71 CTGAACGCCGAGGACCAGAACC
SEQ ID NO: 72 CGGTAGAAGGCGGGCACAGACT
SEQ ID NO: 73 CATCCTGCGTTTCTGTGGCTTCC
SEQ ID NO: 74 GCGGGCATCTGGTGGAGTGTG
SEQ ID NO: 75 TTGGCTGCATTGGCTTGGTTACTCT
SEQ ID NO: 76 CGCTCCAGGCCCTCTCACTACTACTC
SEQ ID NO: 77 TGCGGAGGCATCAAAGGCTTCT
SEQ ID NO: 78 AGTGGCCAGCTGATCATAAAGGTATCTTG
SEQ ID NO: 79 ATGGCTTCGCCTCCGATTGAA
SEQ ID NO: 80 CAGGAGGCCAGGTTAACTTGGTTACTATT
SEQ ID NO: 81 ACGTCCGCGGACTGACACACC
SEQ ID NO: 82 AGTCCAGGCCCAGCTTGAACTTCTC
SEQ ID NO: 83 GAAAGGCGGGGGAATGGACAAC
SEQ ID NO: 84 GCCCAGCCCAAGCAGTATAAGCAGTA
SEQ ID NO: 85 CCTTTGATCCGCGCTTCCCTAAC ,
SEQ ID NO: 86 GCGGCGATTCATGGTCTTCACAC
SEQ ID NO: 87 GCCGCCTCAGCCACTCAGAAAG
SEQ ID NO: 88 GCAGCTGGGATGTAAAAGGTCGTAAAAA
SEQ ID NO: 89 CCGCGAGGGCTGAGAAGGAAC
SEQ ID NO: 90 CGGGGCAAATATCAGGGCAGTG
SEQ ID NO: 91 AGCGCGGTTCCGTTACCTGATG
SEQ ID NO: 92 GACGCCGGCAACATTCACACC
SEQ ID NO: 93 GCTAGGCGGCTTTGTGATTTGTATTATGTTA
SEQ ID NO: 94 TCCCCGGTCACTCATCTTCACAGTC
SEQ ID NO: 95 CAGACGGGGACAAAGTGGGTCG
SEQ ID NO: 96 CAGCCGCAACTCCTTCCCTTCA
SEQ ID NO: 97 AAGCCGTGCGCAAACAACTATGG
SEQ ID NO: 98 ATTCGGCGGATGGTGGTCAAGAT
SEQ ID NO: 99 CTATGCCCGCTCACGACTCACAGT
SEQ ID NO: 100 GAATTACGGGCAAAGAACTCCTCCAG
SEQ ID NO: 101 GAACCGGAGAACCCTGCTGTGACT
SEQ ID NO: 102 ATGCCGCATGCCCAGAACATC
SEQ ID NO: 103 GGTGGGCCTCAAGAGGAGCAGAT
SEQ ID NO: 104 TTGCCGACCCCAGCTTCTTTGTA
SEQ ID NO: 105 GCCACGCCAGTGACCGTATCATT
SEQ ID NO: 106 GGTCGAAGGCCCGTGTGAAGAA
SEQ ID NO: 107 TGCCTTGGGGATTGCTCACTTG
SEQ ID NO: 108 ATGATGGGCCGCTCGTTGAAG
SEQ ID NO: 109 CCTGGGCCTGGAAAAGTAGAGATAACC
SEQ ID NO: 110 AGGCCAGCCCTTGGACAGAGC
SEQ ID NO: 111 TGCCGAGGGTGAGCCTGTATGTAAT
SEQ ID NO: 112 TGCTGCTAGTGGCATTGCTGGAGT
SEQ ID NO: 113 GACCCTCGGGCGGATAAAAAAGAG
SEQ ID NO: 114 GCGCCGCTCCAGCTCGTAGA
SEQ ID NO: 115 ATCTCCGCGTTCTTATCCCACCTG
SEQ ID NO: 116 CCACCGGGGTAGCCAATGACTG
SEQ ID NO: 117 AGGGCTCAGTCCTTCTCTTCTCCGT
SEQ ID NO: 118 TCCCGAGGGCAAAGGGTCACT
SEQ ID NO: 119 CGCATCAAGGAGCTCACCTACCAGA
SEQ ID NO: 120 GGCACCAATGTCCCGGCTCTT

## Claims

1. A method of reprogramming pluripotent stem cells (PSC) comprising the steps
(a) conditioning PSCs epigenetically, optionally conditioning PSCs post-translationally, and
(b) metabolically reprogramming PSCs.

2. The method of claim 1, wherein tumorigenicity of the PSCs is eliminated and/or the proliferation of the PSCs is arrested reversibly.

3. The method of any of claims 1 to 2, wherein the PSCs are epigenetically conditioned by altering, preferably increasing or decreasing, histone acetylation, and/or by altering, preferably increasing or decreasing, genome methylation, more preferably by increasing or decreasing histone methylation and/or DNA methylation, and optionally wherein the PSCs are conditioned post-translationally, preferably by altering, preferably increasing or decreasing, protein acetylation, phosphorylation, methylation, ribosylation, ubiquitination, SUMOylation, and/or glycosylation, and optionally wherein the PSCs are metabolically reprogrammed by altering the reactive oxygen species (ROS) signalling, cellular glycolysis, oxidative phosphorylation, pentose phosphate pathway, glycogen metabolism and/or the antioxidant systems, preferably by increasing the ROS signalling, by inhibiting glycolysis, by increasing oxidative phosphorylation, by increasing the pentose phosphate pathway, by increasing glycogen synthase activity and/or by inducing antioxidant enzymes including but not limited to NADPH dehydrogenase quinone1 (Nqo1), Sulfiredoxin 1 (Srxn1), Glutamate-cysteine ligase catalytic subunit (Gclc), Glutamate-cysteine ligase modifier Subunit (Gclm), Glutathionc S-transferase Mu 1 (Gstm1).

4. The method of any of claims 1 to3 further comprising the step
(d) differentiating the PSCs, preferably differentiating the PSCs into cardiomyocytes, cardiac pacemaker cells, skeletal muscle cells, smooth muscle cells, vascular smooth muscle cells, endothelial cells, kidney glomerulus parietal cells, kidney glomerulus podocytes, kidney proximal tubule brush border cells, loop of Henle thin segment cells, thick ascending limb cells, kidney distal tubule cells, kidney collecting duct cells, interstitial kidney cells, hepatocytes, sinusoidal hepatic endothelial cells, hepatic stellate cells, kupffer cells, neurons, pyramidal cells, basket cells, betz cells, medium spiny neurons, purkinje cells, renshaw cells, lugaro cells, unipolar brush cells, granule cells, anterior horn cells, spindle cells, schwann cells, satellite cells, olfactory ensheathing cells, retinal ganglion cells, retinal cone cells, retinal rod cells, langerhans cells, melanocytes, epithelial cells, fibroblasts, keratinocytes, merkel cells, hair follicle cells, osteoblasts, osteoclasts, macrophages, neutrophils, dendritic cells, eosinophils, mast cells, basophils, natural killer cells, lymphocytes, T cells, B cells; hematopoietic stem cells, mesenchymal stem cells, pancreatic α cells, pancreatic β cells, pancreatic gamma cells and pancreatic delta cells.

5. A differentiated cell reprogrammed by the method of claim 4.

6. A pluripotent stem cell reprogrammed by the method of any of claims 1 to 3.

7. A reprogrammed pluripotent stem cell (pPSC) **characterized in that** the expression of the germ cell markers is increased, optionally further **characterized in that** the expression levels of tumour suppressor is increased, optionally further **characterized in that** the expression level of metabolic markers, preferably selected from the group consisting of oxidative phosphorylation marker, pentose phosphate pathway marker, NADPH generating enzyme marker, glycolysis marker, and glycogen metabolism marker, is altered, optionally further **characterized in that** the expression of core pluripotency markers remain unaltered in comparison to the expression in embryonic stem cells.

8. A reprogrammed pluripotent stem cell (pPSC) according to any of claims 6 to 7 for use in treating infertility in male caused by anti-cancer therapy or other therapy which damages the SSC population within the testis, environmental factors, heat, cold, food, radiation, chemical toxicity, infection, inflammation, autoimmune disease, physical injury or genetics.

9. A reprogrammed pluripotent stem cell (pPSC) according to any of claims 6 to 7 for use in cell or tissue therapy for tissue and organ regeneration of the heart, cardiovascular system, brain, neurological system, eye, ear, liver, kidney, pancreas, endocrine glands, lung, intestines, muscle, skin, hair, joints, bones, and/or teeth.

10. A method of screening for a pharmaceutical, preferably a pharmaceutical directed against cancer or contraception drugs, comprising the use of a differentiated cell according to claim 5 and/or a reprogrammed pluripotent stem cell (pPSC) according to any of claims 6 to 7.

11. A method of testing the toxicity of a pharmaceutical, preferably a pharmaceutical directed against cancer or contraception drugs, comprising the use of a differentiated cell according to claim 5 and/or a reprogrammed pluripotent stem cell (pPSC) according to any of claims 6 to 7.

12. A method of testing the toxicity of an environmental substance (molecule or particle) preferably a substance that might cause cancer, comprising the use of a differentiated cell according to claim 5 and/or a reprogrammed pluripotent stem cell (pPSC) according to any of claims 6 to 7.

13. A pharmaceutical comprising a differentiated cell according to claim 5 and/or a reprogrammed pluripotent stem cell (pPSC) according to any of claims 6 to 7, and optionally further comprising a pharmaceutically acceptable carrier and/or excipient and optionally one or more additional active substances.

14. A method of identifying genes involved in disease, preferably involved in cancer, infertility, oligospermia, aspermia, hypospermia, azoospermia, teratospermia, asthenozoospermia, cardiovascular disease, atherosclerosis, hepatitis, fatty liver disease, cirrhosis, primary sclerosing cholangitis, hemochromatosis, chronic kidney disease, glomerulonephritis, polycystic kidney disease, alzheimer's disease, parkinson's disease, multiple sclerosis, amyotrophic lateral sclerosis, motor neuron diseases, lewy body disease, huntington's disease, spinocerebellar ataxia, friedreich's ataxia, spinal muscular atrophy, retinopathy, macular degeneration and diabetes comprising the use of a differentiated cell according to claim 5 and/or a reprogrammed pluripotent stem cell (pPSC) according to any of claims 6 to 7.

15. A method of producing germ cells comprising the use of a reprogrammed pluripotent stem cell (pPSC) according to any of claims 5 to 7.


SEQUENCE LISTING

<110> ETH Zurich

<120> REPROGRAMMING OF PLURIPOTENT STEM CELLS FOR IMPROVED CONTROL OF THEIR
DIFFERENTIATION PATHWAYS

<130> 501-7

<160> 120

<170> BiSSAP 1.2

<210> 1
<211> 21
<212> DNA
<213> Mus musculus

<220>
<221> source
<222> 1..21
<223> /organism="Mus musculus"
      /mol_type="unassigned DNA"

<400> 1
acccccaatg tgtccgtcgt g                                                21

<210> 2
<211> 25
<212> DNA
<213> Mus musculus

<220>
<221> source
<222> 1..25
<223> /organism="Mus musculus"
      /mol_type="unassigned DNA"

<400> 2
agatgcctgc ttcaccacct tcttg                                            25

<210> 3
<211> 23
<212> DNA
<213> Mus musculus

<220>
<221> source
<222> 1..23
<223> /organism="Mus musculus"
      /mol_type="unassigned DNA"

<400> 3
acctcagcct ccagcagatg caa                                              23

<210> 4
<211> 22
<212> DNA
<213> Mus musculus

<220>
<221> source
<222> 1..22
<223> /organism="Mus musculus"
      /mol_type="unassigned DNA"

<400> 4
ccgcttgcac ttcacccttt gg                                                22

<210> 5
<211> 26
<212> DNA
<213> Mus musculus

<220>
<221> source
<222> 1..26
<223> /organism="Mus musculus"
      /mol_type="unassigned DNA"

<400> 5
tgctgaagca gaagaggatc accttg                                            26

<210> 6
<211> 25
<212> DNA
<213> Mus musculus

<220>
<221> source
<222> 1..25
<223> /organism="Mus musculus"
      /mol_type="unassigned DNA"

<400> 6
tgttcttaag gctgagctgc aaggc                                             25

<210> 7
<211> 26
<212> DNA
<213> Mus musculus

<220>
<221> source
<222> 1..26
<223> /organism="Mus musculus"
      /mol_type="unassigned DNA"

<400> 7
agggttcttg ctgggttttg attctg                                            26

<210> 8
<211> 27
<212> DNA
<213> Mus musculus

<220>
<221> source
<222> 1..27
<223> /organism="Mus musculus"
      /mol_type="unassigned DNA"

<400> 8
aacggtcttg ccagtacttg ctctcat                                           27

<210> 9
<211> 22
<212> DNA
<213> Mus musculus

<220>
<221> source
<222> 1..22
<223> /organism="Mus musculus"
      /mol_type="unassigned DNA"

<400> 9
tgcccgggac ccaagagaca ta                                                22


<210> 10
<211> 23
<212> DNA
<213> Mus musculus

<220>
<221> source
<222> 1..23
<223> /organism="Mus musculus"
      /mol_type="unassigned DNA"

<400> 10
agtgagttcc ggctggctga ggt                                               23


<210> 11
<211> 22
<212> DNA
<213> Mus musculus

<220>
<221> source
<222> 1..22
<223> /organism="Mus musculus"
      /mol_type="unassigned DNA"

<400> 11
cgcatgggaa ggaagggaca at                                                22


<210> 12
<211> 23
<212> DNA
<213> Mus musculus

<220>
<221> source
<222> 1..23
<223> /organism="Mus musculus"
      /mol_type="unassigned DNA"

<400> 12
ccgctgatcg aactgaaggg aac                                               23


<210> 13
<211> 26
<212> DNA
<213> Mus musculus

<220>
<221> source
<222> 1..26
<223> /organism="Mus musculus"
      /mol_type="unassigned DNA"

<400> 13
gccattattg tgtcggagga agagga                                            26

<210> 14
<211> 26
<212> DNA
<213> Mus musculus

<220>
<221> source
<222> 1..26
<223> /organism="Mus musculus"
      /mol_type="unassigned DNA"

<400> 14
gctcccccgt ttggtacctt tagaac                                          26


<210> 15
<211> 24
<212> DNA
<213> Mus musculus

<220>
<221> source
<222> 1..24
<223> /organism="Mus musculus"
      /mol_type="unassigned DNA"

<400> 15
cgccctgtcc ctttcagttt tgtc                                            24


<210> 16
<211> 23
<212> DNA
<213> Mus musculus

<220>
<221> source
<222> 1..23
<223> /organism="Mus musculus"
      /mol_type="unassigned DNA"

<400> 16
agtcccgcgt ttcaaagcaa cag                                             23


<210> 17
<211> 23
<212> DNA
<213> Mus musculus

<220>
<221> source
<222> 1..23
<223> /organism="Mus musculus"
      /mol_type="unassigned DNA"

<400> 17
tgcagccgta cctgtggaga aca                                             23


<210> 18
<211> 25
<212> DNA
<213> Mus musculus

<220>
<221> source
<222> 1..25

<223> /organism="Mus musculus"
      /mol_type="unassigned DNA"

<400> 18
gtcccgttca aactcatttc cttcg                                          25


<210> 19
<211> 30
<212> DNA
<213> Mus musculus

<220>
<221> source
<222> 1..30
<223> /organism="Mus musculus"
      /mol_type="unassigned DNA"

<400> 19
acctccggct tatacaactg ttaactacca                                     30


<210> 20
<211> 24
<212> DNA
<213> Mus musculus

<220>
<221> source
<222> 1..24
<223> /organism="Mus musculus"
      /mol_type="unassigned DNA"

<400> 20
aagcactgcc cgacttcttc tgaa                                           24


<210> 21
<211> 22
<212> DNA
<213> Mus musculus

<220>
<221> source
<222> 1..22
<223> /organism="Mus musculus"
      /mol_type="unassigned DNA"

<400> 21
cagcgctatg tttgccacct gc                                             22


<210> 22
<211> 21
<212> DNA
<213> Mus musculus

<220>
<221> source
<222> 1..21
<223> /organism="Mus musculus"
      /mol_type="unassigned DNA"

<400> 22
tgggggctct ggttcctggt t                                              21


<210> 23
<211> 23

<212> DNA
<213> Mus musculus

<220>
<221> source
<222> 1..23
<223> /organism="Mus musculus"
      /mol_type="unassigned DNA"

<400> 23
tccgcaagga cagagaagaa ccc                                              23


<210> 24
<211> 25
<212> DNA
<213> Mus musculus

<220>
<221> source
<222> 1..25
<223> /organism="Mus musculus"
      /mol_type="unassigned DNA"

<400> 24
tgctcgtccc agtggtaaca gagag                                            25


<210> 25
<211> 20
<212> DNA
<213> Mus musculus

<220>
<221> source
<222> 1..20
<223> /organism="Mus musculus"
      /mol_type="unassigned DNA"

<400> 25
cgccaccacc gaggaggagt                                                  20


<210> 26
<211> 22
<212> DNA
<213> Mus musculus

<220>
<221> source
<222> 1..22
<223> /organism="Mus musculus"
      /mol_type="unassigned DNA"

<400> 26
ccgggttcaa caagggagca gt                                               22


<210> 27
<211> 27
<212> DNA
<213> Mus musculus

<220>
<221> source
<222> 1..27
<223> /organism="Mus musculus"
      /mol_type="unassigned DNA"

<400> 27
tggctgcgag aagagcttta ttcagtc                                          27


<210> 28
<211> 25
<212> DNA
<213> Mus musculus

<220>
<221> source
<222> 1..25
<223> /organism="Mus musculus"
      /mol_type="unassigned DNA"

<400> 28
cgtgtatccc cagtgcctct gtcat                                            25


<210> 29
<211> 23
<212> DNA
<213> Mus musculus

<220>
<221> source
<222> 1..23
<223> /organism="Mus musculus"
      /mol_type="unassigned DNA"

<400> 29
gcgagtggtg gcagctgtcc tac                                              23


<210> 30
<211> 24
<212> DNA
<213> Mus musculus

<220>
<221> source
<222> 1..24
<223> /organism="Mus musculus"
      /mol_type="unassigned DNA"

<400> 30
ggctgctctt caccgcacac atag                                             24


<210> 31
<211> 22
<212> DNA
<213> Mus musculus

<220>
<221> source
<222> 1..22
<223> /organism="Mus musculus"
      /mol_type="unassigned DNA"

<400> 31
gtggggctgt ggcaggagaa tg                                               22


<210> 32
<211> 28
<212> DNA
<213> Mus musculus

<220>
<221> source
<222> 1..28
<223> /organism="Mus musculus"
      /mol_type="unassigned DNA"

<400> 32
gtagtgtcgg gaggcaatgt atagggaa                                          28


<210> 33
<211> 24
<212> DNA
<213> Mus musculus

<220>
<221> source
<222> 1..24
<223> /organism="Mus musculus"
      /mol_type="unassigned DNA"

<400> 33
gagggcccac tcaccattga catt                                              24


<210> 34
<211> 23
<212> DNA
<213> Mus musculus

<220>
<221> source
<222> 1..23
<223> /organism="Mus musculus"
      /mol_type="unassigned DNA"

<400> 34
tccagtgccc tggggtcctt tag                                               23


<210> 35
<211> 23
<212> DNA
<213> Mus musculus

<220>
<221> source
<222> 1..23
<223> /organism="Mus musculus"
      /mol_type="unassigned DNA"

<400> 35
ggcgcatgtt tcctttcttg agc                                               23


<210> 36
<211> 27
<212> DNA
<213> Mus musculus

<220>
<221> source
<222> 1..27
<223> /organism="Mus musculus"
      /mol_type="unassigned DNA"

<400> 36
agtgggagcc agtgttagga gattctg                                           27

<210> 37
<211> 24
<212> DNA
<213> Mus musculus

<220>
<221> source
<222> 1..24
<223> /organism="Mus musculus"
      /mol_type="unassigned DNA"

<400> 37
tggcgtagaa atgctgaggg tgag                                              24


<210> 38
<211> 29
<212> DNA
<213> Mus musculus

<220>
<221> source
<222> 1..29
<223> /organism="Mus musculus"
      /mol_type="unassigned DNA"

<400> 38
ctgttaccgg agcaagcttt tgacttatt                                         29


<210> 39
<211> 22
<212> DNA
<213> Mus musculus

<220>
<221> source
<222> 1..22
<223> /organism="Mus musculus"
      /mol_type="unassigned DNA"

<400> 39
cgctcgggac cccaagacat ac                                                22


<210> 40
<211> 25
<212> DNA
<213> Mus musculus

<220>
<221> source
<222> 1..25
<223> /organism="Mus musculus"
      /mol_type="unassigned DNA"

<400> 40
tctgccggta gaaagggaag aggtc                                             25


<210> 41
<211> 24
<212> DNA
<213> Mus musculus

<220>
<221> source
<222> 1..24

<223> /organism="Mus musculus"
      /mol_type="unassigned DNA"

<400> 41
ctgcccatca aaagccacga agta                                              24


<210> 42
<211> 22
<212> DNA
<213> Mus musculus

<220>
<221> source
<222> 1..22
<223> /organism="Mus musculus"
      /mol_type="unassigned DNA"

<400> 42
ccgggaaaac gaatggaact gc                                                22


<210> 43
<211> 21
<212> DNA
<213> Mus musculus

<220>
<221> source
<222> 1..21
<223> /organism="Mus musculus"
      /mol_type="unassigned DNA"

<400> 43
cggccggcac ctacacttac g                                                 21


<210> 44
<211> 20
<212> DNA
<213> Mus musculus

<220>
<221> source
<222> 1..20
<223> /organism="Mus musculus"
      /mol_type="unassigned DNA"

<400> 44
cgggcggctc tacggacact                                                   20


<210> 45
<211> 25
<212> DNA
<213> Mus musculus

<220>
<221> source
<222> 1..25
<223> /organism="Mus musculus"
      /mol_type="unassigned DNA"

<400> 45
tgctttcccg agacccagtt catag                                             25


<210> 46
<211> 24

<212> DNA
<213> Mus musculus

<220>
<221> source
<222> 1..24
<223> /organism="Mus musculus"
      /mol_type="unassigned DNA"

<400> 46
agtcccccgg ttcctccatt acat                                              24


<210> 47
<211> 23
<212> DNA
<213> Mus musculus

<220>
<221> source
<222> 1..23
<223> /organism="Mus musculus"
      /mol_type="unassigned DNA"

<400> 47
ggacagaggc caccgcacac taa                                               23


<210> 48
<211> 24
<212> DNA
<213> Mus musculus

<220>
<221> source
<222> 1..24
<223> /organism="Mus musculus"
      /mol_type="unassigned DNA"

<400> 48
aatgagggca gggggcagat aaaa                                              24


<210> 49
<211> 22
<212> DNA
<213> Mus musculus

<220>
<221> source
<222> 1..22
<223> /organism="Mus musculus"
      /mol_type="unassigned DNA"

<400> 49
accggcgacc aggctcatac ac                                                22


<210> 50
<211> 24
<212> DNA
<213> Mus musculus

<220>
<221> source
<222> 1..24
<223> /organism="Mus musculus"
      /mol_type="unassigned DNA"

<400> 50
gcccactgcg tcggtagaga gact                                              24


<210> 51
<211> 24
<212> DNA
<213> Mus musculus

<220>
<221> source
<222> 1..24
<223> /organism="Mus musculus"
      /mol_type="unassigned DNA"

<400> 51
gtggcctggc agaaaacctc actt                                              24


<210> 52
<211> 25
<212> DNA
<213> Mus musculus

<220>
<221> source
<222> 1..25
<223> /organism="Mus musculus"
      /mol_type="unassigned DNA"

<400> 52
ctggcgtttg ctgtctcttt cttcc                                             25


<210> 53
<211> 28
<212> DNA
<213> Mus musculus

<220>
<221> source
<222> 1..28
<223> /organism="Mus musculus"
      /mol_type="unassigned DNA"

<400> 53
aacgccaaac ccttttattc agtgctac                                          28


<210> 54
<211> 26
<212> DNA
<213> Mus musculus

<220>
<221> source
<222> 1..26
<223> /organism="Mus musculus"
      /mol_type="unassigned DNA"

<400> 54
tgcccaacag cgacaaacaa gtaact                                            26


<210> 55
<211> 23
<212> DNA
<213> Mus musculus

<220>
<221> source
<222> 1..23
<223> /organism="Mus musculus"
      /mol_type="unassigned DNA"

<400> 55
ccgcccattt ttacccccta caa                                              23


<210> 56
<211> 24
<212> DNA
<213> Mus musculus

<220>
<221> source
<222> 1..24
<223> /organism="Mus musculus"
      /mol_type="unassigned DNA"

<400> 56
accccagccc atatcctctc aaca                                             24


<210> 57
<211> 30
<212> DNA
<213> Mus musculus

<220>
<221> source
<222> 1..30
<223> /organism="Mus musculus"
      /mol_type="unassigned DNA"

<400> 57
agattcgctc ttcacggaat atgttttaca                                       30


<210> 58
<211> 28
<212> DNA
<213> Mus musculus

<220>
<221> source
<222> 1..28
<223> /organism="Mus musculus"
      /mol_type="unassigned DNA"

<400> 58
cattgccctc gtgtcttcaa taagtgac                                         28


<210> 59
<211> 22
<212> DNA
<213> Mus musculus

<220>
<221> source
<222> 1..22
<223> /organism="Mus musculus"
      /mol_type="unassigned DNA"

<400> 59
gaccgccttt accccgatag cc                                               22

<210> 60
<211> 20
<212> DNA
<213> Mus musculus

<220>
<221> source
<222> 1..20
<223> /organism="Mus musculus"
      /mol_type="unassigned DNA"

<400> 60
tgagggcctt gcgcttctgg                                                    20


<210> 61
<211> 25
<212> DNA
<213> Mus musculus

<220>
<221> source
<222> 1..25
<223> /organism="Mus musculus"
      /mol_type="unassigned DNA"

<400> 61
ctaatcgcct cacgttggac tctgg                                              25


<210> 62
<211> 24
<212> DNA
<213> Mus musculus

<220>
<221> source
<222> 1..24
<223> /organism="Mus musculus"
      /mol_type="unassigned DNA"

<400> 62
caccgcctct cctccttgag aaac                                               24


<210> 63
<211> 20
<212> DNA
<213> Mus musculus

<220>
<221> source
<222> 1..20
<223> /organism="Mus musculus"
      /mol_type="unassigned DNA"

<400> 63
ccgcctgccg gtagacttgg                                                    20


<210> 64
<211> 25
<212> DNA
<213> Mus musculus

<220>
<221> source
<222> 1..25

<223> /organism="Mus musculus"
      /mol_type="unassigned DNA"

<400> 64
ctagatgggg ctggggagaa agaag                                          25


<210> 65
<211> 24
<212> DNA
<213> Mus musculus

<220>
<221> source
<222> 1..24
<223> /organism="Mus musculus"
      /mol_type="unassigned DNA"

<400> 65
caagagcggg gacatcaaga catc                                           24


<210> 66
<211> 23
<212> DNA
<213> Mus musculus

<220>
<221> source
<222> 1..23
<223> /organism="Mus musculus"
      /mol_type="unassigned DNA"

<400> 66
accacccagc ggaacacaaa gag                                            23


<210> 67
<211> 27
<212> DNA
<213> Mus musculus

<220>
<221> source
<222> 1..27
<223> /organism="Mus musculus"
      /mol_type="unassigned DNA"

<400> 67
agatccacag cgatatccag acattca                                        27


<210> 68
<211> 24
<212> DNA
<213> Mus musculus

<220>
<221> source
<222> 1..24
<223> /organism="Mus musculus"
      /mol_type="unassigned DNA"

<400> 68
tcggacatca ccaggattgg acat                                           24


<210> 69
<211> 24

<212> DNA
<213> Mus musculus

<220>
<221> source
<222> 1..24
<223> /organism="Mus musculus"
      /mol_type="unassigned DNA"

<400> 69
aagcactgcc gggatatgga agaa                                              24


<210> 70
<211> 21
<212> DNA
<213> Mus musculus

<220>
<221> source
<222> 1..21
<223> /organism="Mus musculus"
      /mol_type="unassigned DNA"

<400> 70
gcgcgggggc ctgtagtaga a                                                 21


<210> 71
<211> 22
<212> DNA
<213> Mus musculus

<220>
<221> source
<222> 1..22
<223> /organism="Mus musculus"
      /mol_type="unassigned DNA"

<400> 71
ctgaacgccg aggaccagaa cc                                                22


<210> 72
<211> 22
<212> DNA
<213> Mus musculus

<220>
<221> source
<222> 1..22
<223> /organism="Mus musculus"
      /mol_type="unassigned DNA"

<400> 72
cggtagaagg cgggcacaga ct                                                22


<210> 73
<211> 23
<212> DNA
<213> Mus musculus

<220>
<221> source
<222> 1..23
<223> /organism="Mus musculus"
      /mol_type="unassigned DNA"

<400> 73
catcctgcgt ttctgtggct tcc                                              23

<210> 74
<211> 21
<212> DNA
<213> Mus musculus

<220>
<221> source
<222> 1..21
<223> /organism="Mus musculus"
      /mol_type="unassigned DNA"

<400> 74
gcgggcatct ggtggagtgt g                                                21

<210> 75
<211> 25
<212> DNA
<213> Mus musculus

<220>
<221> source
<222> 1..25
<223> /organism="Mus musculus"
      /mol_type="unassigned DNA"

<400> 75
ttggctgcat tggcttggtt actct                                            25

<210> 76
<211> 26
<212> DNA
<213> Mus musculus

<220>
<221> source
<222> 1..26
<223> /organism="Mus musculus"
      /mol_type="unassigned DNA"

<400> 76
cgctccaggc cctctcacta ctactc                                           26

<210> 77
<211> 22
<212> DNA
<213> Mus musculus

<220>
<221> source
<222> 1..22
<223> /organism="Mus musculus"
      /mol_type="unassigned DNA"

<400> 77
tgcggaggca tcaaaggctt ct                                               22

<210> 78
<211> 29
<212> DNA
<213> Mus musculus

<220>
<221> source
<222> 1..29
<223> /organism="Mus musculus"
      /mol_type="unassigned DNA"

<400> 78
agtggccagc tgatcataaa ggtatcttg                                          29


<210> 79
<211> 21
<212> DNA
<213> Mus musculus

<220>
<221> source
<222> 1..21
<223> /organism="Mus musculus"
      /mol_type="unassigned DNA"

<400> 79
atggcttcgc ctccgattga a                                                  21


<210> 80
<211> 29
<212> DNA
<213> Mus musculus

<220>
<221> source
<222> 1..29
<223> /organism="Mus musculus"
      /mol_type="unassigned DNA"

<400> 80
caggaggcca ggttaacttg gttactatt                                          29


<210> 81
<211> 21
<212> DNA
<213> Mus musculus

<220>
<221> source
<222> 1..21
<223> /organism="Mus musculus"
      /mol_type="unassigned DNA"

<400> 81
acgtccgcgg actgacacac c                                                  21


<210> 82
<211> 25
<212> DNA
<213> Mus musculus

<220>
<221> source
<222> 1..25
<223> /organism="Mus musculus"
      /mol_type="unassigned DNA"

<400> 82
agtccaggcc cagcttgaac ttctc                                              25

<210> 83
<211> 22
<212> DNA
<213> Mus musculus

<220>
<221> source
<222> 1..22
<223> /organism="Mus musculus"
      /mol_type="unassigned DNA"

<400> 83
gaaaggcggg ggaatggaca ac                                                22


<210> 84
<211> 26
<212> DNA
<213> Mus musculus

<220>
<221> source
<222> 1..26
<223> /organism="Mus musculus"
      /mol_type="unassigned DNA"

<400> 84
gcccagccca agcagtataa gcagta                                            26


<210> 85
<211> 23
<212> DNA
<213> Mus musculus

<220>
<221> source
<222> 1..23
<223> /organism="Mus musculus"
      /mol_type="unassigned DNA"

<400> 85
cctttgatcc gcgcttccct aac                                               23


<210> 86
<211> 23
<212> DNA
<213> Mus musculus

<220>
<221> source
<222> 1..23
<223> /organism="Mus musculus"
      /mol_type="unassigned DNA"

<400> 86
gcggcgattc atggtcttca cac                                               23


<210> 87
<211> 22
<212> DNA
<213> Mus musculus

<220>
<221> source
<222> 1..22

<223> /organism="Mus musculus"
      /mol_type="unassigned DNA"

<400> 87
gccgcctcag ccactcagaa ag                                              22


<210> 88
<211> 28
<212> DNA
<213> Mus musculus

<220>
<221> source
<222> 1..28
<223> /organism="Mus musculus"
      /mol_type="unassigned DNA"

<400> 88
gcagctggga tgtaaaaggt cgtaaaaa                                        28


<210> 89
<211> 21
<212> DNA
<213> Mus musculus

<220>
<221> source
<222> 1..21
<223> /organism="Mus musculus"
      /mol_type="unassigned DNA"

<400> 89
ccgcgagggc tgagaaggaa c                                               21


<210> 90
<211> 22
<212> DNA
<213> Mus musculus

<220>
<221> source
<222> 1..22
<223> /organism="Mus musculus"
      /mol_type="unassigned DNA"

<400> 90
cggggcaaat atcagggcag tg                                              22


<210> 91
<211> 22
<212> DNA
<213> Mus musculus

<220>
<221> source
<222> 1..22
<223> /organism="Mus musculus"
      /mol_type="unassigned DNA"

<400> 91
agcgcggttc cgttacctga tg                                              22


<210> 92
<211> 21

<212> DNA
<213> Mus musculus

<220>
<221> source
<222> 1..21
<223> /organism="Mus musculus"
      /mol_type="unassigned DNA"

<400> 92
gacgccggca acattcacac c                                                21


<210> 93
<211> 31
<212> DNA
<213> Mus musculus

<220>
<221> source
<222> 1..31
<223> /organism="Mus musculus"
      /mol_type="unassigned DNA"

<400> 93
gctaggcggc tttgtgattt gtattatgtt a                                     31


<210> 94
<211> 25
<212> DNA
<213> Mus musculus

<220>
<221> source
<222> 1..25
<223> /organism="Mus musculus"
      /mol_type="unassigned DNA"

<400> 94
tccccggtca ctcatcttca cagtc                                            25


<210> 95
<211> 22
<212> DNA
<213> Mus musculus

<220>
<221> source
<222> 1..22
<223> /organism="Mus musculus"
      /mol_type="unassigned DNA"

<400> 95
cagacgggga caaagtgggt cg                                               22


<210> 96
<211> 22
<212> DNA
<213> Mus musculus

<220>
<221> source
<222> 1..22
<223> /organism="Mus musculus"
      /mol_type="unassigned DNA"

<400> 96
cagccgcaac tccttccctt ca                                                22

<210> 97
<211> 23
<212> DNA
<213> Mus musculus

<220>
<221> source
<222> 1..23
<223> /organism="Mus musculus"
      /mol_type="unassigned DNA"

<400> 97
aagccgtgcg caaacaacta tgg                                               23

<210> 98
<211> 23
<212> DNA
<213> Mus musculus

<220>
<221> source
<222> 1..23
<223> /organism="Mus musculus"
      /mol_type="unassigned DNA"

<400> 98
attcggcgga tggtggtcaa gat                                               23

<210> 99
<211> 24
<212> DNA
<213> Mus musculus

<220>
<221> source
<222> 1..24
<223> /organism="Mus musculus"
      /mol_type="unassigned DNA"

<400> 99
ctatgcccgc tcacgactca cagt                                              24

<210> 100
<211> 26
<212> DNA
<213> Mus musculus

<220>
<221> source
<222> 1..26
<223> /organism="Mus musculus"
      /mol_type="unassigned DNA"

<400> 100
gaattacggg caaagaactc ctccag                                            26

<210> 101
<211> 24
<212> DNA
<213> Mus musculus

<220>
<221> source
<222> 1..24
<223> /organism="Mus musculus"
      /mol_type="unassigned DNA"

<400> 101
gaaccggaga accctgctgt gact                                              24


<210> 102
<211> 21
<212> DNA
<213> Mus musculus

<220>
<221> source
<222> 1..21
<223> /organism="Mus musculus"
      /mol_type="unassigned DNA"

<400> 102
atgccgcatg cccagaacat c                                                 21


<210> 103
<211> 23
<212> DNA
<213> Mus musculus

<220>
<221> source
<222> 1..23
<223> /organism="Mus musculus"
      /mol_type="unassigned DNA"

<400> 103
ggtgggcctc aagaggagca gat                                               23


<210> 104
<211> 23
<212> DNA
<213> Mus musculus

<220>
<221> source
<222> 1..23
<223> /organism="Mus musculus"
      /mol_type="unassigned DNA"

<400> 104
ttgccgaccc cagcttcttt gta                                               23


<210> 105
<211> 23
<212> DNA
<213> Mus musculus

<220>
<221> source
<222> 1..23
<223> /organism="Mus musculus"
      /mol_type="unassigned DNA"

<400> 105
gccacgccag tgaccgtatc att                                               23

<210> 106
<211> 22
<212> DNA
<213> Mus musculus

<220>
<221> source
<222> 1..22
<223> /organism="Mus musculus"
      /mol_type="unassigned DNA"

<400> 106
ggtcgaaggc ccgtgtgaag aa                                                22


<210> 107
<211> 22
<212> DNA
<213> Mus musculus

<220>
<221> source
<222> 1..22
<223> /organism="Mus musculus"
      /mol_type="unassigned DNA"

<400> 107
tgccttgggg attgctcact tg                                                22


<210> 108
<211> 21
<212> DNA
<213> Mus musculus

<220>
<221> source
<222> 1..21
<223> /organism="Mus musculus"
      /mol_type="unassigned DNA"

<400> 108
atgatgggcc gctcgttgaa g                                                 21


<210> 109
<211> 27
<212> DNA
<213> Mus musculus

<220>
<221> source
<222> 1..27
<223> /organism="Mus musculus"
      /mol_type="unassigned DNA"

<400> 109
cctgggcctg gaaaagtaga gataacc                                           27


<210> 110
<211> 21
<212> DNA
<213> Mus musculus

<220>
<221> source
<222> 1..21

<223> /organism="Mus musculus"
      /mol_type="unassigned DNA"

<400> 110
aggccagccc ttggacagag c                                                21


<210> 111
<211> 25
<212> DNA
<213> Mus musculus

<220>
<221> source
<222> 1..25
<223> /organism="Mus musculus"
      /mol_type="unassigned DNA"

<400> 111
tgccgagggt gagcctgtat gtaat                                            25


<210> 112
<211> 24
<212> DNA
<213> Mus musculus

<220>
<221> source
<222> 1..24
<223> /organism="Mus musculus"
      /mol_type="unassigned DNA"

<400> 112
tgctgctagt ggcattgctg gagt                                             24


<210> 113
<211> 24
<212> DNA
<213> Mus musculus

<220>
<221> source
<222> 1..24
<223> /organism="Mus musculus"
      /mol_type="unassigned DNA"

<400> 113
gaccctcggg cggataaaaa agag                                             24


<210> 114
<211> 20
<212> DNA
<213> Mus musculus

<220>
<221> source
<222> 1..20
<223> /organism="Mus musculus"
      /mol_type="unassigned DNA"

<400> 114
gcgccgctcc agctcgtaga                                                  20


<210> 115
<211> 24

<212> DNA
<213> Mus musculus

<220>
<221> source
<222> 1..24
<223> /organism="Mus musculus"
      /mol_type="unassigned DNA"

<400> 115
atctccgcgt tcttatccca cctg                                              24


<210> 116
<211> 22
<212> DNA
<213> Mus musculus

<220>
<221> source
<222> 1..22
<223> /organism="Mus musculus"
      /mol_type="unassigned DNA"

<400> 116
ccaccggggt agccaatgac tg                                                22


<210> 117
<211> 25
<212> DNA
<213> Mus musculus

<220>
<221> source
<222> 1..25
<223> /organism="Mus musculus"
      /mol_type="unassigned DNA"

<400> 117
agggctcagt ccttctcttc tccgt                                             25


<210> 118
<211> 21
<212> DNA
<213> Mus musculus

<220>
<221> source
<222> 1..21
<223> /organism="Mus musculus"
      /mol_type="unassigned DNA"

<400> 118
tcccgagggc aaagggtcac t                                                 21


<210> 119
<211> 25
<212> DNA
<213> Mus musculus

<220>
<221> source
<222> 1..25
<223> /organism="Mus musculus"
      /mol_type="unassigned DNA"

<400> 119
cgcatcaagg agctcaccta ccaga                                          25


<210> 120
<211> 21
<212> DNA
<213> Mus musculus

<220>
<221> source
<222> 1..21
<223> /organism="Mus musculus"
      /mol_type="unassigned DNA"

<400> 120
ggcaccaatg tcccggctct t                                              21